(19) Europäisches Patentamt
European Patent Office
Office européen des brevets

(11) **EP 4 129 261 A1**

(12) **EUROPEAN PATENT APPLICATION**
published in accordance with Art. 153(4) EPC

(43) Date of publication:
**08.02.2023  Bulletin 2023/06**

(21) Application number: **21778771.2**

(22) Date of filing: **03.02.2021**

(51) International Patent Classification (IPC):
**A61H 1/02** *(1968.09)*  **G16H 20/30** *(2018.01)*
**A61B 5/083** *(1990.01)*  **A61B 5/11** *(1990.01)*
**A61B 5/22** *(1985.01)*

(52) Cooperative Patent Classification (CPC):
**A61B 5/083; A61B 5/11; A61B 5/22; A61H 1/02;
G16H 20/30**

(86) International application number:
**PCT/JP2021/003933**

(87) International publication number:
**WO 2021/199660 (07.10.2021 Gazette 2021/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.03.2020  JP 2020063624**

(71) Applicant: **Sony Group Corporation
Minato-Ku, Tokyo, 108-0075 (JP)**

(72) Inventors:
• **FUJIMOTO, Takashi
Tokyo 108-0075 (JP)**
• **NISHIMURA, Kiminobu
Tokyo 108-0075 (JP)**
• **MATSUZAWA, Sota
Tokyo 108-0075 (JP)**
• **KATSU, Masanori
Tokyo 108-0075 (JP)**
• **SHIGYO, Maki
Tokyo 108-0075 (JP)**

(74) Representative: **MFG Patentanwälte
Meyer-Wildhagen Meggle-Freund
Gerhard PartG mbB
Amalienstraße 62
80799 München (DE)**

(54) **INFORMATION PROCESSING DEVICE, INFORMATION PROCESSING SYSTEM, INFORMATION PROCESSING METHOD, AND PROGRAM**

(57)    A configuration that enables a rehabilitation execution user to appropriately execute rehabilitation while confirming whether or not exercise being executed by the user is an appropriate exercise according to an exercise prescription is achieved. The configuration includes an exercise type estimation unit configured to estimate an exercise type executed by a user on the basis of detection information of a sensor worn on a body of the user, a database configured to store user-specific exercise intensity corresponding to the exercise type, an exercise type/personalized exercise intensity acquisition unit configured to acquire, from the database, an exercise type/personalized exercise intensity that is user-specific exercise intensity corresponding to the exercise type estimated by the exercise type estimation unit, and an exercise implementation status analysis unit configured to analyze exercise implementation status data generated on the basis of the exercise type/personalized exercise intensity acquired from the database and generate an exercise implementation status analysis result indicating whether or not exercise being executed by the user is an exercise according to a pre-generated exercise prescription, in which the generated exercise implementation status analysis result is output to a display unit.

FIG. 12

**Description**

TECHNICAL FIELD

The present disclosure relates to an information processing apparatus, an information processing system, an information processing method, and a program. More specifically, the present invention relates to an information processing apparatus, an information processing system, an information processing method, and a program for analyzing an execution state of rehabilitation.

BACKGROUND ART

For example, when a heart disease such as myocardial infarction occurs, treatment such as surgery is performed in a hospital, and then rehabilitation in accordance with recovery of physical strength is performed while being hospitalized.

However, a hospital stay period and a rehabilitation period in a hospital are limited, and it is required to perform rehabilitation while visiting the hospital according to a program created by the hospital or spontaneous home rehabilitation at home or the like after discharge.

In a case where rehabilitation is performed in a hospital by visiting a hospital, appropriate exercise or the like can be performed under monitoring of an expert, but in a case where rehabilitation is performed at home, it is necessary to spontaneously perform exercise without monitoring of an expert. In this case, it is necessary to determine by oneself whether or not the amount of exercise is appropriate, and there is a problem that it is difficult to perform rehabilitation involving an optimal amount of exercise.

In particular, in the case of the heart disease, excessive exercise may exacerbate a disease as an adverse effect.

Note that there are, for example, Patent Document 1 (Japanese Patent Application Laid-Open No. 2014 018213) and Patent Document 2 (Japanese Patent Application Laid-Open No. 2016 -158711) as conventional techniques that disclose a configuration for allowing a patient to perform appropriate rehabilitation.

Patent Document 1 discloses a configuration in which a user terminal that performs pulse detection is worn on a patient performing rehabilitation, appropriate pulse rate range data is transmitted from a hospital side practitioner terminal to a user terminal, and the patient refers to the pulse rate transmitted to the user terminal to perform rehabilitation so that the pulse rate rising by exercise falls within the range of the received pulse rate.

However, the pulse rate is mostly calculated based on periodicity of a blood flow, and when there is intense exercise such as sports, the blood flow is greatly disturbed, and the periodicity may be difficult to observe. In this case, the pulse rate is greatly different from the actual heart rate. Therefore, it can be said that the monitoring of the pulse rate alone is insufficient to confirm whether appropriate exercise is performed.

Patent Document 2 discloses a configuration in which a patient performing rehabilitation wears an electrocardiographic garment for measuring an electrocardiogram to acquire the electrocardiogram, a user position is further acquired by a position information detection device, the electrocardiogram and the user position are transmitted to a hospital side terminal via a user terminal, and the amount of exercise or the state of the heart of the user is analyzed on the hospital side.

In this document, the amount of exercise of the user is measured on the basis of a change in the position of the user, and the amount of exercise in a case where the user runs or walks is estimated. However, the exercise performed by the user is not limited to the exercise involving such a position change. For example, there is a case where exercise is performed using a fitness machine such as an indoor running machine or a fixed bike (for example, AEROBIKE (registered trademark)), and in a case where exercise using such a machine is performed, there is a problem that the amount of exercise cannot be estimated because the change in the user position cannot be observed.

CITATION LIST

PATENT DOCUMENT

    Patent Document 1: Japanese Patent Application Laid-Open No. 2014-018213
    Patent Document 2: Japanese Patent Application Laid-Open No. 2016-158711

SUMMARY OF THE INVENTION

PROBLEMS TO BE SOLVED BY THE INVENTION

The present disclosure has been made in view of the above problems, for example, and an object thereof is to provide an information processing apparatus, an information processing system, an information processing method, and a program capable of performing rehabilitation optimal for each user in consideration of exercise intensity according to an exercise type of each user who performs rehabilitation.

SOLUTIONS TO PROBLEMS

A first aspect of the present disclosure provides an information processing apparatus, including:

an exercise type estimation unit configured to estimate an exercise type executed by a user on the basis of sensor detection information;
a database configured to store user-specific exercise intensity corresponding to the exercise type;
an exercise type/personalized exercise intensity acquisition unit configured to acquire, from the database, an exercise type/personalized exercise intensity that is the user-specific exercise intensity corresponding to the exercise type estimated by the exercise type estimation unit;
an exercise implementation status analysis unit configured to analyze exercise implementation status data generated on the basis of the exercise type/personalized exercise intensity acquired from the database, and generate an exercise implementation status analysis result indicating whether or not exercise being executed by the user is an exercise according to a pre-generated exercise prescription.

Furthermore, a second aspect of the present disclosure provides an information processing apparatus, including:

an exercise type estimation unit configured to estimate an exercise type executed by a user on the basis of sensor detection information;
a steady state activity amount analysis unit configured to input an estimated exercise type estimated by the exercise type estimation unit and an exhaled gas analysis result of the user to calculate an exhalation analysis result-based exercise type/personalized activity amount;
an exercise intensity-based exercise type/personalized activity amount calculation unit configured to acquire an exercise type corresponding standard exercise intensity estimated by the exercise type estimation unit from an exercise type corresponding standard exercise intensity database storing the exercise type corresponding standard exercise intensity, and calculate an exercise intensity-based exercise type/personalized activity amount on the basis of the acquired standard exercise intensity and a weight of the user; and
an exercise type/personalized exercise intensity calculation unit configured to calculate an exercise type/personalized exercise intensity that is the user corresponding exercise intensity corresponding to the exercise type on the basis of each data of (a) an exhalation analysis result-based exercise type/personalized activity amount and (b) the exercise intensity-based exercise type/personalized activity amount.

Furthermore, a third aspect of the present disclosure provides an information processing system, including:

a user terminal; and a hospital terminal,
in which the user terminal includes:

an exercise type estimation unit configured to estimate an exercise type executed by a user on the basis of sensor detection information;
a database configured to store user-specific exercise intensity corresponding to the exercise type;
an exercise type/personalized exercise intensity acquisition unit configured to acquire, from the database, an exercise type/personalized exercise intensity that is the user-specific exercise intensity corresponding to the exercise type estimated by the exercise type estimation unit; and
a communication unit configured to transmit exercise implementation status data generated on the basis of the exercise type/personalized exercise intensity acquired from the database to the hospital terminal,
the hospital terminal includes:

an exercise implementation status analysis unit configured to analyze the exercise implementation status data received from the user terminal and generate an exercise implementation status analysis result indicating whether or not the exercise being executed by the user is the exercise according to the pre-generated exercise prescription; and

a communication unit configured to transmit the exercise implementation status analysis result generated by the exercise implementation status analysis unit to the user terminal, and

the user terminal displays the exercise implementation status analysis result received from the hospital terminal on a display unit.

Furthermore, a fourth aspect of the present disclosure provides an information processing system, including:

a user terminal and a geographic information providing server,
in which the user terminal includes:

an exercise type estimation unit configured to estimate an exercise type executed by a user on the basis of sensor detection information;

a database configured to store user-specific exercise intensity corresponding to the exercise type;

an exercise type/personalized exercise intensity acquisition unit configured to acquire, from the database, an exercise type/personalized exercise intensity that is the user-specific exercise intensity corresponding to the exercise type estimated by the exercise type estimation unit;

a maximum value corresponding oxygen intake rate calculation unit configured to input the exercise type/personalized exercise intensity acquired by the exercise type/personalized exercise intensity acquisition unit, and calculate a maximum value corresponding oxygen intake rate according to the exercise type executed by the user; and

a communication unit configured to generate exercise prescription information for realizing the maximum value corresponding oxygen intake rate on the basis of the maximum value corresponding oxygen intake rate calculated by the maximum value corresponding oxygen intake rate calculation unit and transmit the generated exercise prescription information to the geographic information providing server,

the geographic information providing server includes:

an exercise prescription corresponding action route information generation unit configured to analyze the exercise prescription information received from the user terminal and generate the exercise prescription corresponding action route information for realizing the exercise prescription information; and

a communication unit configured to transmit the exercise prescription corresponding action route information to the user terminal, and

the user terminal displays the exercise prescription corresponding action route information received from the geographic information providing server on a display unit.

Furthermore, a fifth aspect of the present disclosure provides
an information processing method executed by an information processing apparatus, including:

an exercise type estimation step of estimating, by an exercise type estimation unit, an exercise type executed by a user on the basis of sensor detection information;

an exercise type/personalized exercise intensity acquisition step of acquiring, by an exercise type/personalized exercise intensity acquisition unit, from the database, an exercise type/personalized exercise intensity that is the user-specific exercise intensity corresponding to the exercise type estimated by the exercise type estimation unit; and

an exercise implementation status analysis step of analyzing, by an exercise implementation status analysis unit, exercise implementation status data generated on the basis of the exercise type/personalized exercise intensity acquired from the database, and generating an exercise implementation status analysis result indicating whether or not exercise being executed by the user is an exercise according to a pre-generated exercise prescription.

Furthermore, a sixth aspect of the present disclosure provides
an information processing method executed by an information processing apparatus, including:

an exercise type estimation step of estimating, by an exercise type estimation unit, an exercise type executed by a user on the basis of sensor detection information;

a steady state activity amount analysis step of inputting, by a steady state activity amount analysis unit, an estimated exercise type estimated by the exercise type estimation unit and an exhaled gas analysis result of the user to calculate an exhalation analysis result-based exercise type/personalized activity amount;

an exercise intensity-based exercise type/personalized activity amount calculation step of acquiring, by an exercise intensity-based exercise type/personalized activity amount calculation unit, an exercise type corresponding standard exercise intensity estimated by the exercise type estimation unit from an exercise type corresponding standard exercise intensity database storing the exercise type corresponding standard exercise intensity, and calculating an exercise intensity-based exercise type/personalized activity amount on the basis of the acquired standard exercise intensity and a weight of the user; and

an exercise type/personalized exercise intensity calculation step of calculating, by an exercise type/personalized exercise intensity calculation unit, an exercise type/personalized exercise intensity that is the user corresponding exercise intensity corresponding to the exercise type on the basis of each data of (a) an exhalation analysis result-based exercise type/personalized activity amount and (b) the exercise intensity-based exercise type/personalized activity amount.

Furthermore, a seventh aspect of the present disclosure provides
a program for allowing an information processing apparatus to execute information processing, the program executing:

an exercise type estimation step of estimating, by an exercise type estimation unit, an exercise type executed by a user on the basis of sensor detection information;
an exercise type/personalized exercise intensity acquisition step of acquiring, by an exercise type/personalized exercise intensity acquisition unit, from the database, an exercise type/personalized exercise intensity that is the user-specific exercise intensity corresponding to the exercise type estimated by the exercise type estimation unit; and
an exercise implementation status analysis step of analyzing, by an exercise implementation status analysis unit, exercise implementation status data generated on the basis of the exercise type/personalized exercise intensity acquired from the database, and generating an exercise implementation status analysis result indicating whether or not exercise being executed by the user is an exercise according to a pre-generated exercise prescription.

Furthermore, an eighth aspect of the present disclosure provides
a program for allowing an information processing apparatus to execute information processing, the program executing:

an exercise type estimation step of estimating, by an exercise type estimation unit, an exercise type executed by a user on the basis of sensor detection information;
a steady state activity amount analysis step of inputting, by a steady state activity amount analysis unit, an estimated exercise type estimated by the exercise type estimation unit and an exhaled gas analysis result of the user to calculate an exhalation analysis result-based exercise type/personalized activity amount;
an exercise intensity-based exercise type/personalized activity amount calculation step of acquiring, by an exercise intensity-based exercise type/personalized activity amount calculation unit, an exercise type corresponding standard exercise intensity estimated by the exercise type estimation unit from an exercise type corresponding standard exercise intensity database storing the exercise type corresponding standard exercise intensity, and calculating an exercise intensity-based exercise type/personalized activity amount on the basis of the acquired standard exercise intensity and a weight of the user; and
an exercise type/personalized exercise intensity calculation step of calculating, by an exercise type/personalized exercise intensity calculation unit, an exercise type/personalized exercise intensity that is the user corresponding exercise intensity corresponding to the exercise type on the basis of each data of (a) an exhalation analysis result-based exercise type/personalized activity amount and (b) the exercise intensity-based exercise type/personalized activity amount.

Note that the program of the present disclosure is, for example, a program that can be provided, by a storage medium or a communication medium provided in a computer readable format, to an information processing apparatus, an image processing apparatus, or a computer system capable of executing various program codes. By providing such a program in a computer-readable format, processing according to the program is achieved on the information processing apparatus or the computer system.

Still other objects, features, and advantages of the present disclosure will become apparent from more detailed description based on examples of the present disclosure described later and the accompanying drawings. Note that, in the present specification, a system is a logical set configuration of a plurality of devices, and is not limited to a system in which devices of respective configurations are in the same housing.

According to the configuration of one embodiment of the present disclosure, a configuration in which rehabilitation can be appropriately executed while confirming whether or not exercise being executed by a rehabilitation execution user is an appropriate exercise according to an exercise prescription can be achieved.

Specifically, for example, the configuration includes an exercise type estimation unit configured to estimate an exercise type executed by a user on the basis of detection information of a sensor worn on a body of the user, a database configured to store user-specific exercise intensity corresponding to the exercise type, an exercise type/personalized exercise intensity acquisition unit configured to acquire, from the database, an exercise type/personalized exercise intensity that is user-specific exercise intensity corresponding to the exercise type estimated by the exercise type estimation unit, and an exercise implementation status analysis unit configured to analyze exercise implementation status data generated on the basis of the exercise type/personalized exercise intensity acquired from the database and generate an exercise implementation status analysis result indicating whether or not exercise being executed by the user is an exercise according to a pre-generated exercise prescription, in which the generated exercise implementation status analysis result is output to a display unit.

By the present configuration, a configuration that enables a rehabilitation execution user to appropriately execute rehabilitation while confirming whether or not exercise being executed by the user is an appropriate exercise according to an exercise prescription is achieved.

Note that the effects described in the present specification are merely examples and are not limited, and other effects may be obtained.

BRIEF DESCRIPTION OF DRAWINGS

Fig. 1 is a diagram for describing an outline of processing of the present disclosure.
Fig. 2 is a diagram for describing the outline of processing of the present disclosure.
Fig. 3 is a diagram for describing the outline of processing of the present disclosure.
Fig. 4 is a diagram for describing the outline of processing of the present disclosure.
Fig. 5 is a diagram for describing data applied to the processing of the present disclosure.
Fig. 6 is a diagram for describing data applied to the processing of the present disclosure.
Fig. 7 is a diagram for describing data applied to the processing of the present disclosure.
Fig. 8 is a diagram for describing data applied to the processing of the present disclosure.
Fig. 9 is a diagram for describing processing executed by a user terminal or the like during rehabilitation execution in a hospital.
Fig. 10 is a diagram for describing an example of data stored in an exercise type corresponding standard exercise intensity database.
Fig. 11 is a diagram for describing an example of data stored in an exercise type corresponding user-specific exercise intensity database.
Fig. 12 is a diagram for describing processing executed by a user terminal or the like during rehabilitation execution after discharge from a hospital.
Fig. 13 is a diagram for describing an example of data stored in an exercise implementation status record database.
Fig. 14 is a diagram for describing an example of data stored in an exercise prescription information database.
Fig. 15 is a diagram for describing an example of display data displayed on a user terminal during rehabilitation execution after discharge from a hospital.
Fig. 16 is a diagram illustrating a flowchart for describing a sequence of processing which a user terminal executes during rehabilitation execution in a hospital.
Fig. 17 is a diagram for describing an example of data processing during rehabilitation execution using AEROBIKE (registered trademark).
Fig. 18 is a flowchart for describing a sequence of data processing during rehabilitation execution using AEROBIKE (registered trademark).
Fig. 19 is a diagram illustrating a flowchart for describing a sequence of processing which a user terminal executes during rehabilitation execution after discharge from a hospital.
Fig. 20 is a diagram for describing processing executed by a user terminal and a hospital terminal in a second embodiment.
Fig. 21 is a diagram illustrating a flowchart for describing a sequence of processing executed by a user terminal in the second embodiment.
Fig. 22 is a diagram illustrating a flowchart for describing a sequence of processing executed by a hospital terminal in the second embodiment.
Fig. 23 is a diagram for describing processing executed by a user terminal and a hospital terminal in a third embodiment.
Fig. 24 is a diagram for describing an example of data stored in an exercise prescription information database used in the third embodiment.
Fig. 25 is a diagram for describing an example of display data displayed on a user terminal in the third embodiment.
Fig. 26 is a diagram illustrating a flowchart for describing a sequence of processing executed by the user terminal in the third embodiment.

Fig. 27 is a diagram for describing an example of display data displayed on a user terminal in a fourth embodiment.

Fig. 28 is a diagram for describing processing executed by a user terminal and a geographic information providing server in the fourth embodiment.

Fig. 29 is a diagram for describing an example of data stored in the geographic information database used in the fourth embodiment.

Fig. 30 is a diagram for describing processing executed by a user terminal and a hospital terminal in a fifth embodiment.

Fig. 31 is a diagram for describing an example of an exercise implementation status record database used in the fifth embodiment.

Fig. 32 is a diagram for describing an example of an exercise prescription information database used in the fifth embodiment.

Fig. 33 is a diagram for describing an example of exercise prescription information used in the fifth embodiment.

Fig. 34 is a diagram for describing an example of the exercise prescription information used in the fifth embodiment.

Fig. 35 is a diagram for describing an example of the exercise prescription information used in the fifth embodiment.

Fig. 36 is a diagram for describing an example of the exercise prescription information used in the fifth embodiment.

Fig. 37 is a diagram for describing an example of the exercise prescription information used in the fifth embodiment.

Fig. 38 is a diagram for describing an example of display data displayed on a user terminal in the fifth embodiment.

Fig. 39 is a diagram for describing a hardware configuration example of the information processing apparatus of the present disclosure.

MODE FOR CARRYING OUT THE INVENTION

Hereinafter, details of an information processing apparatus, an information processing system, an information processing method, and a program of the present disclosure will be described with reference to the drawings. Note that the description will be made according to the following items.

1. Outline of rehabilitation
2. Outline of processing of the present disclosure
3. Description of data used in processing of the present disclosure
4. (First Embodiment) Details of configuration of user terminal and processing to be executed

    4-1. (First phase) Details of rehabilitation analysis data acquisition phase in hospital
    4-2. (Second phase) Processing of rehabilitation execution phase outside hospital

5. Sequence of processing executed by user terminal

    5-1. (First phase) Sequence of processing executed by user terminal in rehabilitation analysis data acquisition phase in hospital
    5-2. (Second phase) Sequence of processing executed by user terminal in rehabilitation execution phase outside hospital

6. (Second embodiment) Embodiment of performing communication between user terminal and hospital terminal
7. Other embodiments

    7-1. (Third embodiment) Embodiment of urging electrocardiogram (ECG) measurement on the basis of analysis result of exercise load
    7-2. (Fourth embodiment) Embodiment of presenting route information of walking or running using map
    7-3. (Fifth embodiment) Embodiment of performing exercise prescription update processing

8. Hardware configuration example of information processing apparatus
9. Summary of configuration of the present disclosure

[1. Outline of rehabilitation]

First, an outline of general rehabilitation will be described

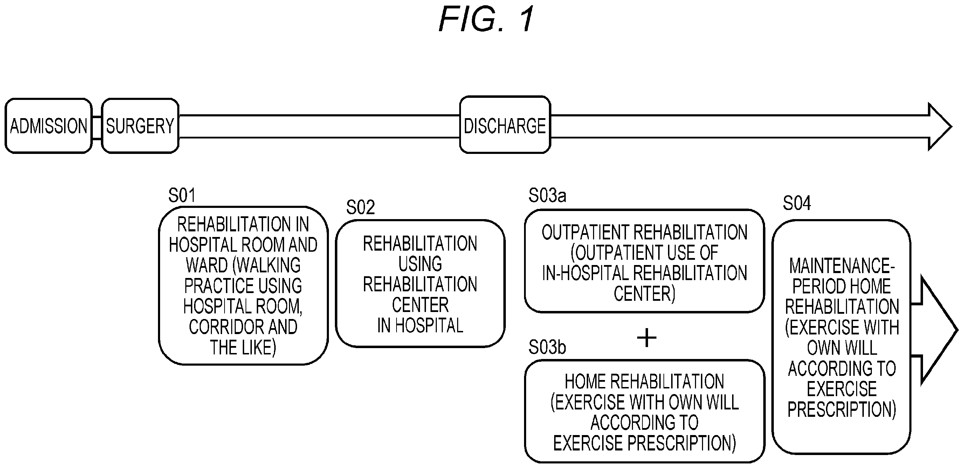

Fig. 1 is a diagram for describing a general flow of rehabilitation.

For example, a user (patient) who has developed a heart disease such as myocardial infarction is hospitalized in a hospital, and then surgery is performed. If the surgery is successful, then rehabilitation (hereinafter described as "rehabilitation") starts.

First, in step S01, a user (patient) performs rehabilitation in a hospital room and a ward. This is rehabilitation focusing on walking practice and the like using a hospital room, a corridor near the hospital room, and the like.

Next, in step S02, a user (patient) performs rehabilitation using an in-hospital rehabilitation center. This is performed using various rehabilitation instruments installed in a rehabilitation center.

The rehabilitation so far is rehabilitation performed in a hospital, and is rehabilitation performed under monitoring of experts.

Thereafter, a user (patient) is discharged from the hospital. After being discharged from the hospital, a user (patient) executes both outpatient rehabilitation in step S03a and home rehabilitation in step S03b.

The outpatient rehabilitation is, for example, rehabilitation performed once or twice a week using various rehabilitation instruments installed in the rehabilitation center.

The home rehabilitation is, for example, rehabilitation in which exercise at home, walking outside, running, or the like is performed on a day other than the outpatient rehabilitation.

Thereafter, in accordance with a recovery status of a user (patient), the outpatient rehabilitation in step S03a ends, and maintenance period home rehabilitation starts in step S04.

Similarly to the home rehabilitation, the maintenance period home rehabilitation is rehabilitation in which exercise at home or walking, running, or the like outside is performed.

During each of these rehabilitation steps, the home rehabilitation in step S03b and the maintenance period home rehabilitation in step S04 are performed in accordance with an exercise prescription designated by a hospital, but an exercise type, a duration of exercise, and the like are finally determined by the intention of a user (patient) himself/herself.

As described above, in a case where rehabilitation is performed by determining the exercise type, the duration of exercise, and the like according to the intention of the user, a case where excessive exercise is performed or a case where too little exercise is performed occurs, and there has been a high possibility that appropriate rehabilitation is not executed.

[2. Outline of processing of the present disclosure]

Next, an outline of processing of the present disclosure will be described.

The present disclosure solves the above problem, and makes it possible to execute the home rehabilitation in step S03b illustrated in Fig. 1 or the maintenance period home rehabilitation in step S04, that is, rehabilitation executed under user management in a state without monitoring of an expert as rehabilitation involving an optimal amount of exercise.

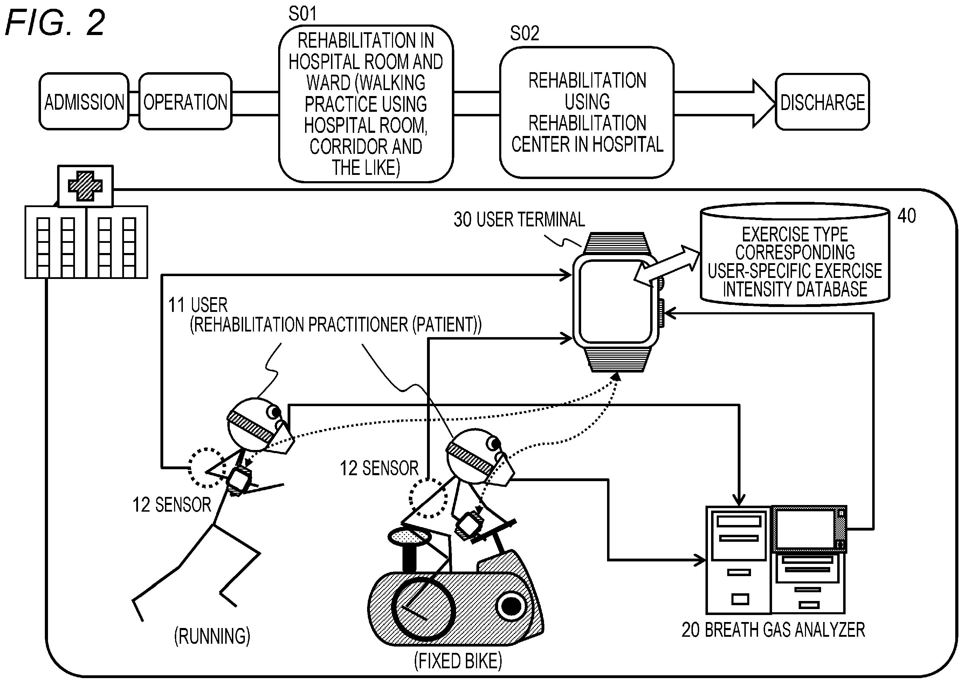

An outline of processing of the present disclosure will be described with reference to Fig. 2 and subsequent drawings.

The processing of the present disclosure includes the following two phases.

(First phase) Data acquisition phase for rehabilitation analysis in hospital.
(Second phase) Rehabilitation execution phase outside hospital.

Fig. 2 is a diagram for describing a "(first phase) rehabilitation analysis data acquisition phase in a hospital".

This first phase is executed in rehabilitation in a hospital room and a ward in step S01 or the rehabilitation using the in-hospital rehabilitation center in step S02 described with reference to Fig. 1.

Fig. 2 illustrates a user (patient) 11 who runs and uses a fixed bike (for example, AEROBIKE (registered trademark)). Note that these are the same one user 11. This indicates that different exercises are performed at different timings.

The user (patient) 11 performs exhaled gas analysis using an exhaled gas analyzer 20 while executing rehabilitation.

Furthermore, a user terminal 30 is worn, and sensor detection information of a sensor 12 worn on a body or the like of the user 11, for example, a sensor 12 such as an acceleration sensor, a gyro, a geomagnetic sensor, or an atmospheric pressure sensor is input to the user terminal 30. Note that the user terminal 30 is, for example, a wearable device, a wristwatch type band, a smartphone, a head mount display (HMD), or the like.

The user terminal 30 analyzes the exhaled gas analysis result acquired using the exhaled gas analyzer 20 or the sensor detection information acquired via the sensor 12, generates exercise intensity information corresponding to various user-specific exercise types corresponding to the individual user (patient) 11, and stores the generated data in an "exercise type corresponding user-specific exercise intensity database 40" in the user terminal 30.

The user-specific exercise intensity according to various exercise types performed by the user (patient) 11, for example, various exercise types such as walking (walking speed = 4.0 km/h on flatland walking), running (speed = 7.0 km/h on flatland running), and AEROBIKE (registered trademark) (load amount = 120 W) is recorded in the exercise type corresponding user-specific exercise intensity database 40.

Note that the exercise intensity is, specifically, for example, metabolic equivalents (METs).

The METs, which are exercise intensity index values indicate how many times each exercise of various exercise types such as walking (walking speed = 4.0km/h on flatland walking), running (speed = 7.0km/h on flatland running), and AEROBIKE (registered trademark) (load amount = 120 W) consumes calories (= activity amount) at rest by taking rest as METs = 1.

For example, METs according to various types of exercise such as METs = 3.0 when walking slowly and METs = 5.2 when climbing stairs are calculated and published. In a hospital, a rehabilitation program is often generated using these METs.

However, the published values of the METs are only standard values, and the exercise intensity is felt differently by individuals.

Therefore, in the system of the present disclosure, the exercise intensity corresponding to the exercise type according to the individual user (patient) 11 who performs the rehabilitation is calculated in the user terminal 30, and the calculated value is recorded in the "exercise type corresponding user-specific exercise intensity database 40".

That is, in the "(First phase) rehabilitation analysis data acquisition phase in a hospital" illustrated in Fig. 2, the "exercise type corresponding user-specific exercise intensity database 40" is generated and recorded in a storage unit in the user terminal 30.

When the "(First phase) rehabilitation analysis data acquisition phase in a hospital" illustrated in Fig. 2 ends, the (second phase) rehabilitation execution phase outside a hospital is performed.

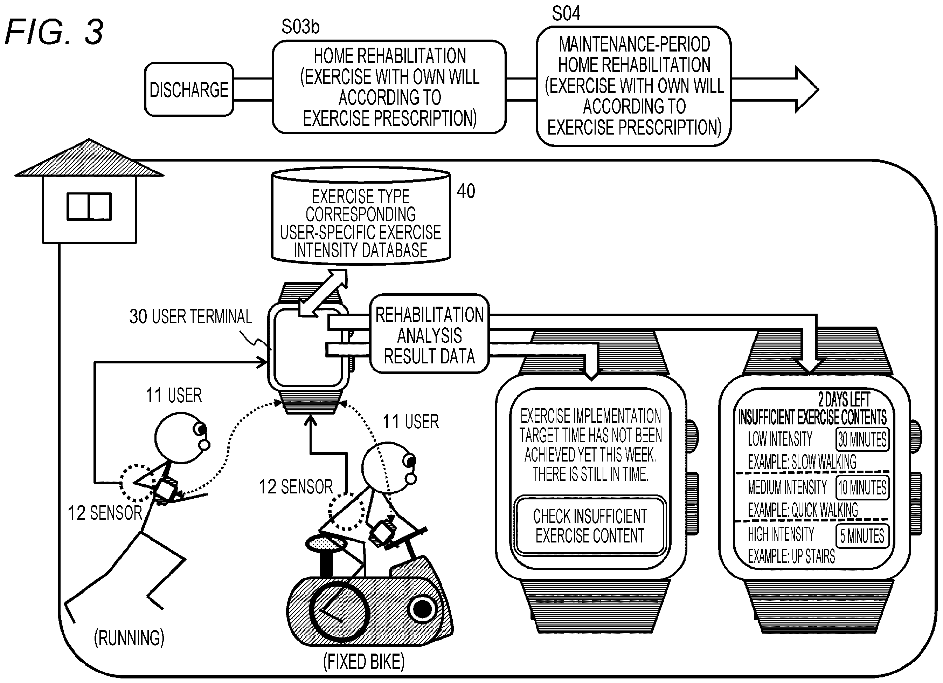

Fig. 3 is a diagram for describing a "(Second phase) rehabilitation execution phase in a hospital".

This second phase is executed in the home rehabilitation in step S03b described with reference to Fig. 1 or the maintenance period home rehabilitation in step S04.

Note that, at the start of this (second phase), the exercise type corresponding user-specific exercise intensity database 40 has been recorded in the user terminal 30.

The exercise type corresponding user-specific exercise intensity database 40 stores exercise intensity data corresponding to an exercise type according to an individual of a user (patient) 11 who performs rehabilitation.

Fig. 3 illustrates the user (patient) 11 who runs and uses AEROBIKE (registered trademark). Note that these are the same one user 11. This indicates that different exercises are performed at different timings.

The user 11 who is a rehabilitation performer wears the user terminal 30, and inputs the sensor detection information of the sensor 12 worn on the body or the like of the user 11, for example, the sensor 12, such as an acceleration sensor, a gyro, a geomagnetic sensor, or an atmospheric pressure sensor, to the user terminal 30.

The data processing unit of the user terminal 30 analyzes the sensor detection information acquired via the sensor 12, and estimates a type of exercise being executed by the user (patient) 11. Further, the user-specific exercise intensity (METs) corresponding to the estimated exercise type is acquired by referring to the exercise type corresponding user-specific exercise intensity database 40.

Furthermore, the data processing unit of the user terminal 30 uses the value of the user-specific exercise intensity (METs) corresponding to the estimated exercise type to analyze whether or not the exercise being executed by the user 11 conforms to an exercise prescription (rehabilitation program) created by a hospital, and displays the analysis result on the display unit of the user terminal 30.

Specifically, a display of a message or analysis data as illustrated in Fig. 3 is executed. For example, the following information is displayed.

(a) Exercise execution target time has not been achieved yet this week. There is still in time.
(b) Insufficient exercise contents

Low intensity = for example, slow walking (30 min)
Medium intensity = for example, quick walking (10 min)
High intensity = for example, climbing stairs (5 min)

The low intensity to high intensity are classification information of "exercise intensity". A time such as 30 minutes indicates an insufficient time.

Note that the above display information is displayed on the basis of an analysis result on a weekly basis or a daily basis, for example.

The user 11 can confirm an appropriate amount of exercise to be executed as rehabilitation by viewing the display information, and can reliably perform rehabilitation with an appropriate amount of exercise.

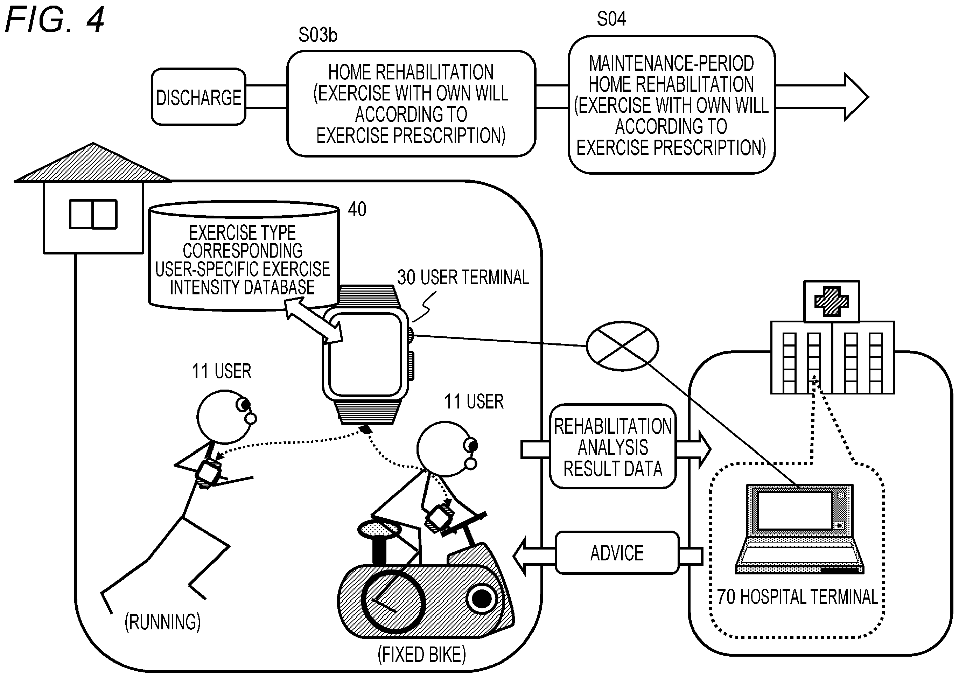

Fig. 4 is a diagram for describing another different processing example of the "(Second phase) rehabilitation execution phase outside a hospital".

Similarly to Fig. 3, the processing illustrated in Fig. 4 is also executed in the processing in the second phase, that is, the home rehabilitation in step S03b described with reference to Fig. 1 or the maintenance period home rehabilitation in step S04.

The example illustrated in Fig. 4 is a processing example in which the user terminal 30 and the hospital terminal 70 communicate with each other.

The user 11 who is a rehabilitation performer wears the user terminal 30, and inputs the sensor detection information of the sensor 12 worn on the body or the like of the user 11, for example, the sensor 12, such as an acceleration sensor, a gyro, a geomagnetic sensor, or an atmospheric pressure sensor, to the user terminal 30.

The data processing unit of the user terminal 30 transmits the sensor detection information acquired via the sensor 12 as it is or the analysis result to the hospital terminal 70.

The hospital terminal 70 first executes data analysis processing similar to the processing executed in the user terminal 30 described above with reference to Fig. 3.

Furthermore, advice information or the like for the user 11 is generated on the basis of the analysis result and transmitted to the user terminal 30.

The user terminal 30 displays the advice information received from the hospital terminal 70. The user 11 can confirm whether or not appropriate rehabilitation is being performed on the basis of the display information.

[3. Description of data used in processing of the present disclosure]

Next, data used in the processing of the present disclosure will be described.

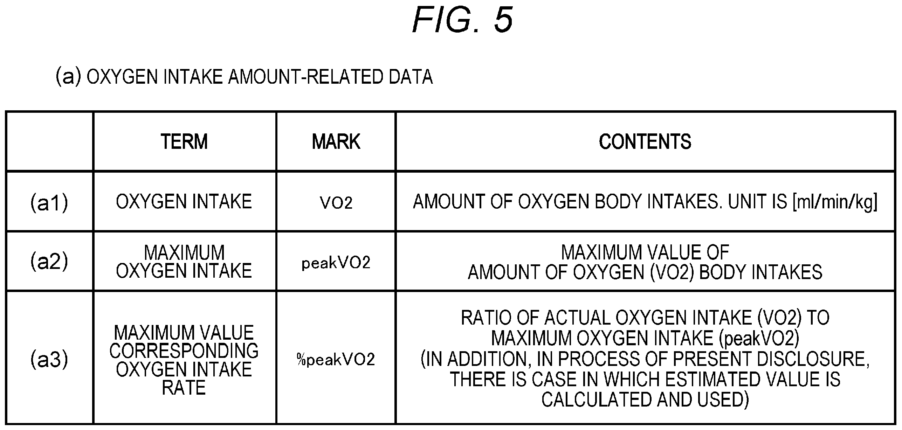

Hereinafter, each data of the following categories (a) to (d) will be sequentially described with reference to Figs. 5 to 7.

    (a) Oxygen intake-related data
    (b) Exercise intensity-related data
    (c) Activity amount-related data
    (d) Other data

(a) Oxygen intake-related data

First, (a) oxygen intake-related data will be described with reference to Fig. 5. The oxygen intake-related data includes the following data:

    (a1) oxygen intake amount (VO2)
    (a2) maximum oxygen intake (peakVO2)
    (a3) maximum value corresponding oxygen intake rate (% peakVO2)

    (a1) The oxygen intake (VO2) is the amount of oxygen taken in by a body, and the unit is [ml/min/kg]. This is a value in units of 1 minute and 1 kg of a body weight.
    (a2) The maximum oxygen intake (peakVO2) is a maximum value of the amount of oxygen (oxygen intake (VO2)) taken in by the body. Specifically, the maximum oxygen intake is, for example, the maximum oxygen intake when intense exercise is performed.
    (a3) The maximum value corresponding oxygen intake rate (% peakVO2) is a ratio of the actual oxygen intake (VO2) to the maximum oxygen intake (peakVO2).

Note that, in the processing of the present disclosure, there is a case where an estimated value is calculated and used.

(b) Exercise intensity-related data

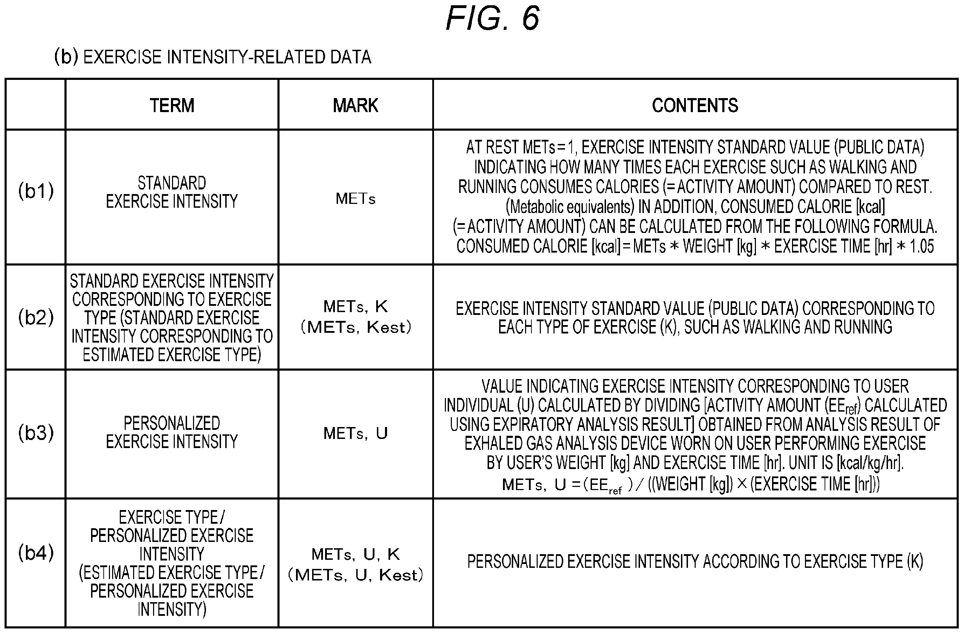

Next, (b) the exercise intensity-related data will be described with reference to Fig. 6. (b) The exercise intensity-related data includes the following data:

    (b1) standard exercise intensity (METs)
    (b2) exercise type corresponding standard exercise intensity (METs, K)
    (b3) personalized exercise intensity (METs, U), and
    (b4) exercise type/personalized exercise intensity (METs, U, K).

As described above, the exercise intensity is specifically, for example, metabolic equivalents (METs).

(b1) The standard exercise intensity (METs) indicates how many times each exercise such as walking (walking speed = 4.0km/h on flatland walking), running (speed = 7.0km/h on flatland running), and AEROBIKE (registered trademark) (load amount = 120 W) consumes calories (= activity amount) at rest by taking rest as METs = 1.

This standard exercise intensity (METs) is published data.

Note that consumed calorie [kcal] (= activity amount) can be calculated by the following Equation.

```
Consumed calorie [kcal] = (METs) × (weight [kg]) × (exercise
time [hr]) × 1.05
```

(b2) The exercise type corresponding standard exercise intensity (METs, K) is an exercise intensity standard value corresponding to each of various exercise types (K) such as walking (walking speed = 4.0 km/h in flatland walking), running (speed = 7.0 km/h in flatland running), and AEROBIKE (registered trademark) (load amount = 120 W). This is also published data.

Note that (K) indicates a value corresponding to the exercise type.

Furthermore, in the processing of the present disclosure, the type of exercise executed by the user is estimated on the basis of detection information of a sensor worn on the user who performs exercise as rehabilitation.

The estimated exercise type is expressed by (Kest), and the estimated exercise type corresponding standard exercise intensity corresponds to this estimated exercise type is expressed by (METs, Kest) .

In the following description, note that the exercise type (K) includes not only the confirmed exercise type but also the estimated exercise type (Kest).

(b3) The personalized exercise intensity (METs, U) is a value indicating an exercise intensity corresponding to a user individual (U) calculated by dividing the "exhalation analysis result use calculated activity amount (EEref)" obtained from the analysis result of the exhaled gas analyzer worn on the user who performs the exercise by a weight [kg] of a user and an exercise time [hr]. The unit is [kcal/kg/hr].

```
METs, U = (EEref)/((weight [kg]) × (exercise time [hr])).
```

Note that (U) indicates a user-specific value corresponding to a specific user.

(b4) The exercise type/personalized exercise intensity (METs, U, K) is a personalized exercise intensity (METs, U) according to the exercise type (K).

As described above, in the processing of the present disclosure, the exercise type executed by the user is estimated on the basis of detection information of a sensor worn on the user who performs exercise as rehabilitation.

The estimated exercise type is expressed by (Kest), and the estimated exercise type/personalized exercise intensity corresponding to this estimated exercise type is expressed by (METs, U, Kest).

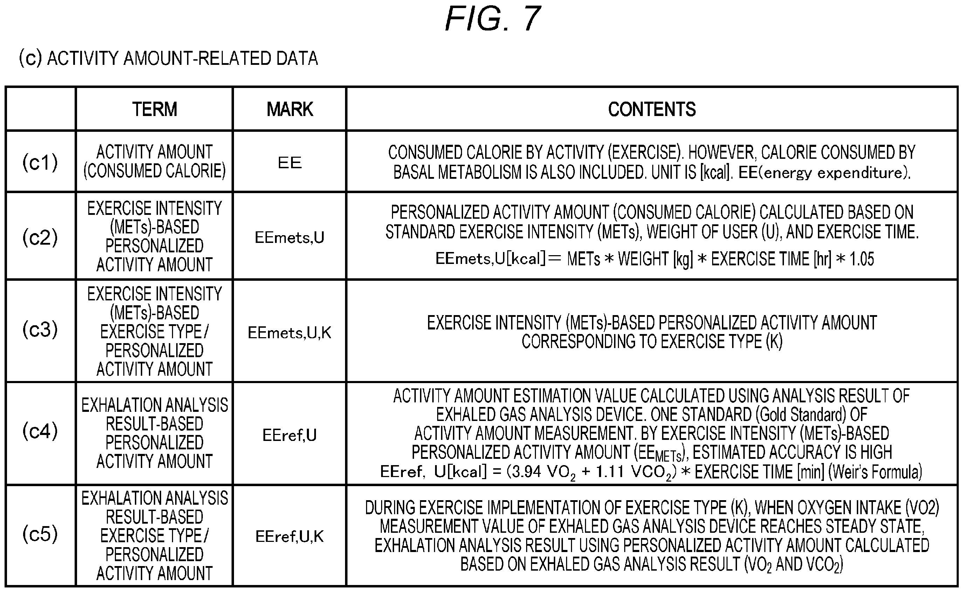

Next, (c) The activity amount-related data will be described with reference to Fig. 7. (c) The activity amount-related data includes the following data:

(c1) activity amount (consumed calorie) (EE),
(c2) exercise intensity (METs)-based personalized activity amount (EEmets, U),
(c3) exercise intensity (METs)-based exercise type/personalized activity amount (EEmets, U, K),
(c4) exhalation analysis result-based personalized activity amount (EEref, U), and
(c5) exhalation analysis result-based exercise type/personalized activity amount (EEref, U, K).
(c1) The activity amount (consumed calorie) (EE) is the consumed calorie by the activity (exercise). However, calorie consumed by basal metabolism is also included.

The unit is [kcal].

Note that EE means (energy expenditure).

(c2) The exercise intensity (METs)-based personalized activity amount (EEmets, U) is a personalized activity amount (consumed calorie) calculated on the basis of the standard exercise intensity (METs), the weight of the user (U), and the exercise time.

```
EEmets, U [kcal] = (METs) × (weight [kg]) × (exercise time [hr])
× 1.05
```

This is calculated by the above Equation.

(c3) The exercise intensity (METs)-based exercise type/personalized activity amount (EEmets, U, K) is an exercise intensity (METs)-based personalized activity amount corresponding to the exercise type (K).

(c4) The exhalation analysis result-based personalized activity amount (EEref, U) is an activity amount estimation value calculated using the analysis result of the exhaled gas analyzer.

This is one of criteria of activity amount measurement (Gold Standard), and the estimation accuracy is higher than the exercise intensity (METs)-based personalized activity amount (EEmets, U).

$$\text{EEref, U [kcal]} = (3.94 \ \text{VO2} + 1.11 \ \text{VCO2}) \times (\text{exercise time [min]})$$
$$\text{(Weir's Equation)}$$

This is calculated by the above Equation.

Note that VO2 is the amount of oxygen, and VCO2 is the amount of carbon dioxide.

(c5) The exhalation analysis result-based exercise type/personalized activity amount (EEref, U, K) is the exhalation analysis result used personalized activity amount calculated on the basis of the exhaled gas analysis result (VO2 and VCO2) when the measurement value of the oxygen intake (VO2) of the exhaled gas analyzer reaches a steady state during the exercise execution of the exercise type (K).

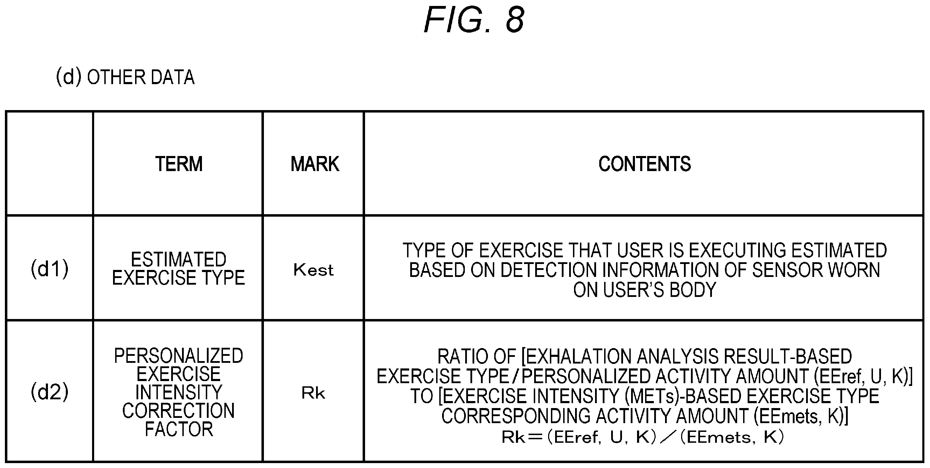

Next, (d) other data will be described with reference to Fig. 8. (d) Other data includes the following data:

(d1) estimated exercise type (Kest), and
(d2) personalized exercise intensity correction factor (Rk).

(d1) The estimated exercise type (Kest) is a type of an exercise being executed by a user estimated on the basis of the detection information of the sensor worn on the body of the user.

(d2) The personalized exercise intensity correction factor (Rk) is a ratio (proportion) of "exhalation analysis result-based exercise type/personalized activity amount (EEref, U, K)" to "exercise intensity (METs)-based exercise type corresponding activity amount (EEmets, U, K)".

$$Rk = (\text{EEref, U, K})/(\text{EEmets, U, K})$$

This is calculated by the above Equation.

[4. (First Embodiment) Details of configuration of user terminal and processing to be executed]

Next, as a first embodiment of the present disclosure, a configuration of the user terminal 30 and details of processing to be executed will be described.

As described with reference to Figs. 2 to 4, the user terminal 30 is the user terminal 30 worn on the body by the user 11 who executes rehabilitation.

As described above, the processing of the present disclosure includes the following two phases:

(First phase) Data acquisition phase for rehabilitation analysis in hospital.
(Second phase) Rehabilitation execution phase outside hospital.

Hereinafter, details of processing executed in these two phases will be sequentially described.

[4-1. (First phase) Details of rehabilitation analysis data acquisition phase in hospital]

First, processing executed in the "(First phase) rehabilitation analysis data acquisition phase in hospital" will be described.

This first phase is executed in rehabilitation in a hospital room and a ward in step S01 or rehabilitation using an in-hospital rehabilitation center in step S02 as described with reference to Fig. 2.

The user (patient) 11 performs exhaled gas analysis using an exhaled gas analyzer 20 while executing rehabilitation.

Furthermore, a user terminal 30 is worn, and sensor detection information of a sensor 12 worn on a body or the like of the user 11, for example, a sensor 12 such as an acceleration sensor, a gyro, a geomagnetic sensor, or an atmospheric pressure sensor is input to the user terminal 30.

The user terminal 30 analyzes the exhaled gas analysis result acquired using the exhaled gas analyzer 20 or the sensor detection information acquired via the sensor 12, generates exercise intensity information corresponding to various user-specific exercise types corresponding to the individual user (patient) 11, and stores the generated data in the user terminal 30.

This is the "exercise type corresponding user-specific exercise intensity database 40" illustrated in Fig. 2.

The user-specific exercise intensities corresponding to various exercise types performed by the user (patient) 11, for example, various exercise types such as walking (walking speed = 4.0 km/h on flatland walking), running (speed = 7.0 km/h on flatland running), and AEROBIKE (registered trademark) (load amount = 120 W) are recorded in the exercise type corresponding user-specific exercise intensity database 40.

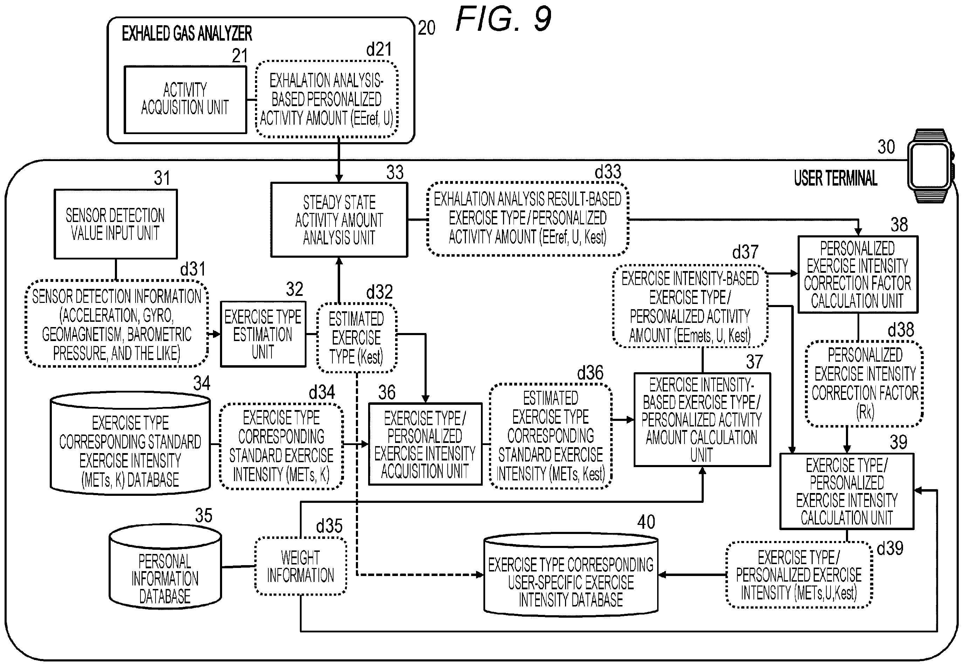

Fig. 9 is a diagram for explaining the configurations and the processing of the user terminal 30 and the exhaled gas analyzer 20 that execute the processing of the "(First phase) rehabilitation analysis data acquisition phase in a hospital".

The exhaled gas analyzer 20 is connected to a mask attached to a mouth of the user 11 who executes exercise as rehabilitation, and analyzes the exhalation of the user 11.

As illustrated in Fig. 9, the exhaled gas analyzer 20 includes an activity amount acquisition unit 21. The activity amount acquisition unit 21 analyzes the exhalation of the user 11, generates an "exhalation analysis result-based personalized activity amount (EEref, U) d21", and outputs the generated "exhalation analysis result-based personalized activity amount (EEref, U) d21" to the user terminal 30.

As described above, the exhalation analysis result-based personalized activity amount (EEref, U) is an activity amount estimation value calculated using the analysis result of the exhaled gas analyzer.

This is one of criteria of activity amount measurement (Gold Standard), and the estimation accuracy is higher than the exercise intensity (METs)-based personalized activity amount (EEmets, U).

$$\text{EEref, U [kcal]} = (3.94 \text{ VO2} + 1.11 \text{ VCO2}) \times (\text{exercise time [min]})$$
$$(\text{Weir's Equation})$$

This is calculated by the above Equation.

The user terminal 30 is a terminal worn on the body while the user 11 who is a rehabilitation performer is performing exercise as rehabilitation.

The sensor detection information input unit 31 of the user terminal 30 acquires sensor detection information d31 of a sensor worn on the body of the user 11, for example, a sensor such as an acceleration sensor, a gyro, a geomagnetic sensor, or an atmospheric pressure sensor, and outputs the sensor detection information d31 to the exercise information estimation unit 32.

Note that d of the reference numeral d31 means data.

The exercise information estimation unit 32 estimates the exercise type of the user 11 on the basis of the input sensor detection information d31. For example, the type of exercise being executed by the user 11 is estimated, such as walking (walking speed = 4.0 km/h on flatland walking), running (running speed = 7.0 km/h on flatland running), and AEROBIKE (registered trademark) (load amount = 120 W).

The exercise information estimation unit 32 outputs the estimated exercise type (Kest) as the estimation result to the steady state activity amount analysis unit 33 and the exercise type corresponding standard exercise intensity acquisition unit 36.

Further, the estimated exercise type (Kest) is also output to the exercise type corresponding user-specific exercise intensity database and registered in the database.

The steady state activity amount analysis unit 33 inputs the following data:

(a) exhalation analysis result-based personalized activity amount (EEref, U) d21 generated by the activity amount acquisition unit 21, and
(b) estimated exercise type (Kest) d32 indicating an exercise type estimated by the exercise information estimation unit 32 and being executed by the user.

The steady state activity amount analysis unit 33 generates the exhalation analysis result-based exercise type/personalized activity amount (EEref, U, Kest) d33 on the basis of the pieces of data.

Note that since the exercise type (K) is an estimation result, and therefore is indicated as (Kest).

As described above with reference to Fig. 7, the exhalation analysis result-based exercise type/personalized activity amount (EEref, U, K) is the exhalation analysis result used personalized activity amount calculated on the basis of the exhaled gas analysis result (VO2 and VCO2) when the measurement value of the oxygen intake (VO2) of the exhaled gas analyzer reaches a steady state during the exercise execution of the exercise type (K).

The exhalation analysis result-based exercise type/personalized activity amount (EEref, U, K) is calculated as follows: EEref, U, K [kcal] = (3.94 VO2 + 1.11 VCO2) × (exercise time [min]) (Weir's Equation).

Note that VO2 is the amount of oxygen, and VCO2 is the amount of carbon dioxide.

The estimation accuracy of the exhalation analysis result-based exercise type/personalized activity amount (EEref, U, K) is higher than that of the exercise intensity (METs)-based exercise type corresponding activity amount (EEmets, U, K).

The steady state activity amount analysis unit 33 outputs the calculated exhalation analysis result-based exercise type/personalized activity amount (EEref, U, Kest) d33 to the personalized exercise intensity correction factor calculation unit 37.

Next, the processing executed by the exercise type corresponding standard exercise intensity acquisition unit 36 which is another output destination of the estimated exercise type (Kest) d32 generated by the exercise information estimation unit 32 will be described.

The exercise type corresponding standard exercise intensity acquisition unit 36 searches the exercise type corresponding standard exercise intensity (METs, K) database 34 on the basis of the estimated exercise type (Kest) d32 input from the exercise information estimation unit 32, and acquires the standard exercise intensity (METs, K) registered in association with the estimated exercise type (Kest). The exercise type corresponding standard exercise intensity (METs, K) d34 illustrated in Fig. 9 is acquired.

Note that, although the exercise type corresponding standard exercise intensity (METs, K) database 34 is illustrated as a part of the configuration of the user terminal 30 in Fig. 9, the exercise type corresponding standard exercise intensity (METs, K) database 34 may be a database of an external server, and in this case, the user terminal 30 accesses the external server via the communication unit to acquire the exercise type corresponding standard exercise intensity (METs, K) d34.

Values of standard exercise intensity (METs) associated with various exercise types (walking speed = 4.0 km/h on flatland walking), running (speed = 7.0 km/h on flatland running), and a fixed bike (for example, AEROBIKE (registered trademark)) (load amount = 120 W) are registered in the exercise type corresponding standard exercise intensity (METs, K) database 34.

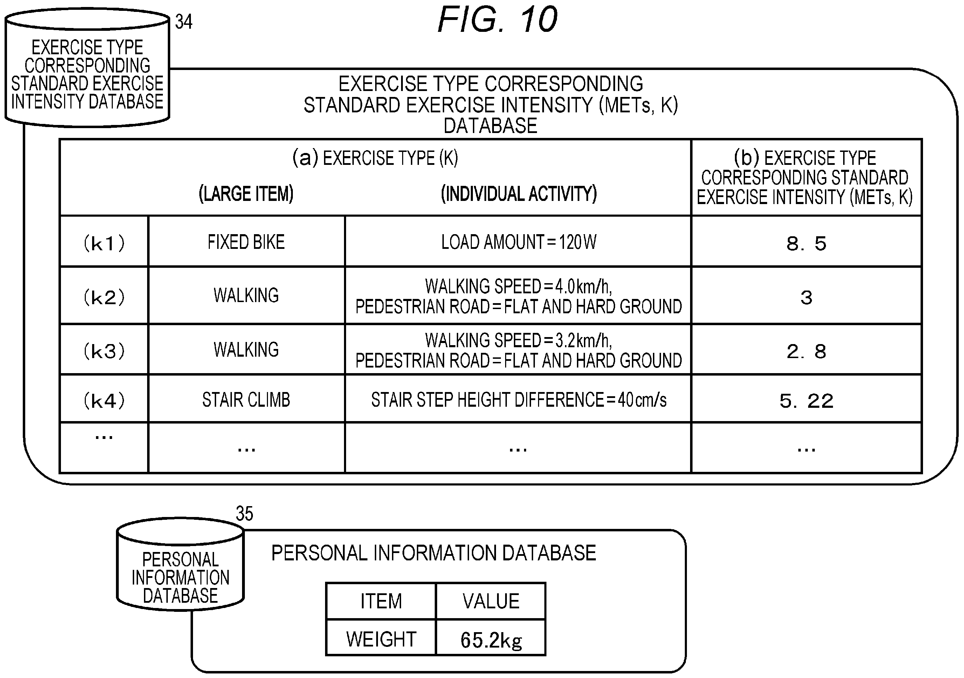

Fig. 10 illustrates an example of registration data of the exercise type corresponding standard exercise intensity (METs, K) database 34.

As illustrated in Fig. 10, the following data is registered in the exercise type corresponding standard exercise intensity (METs, K) database 34 in association with each other.

    (a) Exercise type (K)
    (b) Exercise type corresponding standard exercise intensity (METs, K).

Note that (a) the exercise type (K) includes (large items) such as walking, running, and a fixed bike (for example, AEROBIKE (registered trademark)), and (individual activities) describing detailed information such as a speed of walking or running, a state of a road surface on which walking or running are performed, and a load of a fixed bike (for example, AEROBIKE (registered trademark)).

As described above with reference to Fig. 6, the exercise type corresponding standard exercise intensity (METs, K) is an exercise intensity index value which indicates how many times the exercise type (K) such as walking (walking speed = 4.0km/h on flatland walking), running (speed = 7.0km/h on flatland running), and AEROBIKE (registered trademark) (load amount = 120 W) consumes calories (= activity amount) at rest by taking rest as METs = 1.

The exercise type corresponding standard exercise intensity (METs, K) is published data.

The exercise type corresponding standard exercise intensity acquisition unit 36 illustrated in Fig. 9 searches the exercise type corresponding standard exercise intensity (METs, K) database 34 having the registration data illustrated in Fig. 10 on the basis of the estimated exercise type (Kest) input from the exercise information estimation unit 32, and acquires the standard exercise intensity (METs, K) registered in association with the exercise type (K) that is the same as or the most similar to the estimated exercise type (Kest).

The exercise type corresponding standard exercise intensity acquisition unit 36 outputs the standard exercise intensity (METs, K) acquired from the exercise type corresponding standard exercise intensity (METs, K) database 34 to the exercise intensity-based exercise type/personalized activity amount calculation unit 37 as the "estimated exercise type corresponding standard exercise intensity (METs, Kest) d36" illustrated in Fig. 9.

Note that the "estimated exercise type corresponding standard exercise intensity (METs, Kest) d36" illustrated in Fig. 9 is data based on the estimated exercise type (Rest), and thus is expressed by using (Kest).

The exercise intensity-based exercise type/personalized activity amount calculation unit 37 inputs the following two data:

    (a) the estimated exercise type corresponding standard exercise intensity (METs, Kest) d36 output from the exercise type corresponding standard exercise intensity acquisition unit 36, and
(b) weight information d35 of the user individual acquired from the personal information database 35.

A lower part of Fig. 10 illustrates an example of data recorded in the personal information database 35. As illustrated in Fig. 10, weight information of an individual user is recorded in the personal information database 35.

The exercise intensity-based exercise type/personalized activity amount calculation unit 37 inputs each of the above data (a) and (b) to calculate an exercise intensity-based exercise type/personalized activity amount (EEmets, U, Kest) d37.

As described above with reference to (c3) of Fig. 7, the exercise intensity-based exercise type/personalized activity amount (EEmets, U, K) is an exercise intensity (METs)-based personalized activity amount corresponding to the exercise type (K).

As described above with reference to (c2) of Fig. 7, the exercise intensity (METs)-based personalized activity amount (EEmets, U) is the personalized activity amount (consumed calorie) calculated on the basis of the standard exercise intensity (METs), the weight of the user (U), and the exercise time.

$$\text{EEmets, U [kcal]} = (\text{METs}) \times (\text{weight [kg]}) \times (\text{exercise time [hr]}) \times 1.05$$

This is calculated by the above Equation.

The exercise intensity-based exercise type/personalized activity amount calculation unit 37 uses exercise time information measured by a timer (not illustrated) and the above input information (a) and (b) to calculate the personalized activity amount (consumed calorie) based on Equation, that is, EEmets, U[kcal] = (METs) $\times$ (weight [kg]) $\times$ (exercise time [hr]) $\times$ 1.05.

Note that the activity amount (EE) calculated according to the Equation is a value during execution of a specific exercise type (Kest) by the user, and is calculated as the exercise intensity-based exercise type/personalized activity amount (EEmets, U, Kest) d37.

As illustrated in Fig. 9, the exercise intensity-based exercise type/personalized activity amount calculation unit 37 outputs the calculated exercise intensity-based exercise type/personalized activity amount (EEmets, U, Kest) d37 to the personalized exercise intensity correction factor calculation unit 38.

The personalized exercise intensity correction factor calculation unit 38 inputs each of the following data:

(a) the exhalation analysis result-based exercise type/personalized activity amount (EEref, U, Kest) d33 calculated by the steady state activity amount analysis unit 33, and
(b) the exercise intensity-based exercise type/personalized activity amount (EEmets, U, Kest) d37 calculated by the exercise intensity-based exercise type/personalized activity amount calculation unit 37.

The personalized exercise intensity correction factor calculation unit 38 calculates a personalized exercise intensity correction factor (Rk) d38 on the basis of the two types of input data.

As described above with reference to Fig. 8, the personalized exercise intensity correction factor (Rk) is a ratio (proportion) of "exhalation analysis result-based exercise type/personalized activity amount (EEref, U, K)" to "exercise intensity (METs)-based exercise type corresponding activity amount (EEmets, U, K)".

$$\text{Rk} = (\text{EEref, U, K})/(\text{EEmets, U, K})$$

This is calculated by the above Equation.

The personalized exercise intensity correction factor calculation unit 38 inputs the input data described above, that is, the following data:

(a) the exhalation analysis result-based exercise type/personalized activity amount (EEref, U, Kest) d33 calculated by the steady state activity amount analysis unit 33, and
(b) the exercise intensity-based exercise type/personalized activity amount (EEmets, U, Kest) d37 calculated by the exercise intensity-based exercise type/personalized activity amount calculation unit 37.

The personalized exercise intensity correction factor calculation unit 38 is based on the pieces of data to calculate the personalized exercise intensity correction factor (Rk) to Equation, that is, Rk = (EEref, U, Kest)/(EEmets, Kest).

The personalized exercise intensity correction factor calculation unit 38 outputs the calculated personalized exercise intensity correction factor (Rk) d38 to the exercise type/personalized exercise intensity calculation unit 39.

The exercise type/personalized exercise intensity calculation unit 39 inputs each of the following data:

(a) the personalized exercise intensity correction factor (Rk) d38 calculated by the personalized exercise intensity correction factor calculation unit 38,

(b) the exercise intensity-based exercise type/personalized activity amount d37 calculated by the exercise intensity-based exercise type/personalized activity amount calculation unit 37, and

(c) the weight information d35 of the user individual acquired from the personal information database 35.

The exercise type/personalized exercise intensity calculation unit 39 calculates an exercise type/personalized exercise intensity (METs, U, Kest) d39 by using the pieces of input data.

As described above with reference to (b4) of Fig. 6, the exercise type/personalized exercise intensity (METs, U, K) is the personalized exercise intensity (METs, U) according to the exercise type (K).

In addition, as described above with reference to (b3) of Fig. 6, the personalized exercise intensity (METs, U) is a value indicating an exercise intensity corresponding to a user individual (U) calculated by dividing the "exhalation analysis result use calculated activity amount (EEref)" obtained from the analysis result of the exhaled gas analyzer worn on the user who performs the exercise by a weight [kg] of a user and an exercise time [hr]. The unit is [kcal/kg/hr].

$$\text{METs, U} = (\text{EEref})/((\text{weight [kg]}) \times (\text{exercise time [hr]})).$$

On the basis of the above Equation, the exercise type/personalized exercise intensity (METs, U, K) can be indicated as follows: METs, U, K = (EEref, U, K)/((weight [kg] $\times$ (exercise time [hr])) ... (Equation 1).

Further, as described above, the personalized exercise intensity correction factor (Rk) d38 calculated by the personalized exercise intensity correction factor calculation unit 38 is Rk = (EEref, U, K)/(EEmets, U, K) ... (Equation 2).

The following (Equation 3) is derived from (Equation 1) and (Equation 2).

$$\text{METs, U, K} = ((\text{EEmets, U, K}) \times (\text{Rk}))/((\text{weight [kg]}) \times (\text{exercise time [hr]})) \ \text{... (Equation 3)}$$

The exercise type/personalized exercise intensity calculation unit 39 inputs the exercise time information measured by a timer (not illustrated) and the following input data, that is, the following data:

(a) the personalized exercise intensity correction factor (Rk) d38 calculated by the personalized exercise intensity correction factor calculation unit 38,

(b) weight information d35 of the user individual acquired from the personal information database 35.

The exercise type/personalized exercise intensity calculation unit 39 calculates an exercise type/personalized exercise intensity (METs, U, Kest) d39 by using the pieces of input data.

The exercise type/personalized exercise intensity calculation unit 39 is based on the pieces of input data to calculate the exercise type/personalized exercise intensity (METs, U, K) according to the above (Equation 3).

As described above with reference to (b4) of Fig. 6, the exercise type/personalized exercise intensity (METs, U, K) is the personalized exercise intensity (METs, U) according to the exercise type (K).

In addition, as described above with reference to (b3) of Fig. 6, the personalized exercise intensity (METs, U) is a value indicating an exercise intensity corresponding to a user individual (U) calculated by dividing the "exhalation analysis result use calculated activity amount (EEref)" obtained from the analysis result of the exhaled gas analyzer worn on the user who performs the exercise by a weight [kg] of a user and an exercise time [hr]. The unit is [kcal/kg/hr].

$$\text{METs, U} = (\text{EEref})/((\text{weight [kg]}) \times (\text{exercise time [hr]})).$$

The exercise type/personalized exercise intensity (METs, U, K) is user-specific exercise intensity, and is a value different from the published standard exercise intensity (METs) or the exercise type corresponding standard exercise intensity (METs, K).

That is, the exercise intensity is the user-specific exercise intensity calculated by reflecting the exhaled gas analysis result during the actual exercise execution of the user or the weight of the user.

As illustrated in Fig. 9, the exercise type/personalized exercise intensity (METs, U, Kest) d39 calculated by the exercise type/personalized exercise intensity calculation unit 39 is recorded in the exercise type corresponding user-specific exercise intensity database 40.

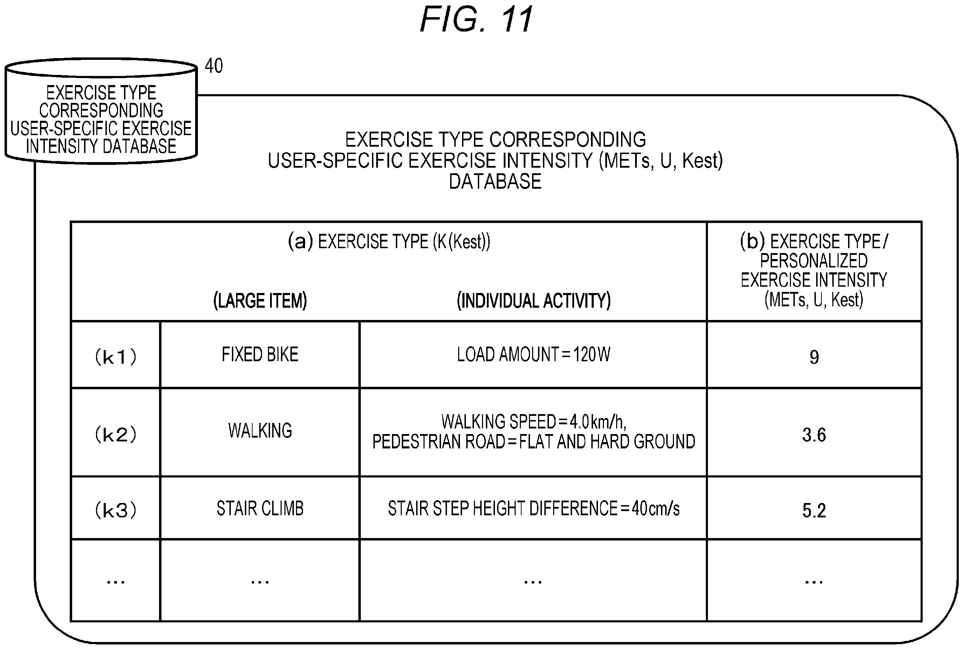

Fig. 11 illustrates an example of registration data of the exercise type corresponding user-specific exercise intensity database 40.

As illustrated in Fig. 11, the following data is registered in the exercise type corresponding user-specific exercise intensity (METs, U, Kest) database 40 in association with each other.

(a) Exercise type (K(Kest))
(b) Exercise type/personalized user-specific exercise intensity (METs, U, Kest).

Note that (a) the exercise type (K (Kest)) includes (large items) such as walking, running, and a fixed bike (for example, AEROBIKE (registered trademark)), and (individual activities) describing detailed information such as a speed of walking or running, a state of a road surface on which walking or running are performed, and a load of a fixed bike (for example, AEROBIKE (registered trademark)).

In addition, since all the data registered in the database is data based on the exercise type estimated by the exercise type estimation unit 32, all the data is indicated as the estimated exercise type (Kest).

The estimated exercise type (Kest) d32 estimated by the exercise type estimation unit 32 illustrated in Fig. 9 is registered in "(a) the exercise type (K (Kest))" in the database of Fig. 11.

As described above, the "(b) the exercise type/personalized user-specific exercise intensity (METs, U, Kest)" registered in the exercise type corresponding user-specific exercise intensity (METs, U, Kest) database 40 is the user-specific exercise intensity calculated by reflecting the exhaled gas analysis result or the weight of the user during the actual exercise.

Therefore, the exercise intensity is the exercise intensity reflecting the actual body state of the user from the exercise type corresponding standard exercise intensity (METs, K) which is the published standard value, and the exercise to be executed in the rehabilitation is programmed and evaluated on the basis of the exercise intensity registered in the database, so the optimal rehabilitation and the planning or evaluation of the exercise can be performed according to the body state of the user.

[4-2. (Second phase) Processing of rehabilitation execution phase outside hospital]

Next, the processing executed in the "(second phase) rehabilitation execution phase outside the hospital" will be described.

This second phase is executed in the home rehabilitation in step S03b as described with reference to Fig. 3 or 4 or the maintenance period home rehabilitation in step S04.

The user (patient) 11 wears the user terminal 30 during the execution of the rehabilitation.

The storage unit of the user terminal 30 records the exercise type corresponding user-specific exercise intensity (METs, U, Kest) database 40 generated in the processing of the "(first phase) rehabilitation analysis data acquisition phase in a hospital" described with reference to Fig. 9.

The database is the exercise type corresponding user-specific exercise intensity (METs, U, Kest) database 40 having the registration data configuration illustrated in Fig. 11. That is, as described with reference to Fig. 11, the database is a database in which the following data is registered in association with each other.

(a) Exercise type (K(Kest))
(b) Exercise type/personalized user-specific exercise intensity (METs, U, Kest).

(Second phase) In the rehabilitation execution phase outside the hospital, the exercise as the rehabilitation executed by the user 11 is evaluated using the data recorded in the database.

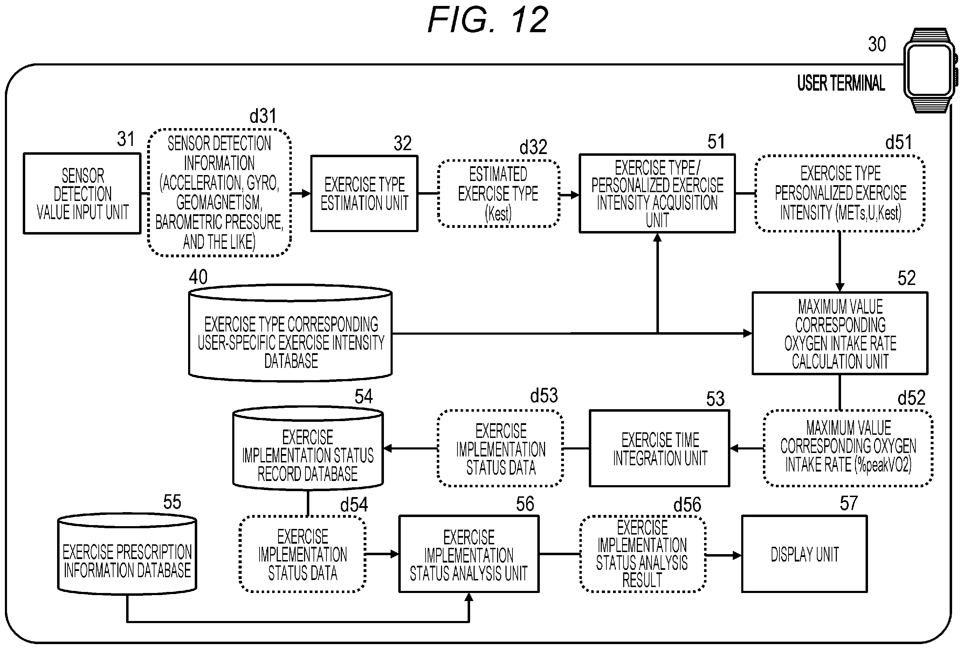

The configuration and processing of the user terminal 30 that executes the processing of the "(second phase) rehabilitation execution phase outside the hospital" will be described with reference to Fig. 12.

The user terminal 30 is a terminal worn on the body while the user 11 who is a rehabilitation performer is performing exercise as rehabilitation.

The sensor detection information input unit 31 of the user terminal 30 acquires sensor detection information d31 of a sensor worn on the body of the user 11, for example, a sensor such as an acceleration sensor, a gyro, a geomagnetic sensor, or an atmospheric pressure sensor, and outputs the sensor detection information d31 to the exercise information estimation unit 32.

The exercise information estimation unit 32 estimates the exercise type of the user 11 on the basis of the input sensor detection information d31. For example, the type of exercise being executed by the user 11 is estimated, such as walking (walking speed = 4.0 km/h on flatland walking), running (running speed = 7.0 km/h on flatland running), and AEROBIKE (registered trademark) (load amount = 120 W).

The exercise type estimation unit 32 outputs the estimated exercise type (Kest) as the estimation result to the exercise type/personalized exercise intensity acquisition unit 51.

The exercise type/personalized exercise intensity acquisition unit 51 searches the exercise type corresponding user-specific exercise intensity (METs, U, Kest) database 40 on the basis of the estimated exercise type (Kest) d32 input from the exercise type estimation unit 32, and acquires the exercise type/personalized user-specific exercise intensity (METs, U, Kest) d51 registered in association with the estimated exercise type (Kest).

The exercise type/personalized user-specific exercise intensity (METs, U, Kest) d51 acquired from the database by the exercise type/personalized exercise intensity acquisition unit 51 is output to a maximum value corresponding oxygen intake rate calculation unit 52.

The maximum value corresponding oxygen intake rate calculation unit 52 acquires, from the exercise type corresponding user-specific exercise intensity (METs, U, Kest) database 40, the maximum value of the personalized user-specific exercise intensity (METs, U, Kest) registered in the database.

The maximum value of the personalized user-specific exercise intensity (METs, U, Kest) registered in the exercise type corresponding user-specific exercise intensity (METs, U, Kest) database 40 is set to be an exercise type user-specific exercise intensity maximum value (METsmax, U, Kest).

The maximum value corresponding oxygen intake rate calculation unit 52 includes:

(a) the exercise type/personalized user-specific exercise intensity (METs, U, Kest) d51 acquired from the exercise type/personalized exercise intensity acquisition unit 51, and
(b) the exercise type user-specific exercise intensity maximum value (METsmax, U, Kest) acquired from the exercise type corresponding user-specific exercise intensity (METs, U, Kest) database 40.

From the pieces of data, the maximum value corresponding oxygen intake rate (% peakVO2) d52 is calculated.

The maximum value corresponding oxygen intake rate (% peakVO2) is calculated by the following (Equation 4).

```
    Maximum value corresponding oxygen intake rate (% peakVO2) =
(Exercise type/personalized user-specific exercise intensity (METs, U,
Kest))/(exercise type user-specific exercise intensity maximum value
(METsmax, U, Kest))  … (Equation 4)
```

That is, the maximum value corresponding oxygen intake rate (% peakVO2) can be calculated by the ratio of the exercise type/personalized user-specific exercise intensity (METs, U, Kest) to the exercise type user-specific exercise intensity maximum value (METsmax, U, Kest).

Note that, as described above with reference to Fig. 5, the maximum oxygen intake (peakVO2) is the maximum value of oxygen amount (oxygen intake (VO2)) consumed by a body, and the maximum value corresponding oxygen intake rate (% peakVO2) is the ratio of the actual oxygen intake (VO2) to the maximal oxygen intake (peakVO2).

The maximum value corresponding oxygen intake rate (% peakVO2) d52 calculated by the maximum value corresponding oxygen intake rate calculation unit 52 is output to an exercise time integration unit 53.

The exercise time integration unit 53 inputs the maximum value corresponding oxygen intake rate (% peakVO2) d52 calculated by the maximum value corresponding oxygen intake rate calculation unit 52, generates exercise implementation status data d53 of the user 11, and records the generated exercise implementation status data d53 in the exercise implementation status record database 54.

The exercise time integration unit 53 calculates, as the exercise implementation status data d53 of the user 11, the exercise time executed by the user 11 in a predetermined section unit of a predefined maximum value corresponding oxygen intake rate (% peakVO2).

The exercise time integration unit 53 generates the exercise implementation status data d53 classified into sections of the predefined maximum value corresponding oxygen intake rate (% peakVO2) for each predefined data generation period (for example, one week, one day, or the like), and records the generated exercise implementation status data d53 in the exercise implementation status record database 54. Note that a timer (not illustrated) is used to measure the exercise time.

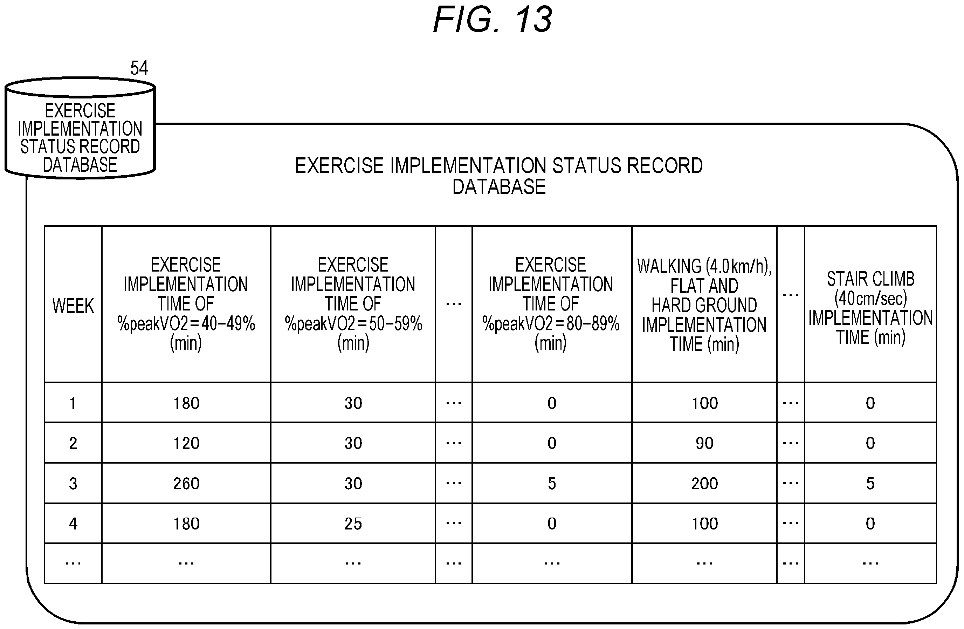

Fig. 13 illustrates an example of data recorded in the exercise implementation status record database 54.

As illustrated in Fig. 13, the exercise time executed by the user 11 is recorded in the exercise implementation status record database 54 in a predetermined section unit of the maximum value corresponding oxygen intake rate (% peakVO2).

Note that the exercise implementation status record database 54 illustrated in Fig. 13 is also configured to record the exercise time corresponding to the exercise type (Kest).

Note that the example illustrated in Fig. 13 is an example in which record data is generated and recorded on a weekly basis. That is, the example illustrated in Fig. 13 is a data recording example in which the exercise time executed by the user 11 in one week is recorded in a predetermined section unit of the maximum value corresponding oxygen intake rate (% peakVO2) and further recorded in an exercise type unit.

Referring to Fig. 12, the processing executed by the user terminal 30 will be described.

As described above, the exercise time integration unit 53 generates the exercise implementation status data d53 classified into sections of the predefined maximum value corresponding oxygen intake rate (% peakVO2) for each predefined data generation period (for example, one week, one day, or the like), and records the generated exercise implementation status data d53 in the exercise implementation status record database 54.

The exercise implementation status analysis unit 56 acquires data recorded in the exercise implementation status record database 54, compares the acquired data with an exercise prescription provided in advance from a rehabilitation supervisor such as a doctor, generates an exercise implementation status analysis result d56, and outputs the generated exercise implementation status analysis result to a display unit 57.

The exercise prescription provided in advance by the rehabilitation supervisor such as a doctor is recorded as the exercise prescription information database 55 in the storage unit of the user terminal 30.

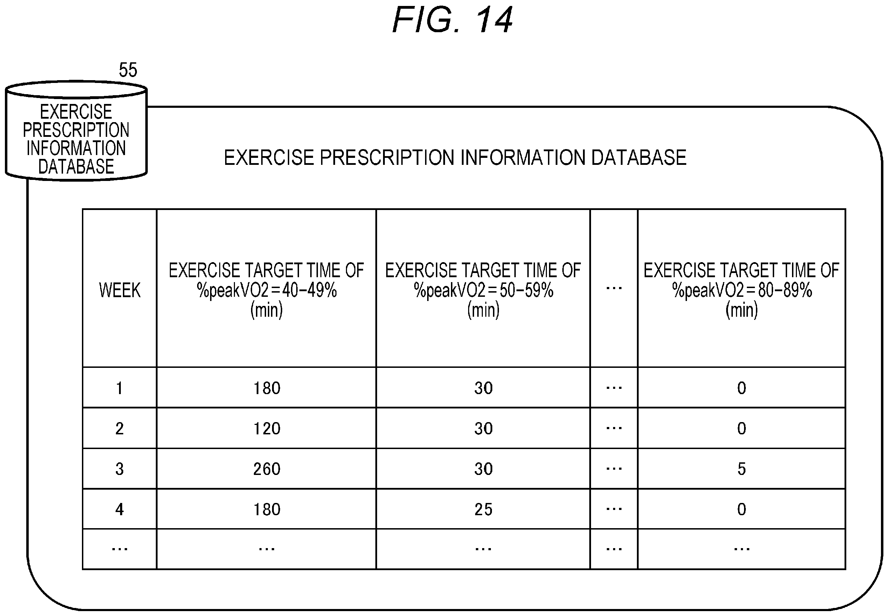

A data example of the exercise prescription information database 55 stored in the storage unit of the user terminal 30 will be described with reference to Fig. 14.

As illustrated in Fig. 14, similarly to the exercise implementation status record database 54 described above with reference to Fig. 13, the exercise time to be executed by the user 11 is recorded in the exercise prescription information database 55 in a predetermined section unit of the maximum value corresponding oxygen intake rate (% peakVO2).

Note that, similarly to the exercise implementation status record database 54 illustrated in Fig. 13 described above, an example in which the exercise prescription information database 55 illustrated in Fig. 14 records the exercise target time in units of weeks is illustrated.

The exercise implementation status analysis unit 56 acquires the following data and performs comparison processing.

(a) The actual exercise time (section unit of maximum value corresponding oxygen intake rate (% peakVO2)) executed by the user 11 recorded in the exercise implementation status record database 54.
(b) The target exercise time (section unit of the maximum value corresponding oxygen intake rate (% peakVO2)) to be executed by the user 11 recorded in the exercise prescription information database 55.

The exercise implementation status analysis unit 56 compares the above two data, executes analysis processing such as whether or not the actual exercise time of the user 11 reaches the target time and furthermore whether or not the actual exercise time is excessive compared to the target time, generates an exercise implementation status analysis result d56 on the basis of the analysis result, and outputs the generated exercise implementation status analysis result to the display unit 57.

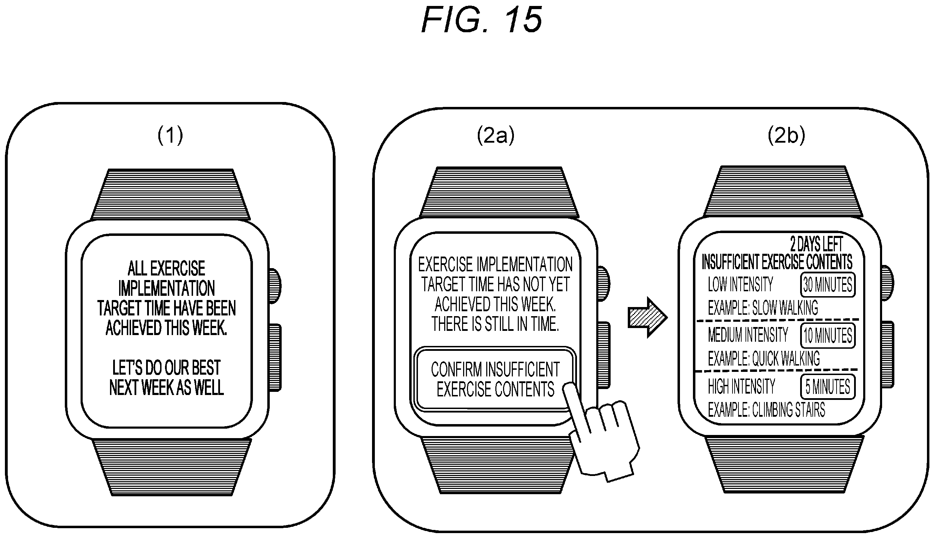

A specific example of the exercise implementation status analysis result d56 displayed on the display unit 57 will be described with reference to Fig. 15.

Fig. 15 illustrates the following two display data examples as specific examples of the exercise implementation status analysis result d56 displayed on the display unit 57.

(1) In a case where the actual exercise time substantially matches the target time
(2) In a case where the actual exercise time is less than the target time

(1) In a case where the actual exercise time substantially matches the target time, for example, the following message isdisplayed as illustrated in Fig. 15(1).

"This week, all the exercise execution target times have been achieved. Let's try our best next week as well".

On the other hand, (2) in a case where the actual exercise time is less than the target time, as illustrated in (2a), a message such as "Exercise execution target time has not been achieved yet this week. There is still in time." is displayed, and furthermore, the user "confirms insufficient exercise contents"

When this message display area is tapped, as illustrated in (2b), a specific insufficient time of each exercise intensity contents and exercise contents are displayed.

The user 11 can confirm whether or not the rehabilitation exercise performed by the user himself/herself is an appropriate exercise according to the exercise prescription by viewing the display data. For example, it is possible to confirm the insufficient exercise for each exercise intensity, and it is possible to accurately execute the exercise according to each exercise intensity.

[5. Sequence of processing executed by user terminal]

Next, a sequence of processing executed by the user terminal 30 will be described.

As described above, the processing of the present disclosure includes the following two phases:

(First phase) Data acquisition phase for rehabilitation analysis in hospital.
(Second phase) Rehabilitation execution phase outside hospital. Hereinafter, a sequence of processing executed by the user terminal 30 in each of these two phases will be sequentially described.

[5-1. (First phase) Sequence of processing executed by user terminal in rehabilitation analysis data acquisition phase in hospital]

First, a sequence of processing executed by the user terminal 30 in the "(first phase) rehabilitation analysis data acquisition phase in a hospital" will be described.

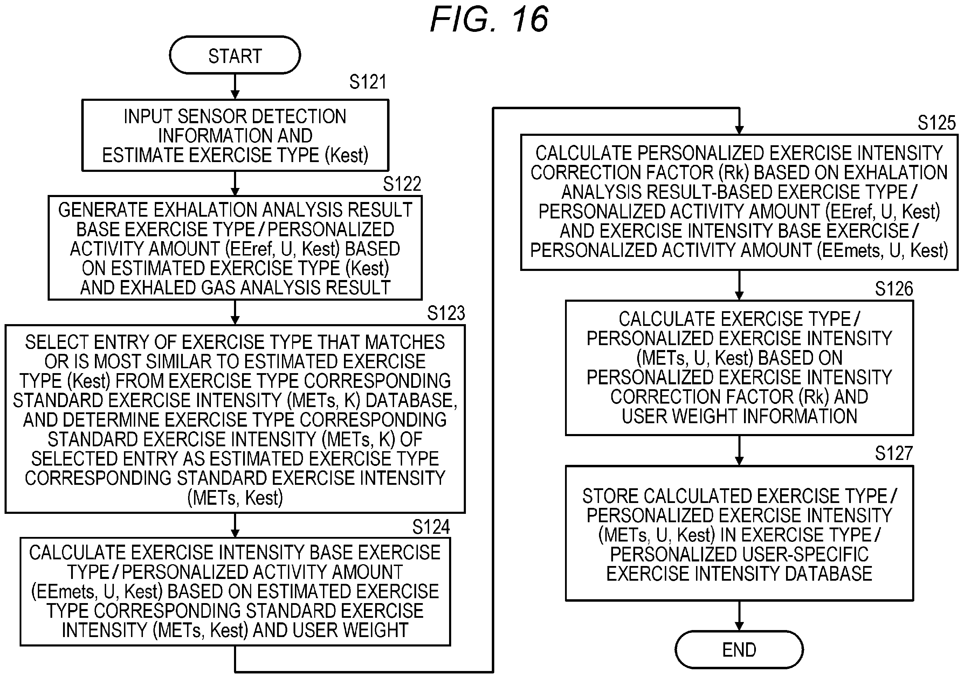

Fig. 16 is a diagram illustrating a flowchart for describing a sequence of processing executed by the user terminal 30 in the "(first phase) rehabilitation analysis data acquisition phase in a hospital".

Note that the processing according to the flow illustrated in Fig. 16 can be executed according to a program stored in the storage unit of the user terminal 30. For example, the processing can be performed as program execution processing by a processor such as a CPU having a program execution function.

Hereinafter, processing of each step of the flow will be sequentially described

(Step S121)

First, in step S121, the user terminal 30 inputs the sensor detection information, and estimates the exercise type being executed by the user. That is, the estimated exercise type (Kest) is determined.

This processing is processing executed by the exercise type estimation unit 32 illustrated in Fig. 9.

The exercise information estimation unit 32 estimates the exercise type of the user 11 on the basis of the input sensor detection information d31. For example, the type of exercise being executed by the user 11 is estimated, such as walking (walking speed = 4.0 km/h on flatland walking), running (running speed = 7.0 km/h on flatland running), and AEROBIKE (registered trademark) (load amount = 120 W).

(Step S122)

Next, in step S122, the user terminal 30 generates an exhalation analysis result-based exercise type/personalized activity amount (EEref, U, Kest) on the basis of the estimated exercise type (Kest) and the exhaled gas analysis result.

This processing is processing executed by the steady state activity amount analysis unit 33 illustrated in Fig. 9.

The steady state activity amount analysis unit 33 inputs the following data:

(a) exhalation analysis result-based personalized activity amount (EEref, U) d21 generated by the activity amount acquisition unit 21, and
(b) the estimated exercise type (Kest) indicating the exercise type being executed by the user by the exercise information estimation unit 32 to generate the exhalation analysis result-based exercise type/personalized activity amount (EEref, U, Kest) on the basis of the pieces of data.

"The exhalation analysis result-based exercise type/personalized activity amount (EEref, U, K)" is the exhalation analysis result used personalized activity amount calculated on the basis of the exhaled gas analysis result ($VO_2$ and $VCO_2$) when the measurement value of the oxygen intake ($VO_2$) of the exhaled gas analyzer reaches a steady state during the exercise execution of the exercise type (K).

(Step S123)

Next, in step S123, the user terminal 30 selects, from the exercise type corresponding standard exercise intensity (METs, K) database 34, an entry of an exercise type matching or the most similar to the estimated exercise type (Rest), and determines the exercise type corresponding standard exercise intensity (METs, K) of the selected entry as the estimated exercise type corresponding standard exercise intensity (METs, Kest).

This processing is processing executed by the exercise type corresponding standard exercise intensity acquisition unit 36 illustrated in Fig. 9.

The exercise type corresponding standard exercise intensity acquisition unit 36 searches the exercise type corresponding standard exercise intensity (METs, K) database 34 having the registration data illustrated in Fig. 10 on the basis of the estimated exercise type (Kest) input from the exercise information estimation unit 32, acquires the standard exercise intensity (METs, K) registered in association with the exercise type (K) that is the same as or the most similar to the estimated exercise type (Rest), and determines the acquired standard exercise intensity (METs, K) as the estimated exercise type corresponding standard exercise intensity (METs, Kest).

(Step S124)

Next, in step S124, the user terminal 30 calculates an exercise intensity-based exercise type/personalized activity amount (EEmets, U, Kest) on the basis of the estimated exercise type corresponding standard exercise intensity (METs, Kest) and the weight of the user.

This processing is processing executed by the exercise intensity-based exercise type/personalized activity amount calculation unit 37 illustrated in Fig. 9.

The exercise intensity-based exercise type/personalized activity amount calculation unit 37

(a) the estimated exercise type corresponding standard exercise intensity (METs, Kest) d36 output from the exercise type corresponding standard exercise intensity acquisition unit 36, and
(b) weight information d35 of the user individual acquired from the personal information database 35.

The exercise intensity-based exercise type/personalized activity amount calculation unit 37 uses the input information (a) and (b) to calculate
the exercise intensity-based exercise type/personalized activity amount (EEmets, U, Kest) d37, which is the personalized activity amount (consumed calories) by Equation, that is, EEmets, U [kcal] = (METs) $\times$ (weight [kg]) $\times$ (exercise time [hr]) $\times$ 1.05.

(Step S125)

Next, in step S125, the user terminal 30 generates, on the basis of each of (a) the exhalation analysis result-based exercise type/personalized activity amount (EEref, U, Kest), and (b) the exercise intensity-based exercise type/personalized activity amount (EEmets, U, Kest),
the personalized exercise intensity correction factor (Rk).

This processing is processing executed by the personalized exercise intensity correction factor calculation unit 38 illustrated in Fig. 9.

The personalized exercise intensity correction factor calculation unit 38 calculates, on the basis of the two types of input data (a) and (b),
the personalized exercise intensity correction factor (Rk) to Equation, that is, Rk = (EEref, U, K)/(EEmets, U, K).

(Step S126)

Next, in step S126, the user terminal 30 calculates the exercise type/personalized exercise intensity (METs, U, Kest) on the basis of the personalized exercise intensity correction factor (Rk) and the user weight information.

This processing is processing executed by the exercise type/personalized exercise intensity calculation unit 39 illustrated in Fig. 9.

The exercise type/personalized exercise intensity calculation unit 39 inputs each of the following data:

(a) the personalized exercise intensity correction factor (Rk) d38 calculated by the personalized exercise intensity correction factor calculation unit 38,
(b) weight information d35 of the user individual acquired from the personal information database 35.

The exercise type/personalized exercise intensity calculation unit 39 calculates an exercise type/personalized exercise intensity (METs, U, Kest) d39 by using the pieces of input data.

As described above with reference to Fig. 9, the exercise type/personalized exercise intensity (METs, U, K) can be indicated as METs, U, K = (EEref, U, K)/((weight [kg]) × (exercise time [hr])) ... (Equation 1).

Further, as described above, the personalized exercise intensity correction factor (Rk) d38 calculated by the personalized exercise intensity correction factor calculation unit 38 is Rk = (EEref, U, K)/(EEmets, U, K) ... (Equation 2).

The following (Equation 3) is derived from (Equation 1) and (Equation 2).

```
METs, U, K = ((EEmets, U, K) × (Rk))/((weight [kg]) × (exercise
time [hr]))  ... (Equation 3)
```

The exercise type/personalized exercise intensity calculation unit 39 uses the exercise time information measured by the timer and the following input data:

(a) the personalized exercise intensity correction factor (Rk) d38 calculated by the personalized exercise intensity correction factor calculation unit 38,
(b) weight information d35 of the user individual acquired from the personal information database 35.

The exercise type/personalized exercise intensity calculation unit 39 calculates an exercise type/personalized exercise intensity (METs, U, Kest) d39 by using the pieces of input data.

The exercise type/personalized exercise intensity calculation unit 39 is based on the pieces of input data to calculate the exercise type/personalized exercise intensity (METs, U, K) according to the above (Equation 3).

As described above with reference to (b4) of Fig. 6, the exercise type/personalized exercise intensity (METs, U, K) is the personalized exercise intensity (METs, U) according to the exercise type (K).

In addition, as described above with reference to (b3) of Fig. 6, the personalized exercise intensity (METs, U) is a value indicating an exercise intensity corresponding to a user individual (U) calculated by dividing the "exhalation analysis result use calculated activity amount (EEref)" obtained from the analysis result of the exhaled gas analyzer worn on the user who performs the exercise by a weight [kg] of a user and an exercise time [hr]. The unit is [kcal/kg/hr].

```
METs, U = (EEref)/((weight [kg]) × (exercise time [hr])).
```

The exercise type/personalized exercise intensity (METs, U, K) is user-specific exercise intensity, and is a value different from the published standard exercise intensity (METs) or the exercise type corresponding standard exercise intensity (METs, K).

That is, the exercise intensity is the user-specific exercise intensity calculated by reflecting the exhaled gas analysis result during the actual exercise execution of the user or the weight of the user.

(Step S127)

Finally, in step S127, the user terminal 30 stores the calculated exercise type/personalized exercise intensity (METs, U, Kest) in the exercise type corresponding user-specific exercise intensity database 40.

This processing is processing executed by the exercise type/personalized exercise intensity calculation unit 39 illustrated in Fig. 9.

As a result, the exercise type corresponding user-specific exercise intensity (METs, U, Kest) database 40 described above with reference to Fig. 11 is generated. The following data is registered in the exercise type corresponding user-specific exercise intensity (METs, U, Kest) database 40 in association with each other.

(a) Exercise type (K(Kest))
(b) Exercise type/personalized user-specific exercise intensity (METs, U, Kest).

As described above, the "(b) the exercise type/personalized user-specific exercise intensity (METs, U, Kest)" registered in the database is the user-specific exercise intensity calculated by reflecting the exhaled gas analysis result or the weight of the user during the actual exercise.

Therefore, the exercise intensity is the exercise intensity reflecting the actual body state of the user from the exercise type corresponding standard exercise intensity (METs, K) which is the published standard value, and the exercise to be executed as the rehabilitation is determined or evaluated on the basis of the exercise intensity registered in the database, so the planning or evaluation the optimal rehabilitation can be performed according to the body state of the user.

(5-1-2. Processing example of inputting exercise type information from exercise instrument without estimating exercise type)

In the above-described processing sequence, the type of the exercise being executed by the user is estimated on the basis of the detection information of the sensor worn on the user 11

However, for example, in a case where an exercise device such as a fixed bike (for example, AEROBIKE (registered trademark)) is used, exercise type information, for example, information such as load setting of AEROBIKE (registered trademark) can be directly acquired from AEROBIKE (registered trademark).

In such a case, the estimation processing of the exercise type using the sensor can be omitted.

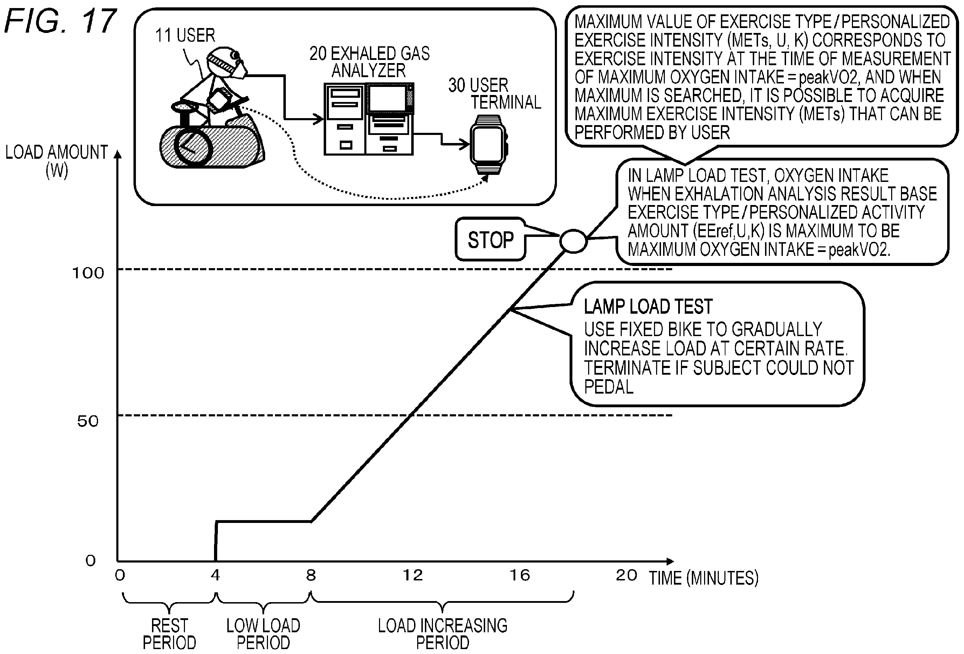

An exercise example using a fixed bike (for example, AEROBIKE (registered trademark)) will be described with reference to Fig. 17.

The user 11 rides on AEROBIKE (registered trademark) and pedals AEROBIKE (registered trademark) while performing the exhaled gas analysis by the exhaled gas analyzer 20.

The analysis result of the exhaled gas analyzer 20 is output to the user terminal 30 worn by the user 11.

AEROBIKE (registered trademark) has a configuration capable of controlling a load amount.

In the graph illustrated in Fig. 17, a horizontal axis represents time (minutes), and a vertical axis represents the load amount (W).

The illustrated example is an example of a lamp load test using AEROBIKE (registered trademark).

The lamp load test is a test that uses AEROBIKE (registered trademark), gradually increases a load at a constant rate, and ends when a user (subject) does not pedal.

As illustrated in the diagram, after a resting time of 4 minutes from 0 to 4 minutes and a low-load period of 4 minutes from 4 minutes to 8 minutes elapses, the load is gradually increased.

In this lamp test, the oxygen intake when the exhalation analysis result-based exercise type/personalized activity amount (EEref, U, K) is maximized is the maximum oxygen intake = peakVO2.

The maximum amount of the exhalation analysis result-based exercise type/personalized activity amount (EEref, U, K) is the activity amount when the load of the aero motorcycle (registered trademark) is gradually increased and the user (subject) does not pedal, and the oxygen intake at this time is the maximum oxygen intake = peakVO2.

The maximum value of the exercise type/personalized exercise intensity (METs, U, K) corresponds to the exercise intensity when the maximum oxygen intake = peakVO2, and the maximum exercise intensity (METs) that can be performed by the user can be acquired by searching for the maximum value.

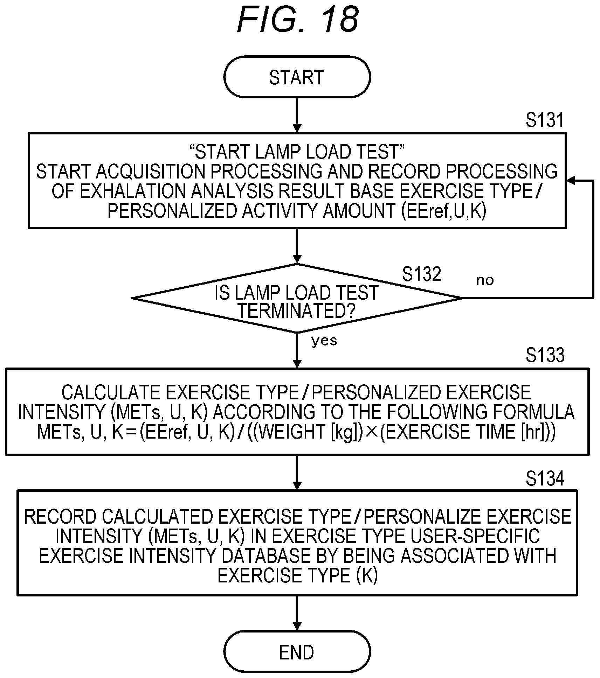

Fig. 18 is a flowchart for describing a sequence of processing of performing exercise using AEROBIKE (registered trademark) illustrated in Fig. 17, inputting exercise type information from exercise instruments, and acquiring the exercise type/personalized exercise intensity (METs, U, K).

Processing of each step of the flow will be sequentially described

(Step S131)

First, in step S131, the lamp load test starts. Specifically, the acquisition processing and the record processing of the exhalation analysis result-based exercise type/personalized activity amount (EEref, U, K) start.

Note that this processing is processing to which the configuration of the exhaled gas analyzer 20 illustrated in Fig. 9 and the steady state activity amount analysis unit 33 of the user terminal 30 are applied. However, the exercise type (K) is directly input from AEROBIKE (registered trademark). For example, the processing of acquiring the exhalation analysis result-based exercise type/personalized activity amount (EEref, U, K) corresponding to the exercise type (K) including load information of AEROBIKE (registered trademark) and the record processing are executed.

(Step S132)

Next, in step S132, it is determined whether or not the lamp load test ends.

As described above, the process ends when the user (subject) 11 does not pedal.

In a case where the process does not end, the process returns to step S131 to continue the lamp test.

In a case where it is determined that the user (subject) 11 does not pedal and the process ends, the process proceeds to step S133.

(Step S133)

In step S133, the exercise type/personalized exercise intensity (METs, U, K) are calculated according to the following Equation.

$$\text{METs, U, K} = (\text{EEref, U, K})/((\text{weight [kg]}) \times (\text{exercise time [hr]}))$$

The processing is executed by the exercise type/personalized exercise intensity calculation unit 39 illustrated in Fig. 9.

The exercise type/personalized exercise intensity calculation unit 39 illustrated in Fig. 9 includes:

(a) the personalized activity amount (EEref, U, K) calculated by the steady state activity amount analysis unit 33,
(b) weight information acquired from the personal information database 35,
(c) exercise time acquired from AEROBIKE (registered trademark);

The exercise type/personalized exercise intensity calculation unit 39 uses these pieces of information to calculate the exercise type/personalized exercise intensity (METs, U, K) according to the above Equation.

The exercise type/personalized exercise intensity calculation unit 39 illustrated in Fig. 9 stores the calculated exercise type/personalized exercise intensity (METs, U, K) in the exercise type corresponding user-specific exercise intensity database 40.

In this way, for example, in a case where the exercise device such as AEROBIKE (registered trademark) is used, the exercise type information, for example, the information such as load setting of AEROBIKE (registered trademark) can be directly acquired from AEROBIKE (registered trademark). Therefore, the estimation processing of the exercise type using the sensor can be omitted, and the exercise type/personalized exercise intensity (METs, U, K) can be generated and recorded in the database.

Note that, as described above, in this lamp test, the oxygen intake at the maximum point of time of the exhalation analysis result-based exercise type/personalized activity amount (EEref, U, K) is the maximum oxygen intake = peakVO2.

The maximum amount of the exhalation analysis result-based exercise type/personalized activity amount (EEref, U, K) is the activity amount when the load of the aero motorcycle (registered trademark) is gradually increased and the user (subject) does not pedal, and the oxygen intake at this time is the maximum oxygen intake = peakVO2.

The maximum value of the exhalation analysis result-based exercise type/personalized activity amount (EEref, U, K) is the maximum exhalation analysis result-based exercise type/personalized activity amount (EErefmax, U, K).

The exercise type user-specific exercise intensity maximum value (METsmax, U, K) is registered in the exercise type corresponding user-specific exercise intensity database 40 according to the following calculation Equation.

$$\text{METsmax, U, K} = (\text{EErefmax, U, K})/((\text{weight [kg]}) \times (\text{exercise time [hr]}))$$

[5-2. (Second phase) Sequence of processing executed by user terminal in rehabilitation execution phase outside hospital]

Next, a sequence of processing executed by the user terminal 30 in the "(second phase) rehabilitation execution phase outside a hospital" will be described.

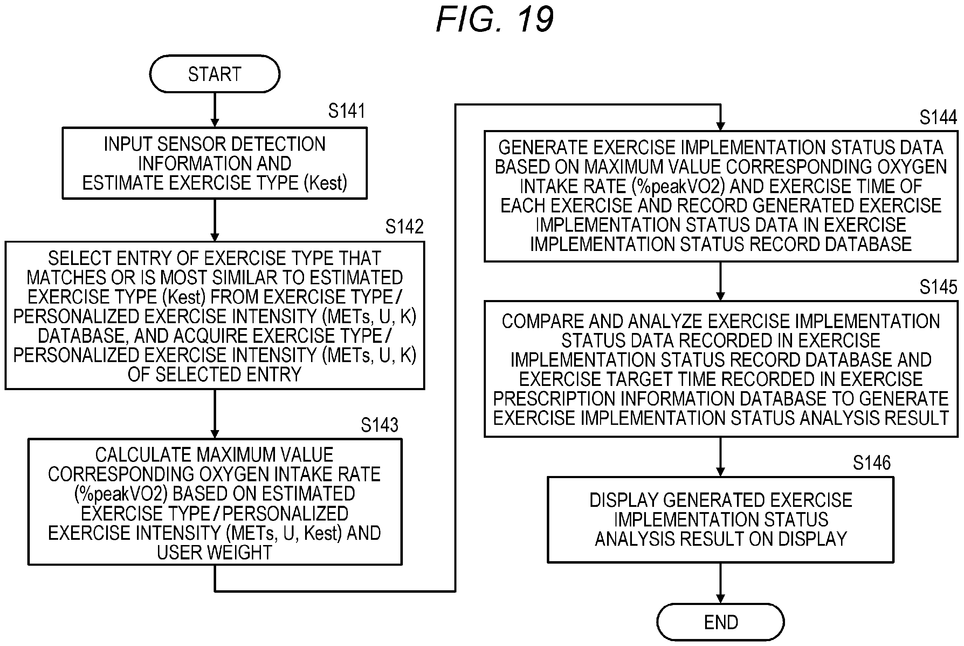

Fig. 19 is a diagram illustrating a flowchart for describing a sequence of processing executed by the user terminal 30 in the "(second phase) rehabilitation execution phase outside a hospital".

Note that the processing according to the flow illustrated in Fig. 19 can be executed according to a program stored in the storage unit of the user terminal 30. For example, the processing can be performed as program execution processing by a processor such as a CPU having a program execution function.

Hereinafter, processing of each step of the flow will be sequentially described

(Step S141)

First, in step S141, the user terminal 30 inputs the sensor detection information and estimates the exercise type being executed by a user. That is, the estimated exercise type (Kest) is determined.

This processing is processing executed by the exercise information estimation unit 32 illustrated in Fig. 12.

The exercise information estimation unit 32 estimates the exercise type of the user 11 on the basis of the input sensor detection information d31. For example, the type of exercise being executed by the user 11 is estimated, such as walking (walking speed = 4.0 km/h on flatland walking), running (running speed = 7.0 km/h on flatland running), and AEROBIKE (registered trademark) (load amount = 120 W).

(Step S142)

Next, in step S142, the user terminal 30 selects an entry of an exercise type matching or the most similar to the estimated exercise type (Kest) from the exercise type corresponding user-specific exercise intensity (METs, U, Kest) database 40, and acquires the exercise type/personalized exercise intensity (METs, U, Kest) of the selected entry.
This processing is processing executed by the exercise type/personalized exercise intensity acquisition unit 51 illustrated in Fig. 12.
The exercise type/personalized exercise intensity acquisition unit 51 searches the exercise type corresponding user-specific exercise intensity (METs, U, Kest) database 40 having the registration data illustrated in Fig. 11 on the basis of the estimated exercise type (Kest) input from the exercise information estimation unit 32, and acquires the exercise type/personalized exercise intensity (METs, U, Kest) registered in association with the exercise type (K) that is the same as or the most similar to the estimated exercise type (Kest).

(Step S143)

Next, in step S143, the user terminal 30 calculates the maximum value corresponding oxygen intake rate (% peakVO2) on the basis of the estimated exercise type/personalized exercise intensity (METs, U, Kest) and the user weight.
This processing is processing executed by the maximum value corresponding oxygen intake rate calculation unit 52 illustrated in Fig. 12.
The maximum value corresponding oxygen intake rate calculation unit 52 inputs the following data:

(a) the exercise type/personalized user-specific exercise intensity (METs, U, Kest) d51 acquired from the exercise type/personalized exercise intensity acquisition unit 51, and
(b) the exercise type user-specific exercise intensity maximum value (METsmax, U, Kest) acquired from the exercise type corresponding user-specific exercise intensity (METs, U, Kest) database 40.

From the pieces of data, the maximum value corresponding oxygen intake rate (% peakVO2) d52 is calculated.
As described above, the maximum value corresponding oxygen intake rate (% peakVO2) is calculated by the following (Equation 4).

```
    Maximum value corresponding oxygen intake rate (% peakVO2) =
(Exercise type/personalized user-specific exercise intensity (METs, U,
Kest))/(exercise type user-specific exercise intensity maximum value
(METsmax, U, Kest))  ... (Equation 4)
```

That is, the maximum value corresponding oxygen intake rate (% peakVO2) can be calculated by the ratio of the exercise type/personalized user-specific exercise intensity (METs, U, Kest) to the exercise type user-specific exercise intensity maximum value (METsmax, U, Kest).

(Step S144)

Next, in step S144, the user terminal 30 generates exercise implementation status data on the basis of the maximum value corresponding oxygen intake rate (% peakVO2) and the exercise time of each exercise, and records the generated exercise implementation status data in the exercise implementation status record database.
This processing is processing executed by the exercise time integration unit 53 illustrated in Fig. 12.
The exercise time integration unit 53 inputs the maximum value corresponding oxygen intake rate (% peakVO2) d52 calculated by the maximum value corresponding oxygen intake rate calculation unit 52, generates exercise implementation status data d53 of the user 11, and records the generated exercise implementation status data d53 in the exercise implementation status record database 54.
The exercise time integration unit 53 generates the exercise implementation status data d53 classified into sections of the predefined maximum value corresponding oxygen intake rate (% peakVO2) for each predefined data generation period (for example, one week, one day, or the like), and records the generated exercise implementation status data d53 in the exercise implementation status record database 54.

As described above, the exercise implementation status record database 54 includes the record data illustrated in Fig. 13. As illustrated in Fig. 13, the exercise time executed by the user 11 is recorded in the exercise implementation status record database 54 in a predetermined section unit of the maximum value corresponding oxygen intake rate (% peakVO2).

Note that the exercise implementation status record database 54 illustrated in Fig. 13 is also configured to record the exercise time corresponding to the exercise type (Kest).

(Step S145)

Next, in step S145, the user terminal 30 compares and analyzes the exercise implementation status data recorded in the exercise implementation status record database 54 with the exercise target time recorded in the exercise prescription information database 55, and generates the exercise implementation status analysis result.

This processing is processing executed by the exercise implementation status analysis unit 56 illustrated in Fig. 12.

The exercise implementation status analysis unit 56 acquires data recorded in the exercise implementation status record database 54, compares the acquired data with an exercise prescription provided in advance from a rehabilitation supervisor such as a doctor, and generates an exercise implementation status analysis result d56.

Note that, as described above with reference to Figs. 12 and 14, the exercise prescription provided in advance from a rehabilitation supervisor such as a doctor is recorded in the exercise prescription information database 55 in the storage unit of the user terminal 30.

The exercise implementation status analysis unit 56 executes analysis processing such as whether or not the actual exercise time of the user 11 reaches the target time and furthermore whether or not the actual exercise time is excessive compared to the target time, and generates the exercise implementation status analysis result d56 on the basis of the analysis result.

(Step S146)

Finally, in step S146, the user terminal 30 displays the exercise implementation status analysis result generated in step S145 on the display unit.

This processing is display processing using the display unit 57 illustrated in Fig. 12.

As described above with reference to Fig. 15, the display unit 57 displays the display data as illustrated in Figs. 15(1) and (2) as the exercise implementation status analysis result d56.

The user 11 can confirm whether or not the rehabilitation exercise performed by the user himself/herself is an appropriate exercise according to the exercise prescription by viewing the display data. For example, it is possible to confirm the insufficient exercise for each exercise intensity, and it is possible to accurately execute the exercise according to each exercise intensity.

[6. (Second embodiment) Embodiment of performing communication between user terminal and hospital terminal]

Next, as a second embodiment, an embodiment of performing communication between a user terminal and a hospital terminal will be described.

This embodiment achieves, for example, the processing described above with reference to Fig. 4.

As described above, Fig. 4 is a diagram for describing a processing example of the "(second phase) rehabilitation execution phase outside a hospital"

The example illustrated in Fig. 4 is a processing example in which the user terminal 30 and the hospital terminal 70 communicate with each other.

The user 11 who is a rehabilitation performer wears the user terminal 30, and inputs the sensor detection information of the sensor 12 worn on the body or the like of the user 11, for example, the sensor 12, such as an acceleration sensor, a gyro, a geomagnetic sensor, or an atmospheric pressure sensor, to the user terminal 30.

The data processing unit of the user terminal 30 transmits the sensor detection information acquired via the sensor 12 as it is or the analysis result to the hospital terminal 70.

The hospital terminal 70 first executes data analysis processing similar to the processing executed in the user terminal 30 described above with reference to Fig. 3.

Furthermore, advice information or the like for the user 11 is generated on the basis of the analysis result and transmitted to the user terminal 30.

The user terminal 30 displays the advice information received from the hospital terminal 70. The user 11 can confirm whether or not appropriate rehabilitation is being performed on the basis of the display information.

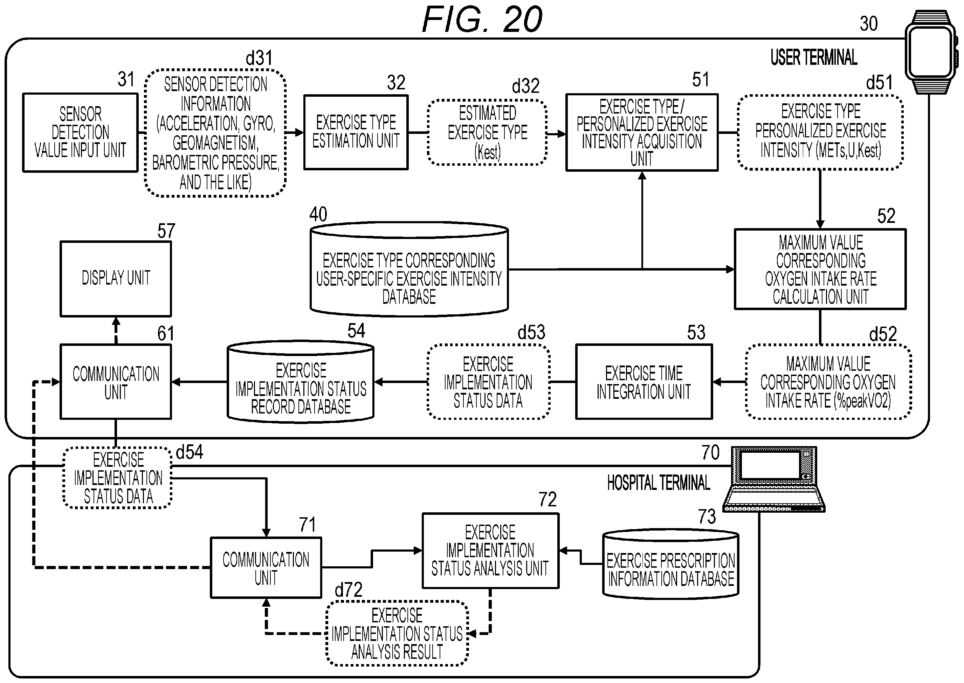

Fig. 20 is a diagram illustrating a configuration example of each device in a case where the above-described processing, that is, communication between the user terminal 30 and the hospital terminal 70 is performed.

The configurations of the user terminal 30 and the hospital terminal 70 illustrated in Fig. 20 and the processing to be executed will be described.

The user terminal 30 is a terminal worn on the body while the user 11 who is a rehabilitation performer is executing an exercise as rehabilitation.

The sensor detection information input unit 31 of the user terminal 30 acquires sensor detection information d31 of a sensor worn on the body of the user 11, for example, a sensor such as an acceleration sensor, a gyro, a geomagnetic sensor, or an atmospheric pressure sensor, and outputs the sensor detection information d31 to the exercise information estimation unit 32.

The exercise information estimation unit 32 estimates the exercise type of the user 11 on the basis of the input sensor detection information d31. For example, the type of exercise being executed by the user 11 is estimated, such as walking (walking speed = 4.0 km/h on flatland walking), running (running speed = 7.0 km/h on flatland running), and AEROBIKE (registered trademark) (load amount = 120 W).

The exercise type estimation unit 32 outputs the estimated exercise type (Kest) as the estimation result to the exercise type/personalized exercise intensity acquisition unit 51.

The exercise type/personalized exercise intensity acquisition unit 51 searches the exercise type corresponding user-specific exercise intensity (METs, U, Kest) database 40 on the basis of the estimated exercise type (Kest) d32 input from the exercise type estimation unit 32, and acquires the exercise type/personalized user-specific exercise intensity (METs, U, Kest) d51 registered in association with the estimated exercise type (Kest).

The exercise type/personalized user-specific exercise intensity (METs, U, Kest) d51 acquired from the database by the exercise type/personalized exercise intensity acquisition unit 51 is output to a maximum value corresponding oxygen intake rate calculation unit 52.

The maximum value corresponding oxygen intake rate calculation unit 52 acquires, from the exercise type corresponding user-specific exercise intensity (METs, U, Kest) database 40, the maximum value of the personalized user-specific exercise intensity (METs, U, Kest) registered in the database.

The maximum value of the personalized user-specific exercise intensity (METs, U, Kest) registered in the exercise type corresponding user-specific exercise intensity (METs, U, Kest) database 40 is set to be an exercise type user-specific exercise intensity maximum value (METsmax, U, Kest).

The maximum value corresponding oxygen intake rate calculation unit 52 includes:

(a) the exercise type/personalized user-specific exercise intensity (METs, U, Kest) d51 acquired from the exercise type/personalized exercise intensity acquisition unit 51, and
(b) the exercise type user-specific exercise intensity maximum value (METsmax, U, Kest) acquired from the exercise type corresponding user-specific exercise intensity (METs, U, Kest) database 40.

From the pieces of data, the maximum value corresponding oxygen intake rate (% peakVO2) d52 is calculated.

The maximum value corresponding oxygen intake rate (% peakVO2) is calculated by the following (Equation 4).

```
Maximum value corresponding oxygen intake rate (% peakVO2) =
(Exercise type/personalized user-specific exercise intensity (METs, U,
Kest))/(exercise type user-specific exercise intensity maximum value
(METsmax, U, Kest))  … (Equation 4)
```

That is, the maximum value corresponding oxygen intake rate (% peakVO2) can be calculated by the ratio of the exercise type/personalized user-specific exercise intensity (METs, U, Kest) to the exercise type user-specific exercise intensity maximum value (METsmax, U, Kest).

Note that, as described above with reference to Fig. 5, the maximum oxygen intake (peakVO2) is the maximum value of oxygen amount (oxygen intake (VO2)) consumed by a body, and the maximum value corresponding oxygen intake rate (% peakVO2) is the ratio of the actual oxygen intake (VO2) to the maximal oxygen intake (peakVO2).

The maximum value corresponding oxygen intake rate (% peakVO2) d52 calculated by the maximum value corresponding oxygen intake rate calculation unit 52 is output to an exercise time integration unit 53.

The exercise time integration unit 53 inputs the maximum value corresponding oxygen intake rate (% peakVO2) d52 calculated by the maximum value corresponding oxygen intake rate calculation unit 52, generates exercise implementation status data d53 of the user 11, and records the generated exercise implementation status data d53 in the exercise implementation status record database 54.

The exercise implementation status data of the user 11 recorded in the exercise implementation status record database 54 is periodically transmitted to the hospital terminal 70 via the communication unit 61, for example, on a weekly basis. Fig. 20 illustrates the exercise implementation status data d54.

The communication unit 71 of the hospital terminal 70 receives the exercise implementation status data d54 transmitted from the user terminal 30, and transfers the received exercise implementation status data d54 to the exercise implementation status analysis unit 72.

The exercise implementation status analysis unit 72 acquires the exercise implementation status data d54 received from the user terminal 30, compares the acquired data with the exercise prescription corresponding to the user 11 created in advance in the hospital, generates an exercise implementation status analysis result d72, and transmits the generated exercise implementation status analysis result to the user terminal 30 via the communication unit 71.

Note that the exercise prescription corresponding to the user 11 created in advance in the hospital is recorded in the storage unit of the hospital terminal 70 as the exercise prescription information database 73.

Data similar to the data of the exercise prescription information database 55 stored in the storage unit of the user terminal 30 described above with reference to Fig. 14 is recorded in the exercise prescription information database 73.

The exercise implementation status analysis unit 72 of the hospital terminal 70 acquires the following data and performs comparison processing.

(a) Actual exercise time (section unit of maximum value corresponding oxygen intake rate (% peakVO2)) executed by the user 11 recorded in the exercise implementation status data d54 received from the user terminal 30
(b) Target exercise time (section unit of maximum value corresponding oxygen intake rate (% peakVO2)) to be executed by the user 11 recorded in the exercise prescription information database 73

The exercise implementation status analysis unit 72 compares the above two data, executes the analysis processing such as whether or not the actual exercise time of the user 11 reaches the target time and furthermore whether or not the actual exercise time is excessive compared to the target time, and generates the exercise implementation status analysis result d72 on the basis of the analysis result.

The exercise implementation status analysis result d72 generated by the exercise implementation status analysis unit 72 of the hospital terminal 70 is transmitted to the user terminal 30 via the communication unit 71.

The communication unit 61 of the user terminal 30 outputs the exercise implementation status analysis result d72 received from the hospital terminal 70 to the display unit 57.

The exercise implementation status analysis result d72 displayed on the display unit 57 of the user terminal 30 is, for example, the data described above with reference to Fig. 15.

Fig. 15 illustrates the following two display data examples.

(1) In a case where the actual exercise time substantially matches the target time
(2) In a case where the actual exercise time is less than the target time

(1) In a case where the actual exercise time substantially matches the target time, for example, the following message is displayed as illustrated in Fig. 15(1).

"This week, all the exercise execution target times have been achieved. Let's try our best next week as well".

On the other hand, (2) in a case where the actual exercise time is less than the target time, as illustrated in (2a), a message such as "Exercise execution target time has not been achieved yet this week. There is still in time" is displayed, and furthermore, the user is specifically configured to "confirm insufficient exercise contents".

When this message display area is tapped, as illustrated in (2b), a specific insufficient time of each exercise intensity contents and exercise contents are displayed.

The user 11 can confirm whether or not the rehabilitation exercise performed by the user himself/herself is an appropriate exercise according to the exercise prescription by viewing the display data. For example, it is possible to confirm the insufficient exercise for each exercise intensity, and it is possible to accurately execute the exercise according to each exercise intensity.

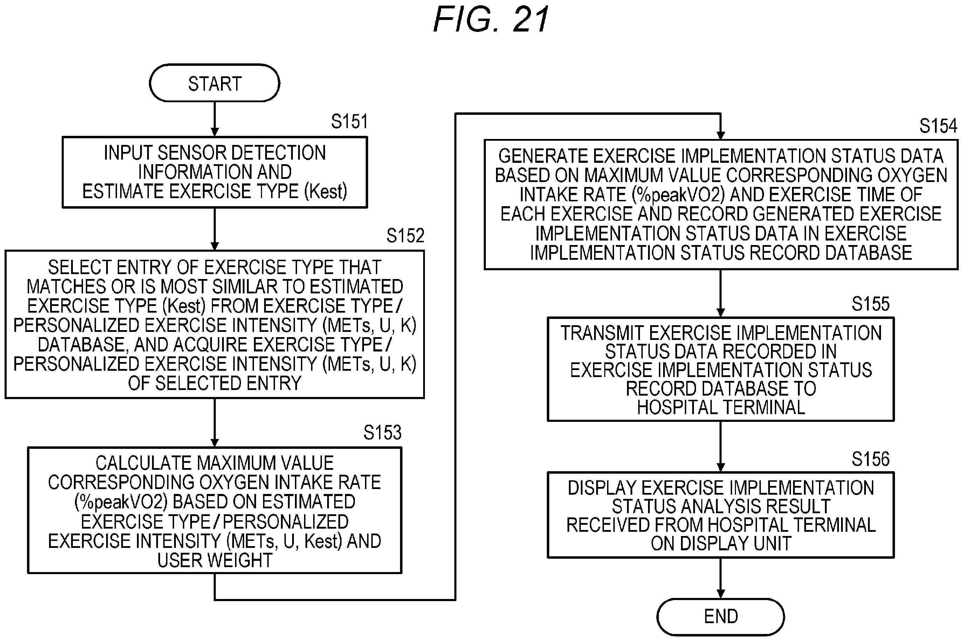

Next, a sequence of processing executed by the user terminal 30 in the present embodiment will be described with reference to a flowchart illustrated in Fig. 21.

Note that the processing according to the flow illustrated in Fig. 21 can be executed according to a program stored in the storage unit of the user terminal 30. For example, the processing can be performed as program execution processing by a processor such as a CPU having a program execution function.

Hereinafter, processing of each step of the flow will be sequentially described

(Step S151)

First, in step S151, the user terminal 30 inputs the sensor detection information, and estimates the exercise type being executed by the user. That is, the estimated exercise type (Kest) is determined.

This processing is processing executed by the exercise information estimation unit 32 illustrated in Fig. 20.

The exercise information estimation unit 32 estimates the exercise type of the user 11 on the basis of the input sensor detection information d31. For example, the type of exercise being executed by the user 11 is estimated, such as walking (walking speed = 4.0 km/h on flatland walking), running (running speed = 7.0 km/h on flatland running), and AEROBIKE (registered trademark) (load amount = 120 W).

(Step S152)

Next, in step S152, the user terminal 30 selects an entry of an exercise type matching or the most similar to the estimated exercise type (Kest) from the exercise type corresponding user-specific exercise intensity (METs, U, Kest) database 40, and acquires the exercise type/personalized exercise intensity (METs, U, Kest) of the selected entry.

This processing is processing executed by the exercise type/personalized exercise intensity acquisition unit 51 illustrated in Fig. 20.

The exercise type/personalized exercise intensity acquisition unit 51 searches the exercise type corresponding user-specific exercise intensity (METs, U, Kest) database 40 having the registration data illustrated in Fig. 11 on the basis of the estimated exercise type (Kest) input from the exercise information estimation unit 32, and acquires the exercise type/personalized exercise intensity (METs, U, Kest) registered in association with the exercise type (K) that is the same as or the most similar to the estimated exercise type (Kest).

(Step S153)

Next, in step S153, the user terminal 30 calculates the maximum value corresponding oxygen intake rate (% peakVO2) on the basis of the estimated exercise type/personalized exercise intensity (METs, U, Kest) and the user weight.

This processing is processing executed by the maximum value corresponding oxygen intake rate calculation unit 52 illustrated in Fig. 20.

The maximum value corresponding oxygen intake rate calculation unit 52 inputs the following data:

(a) the exercise type/personalized user-specific exercise intensity (METs, U, Kest) d51 acquired from the exercise type/personalized exercise intensity acquisition unit 51, and
(b) the exercise type user-specific exercise intensity maximum value (METsmax, U, Kest) acquired from the exercise type corresponding user-specific exercise intensity (METs, U, Kest) database 40.

From the pieces of data, the maximum value corresponding oxygen intake rate (% peakVO2) d52 is calculated.

As described above, the maximum value corresponding oxygen intake rate (% peakVO2) is calculated by the following (Equation 4).

```
Maximum value corresponding oxygen intake rate (% peakVO2) =
(Exercise type/personalized user-specific exercise intensity (METs, U,
Kest))/(exercise type user-specific exercise intensity maximum value
(METsmax, U, Kest))  … (Equation 4)
```

That is, the maximum value corresponding oxygen intake rate (% peakVO2) can be calculated by the ratio of the exercise type/personalized user-specific exercise intensity (METs, U, Kest) to the exercise type user-specific exercise intensity maximum value (METsmax, U, Kest).

(Step S154)

Next, in step S154, the user terminal 30 generates exercise implementation status data on the basis of the maximum value corresponding oxygen intake rate (% peakVO2) and the exercise time of each exercise, and records the generated exercise implementation status data in the exercise implementation status record database.

This processing is processing executed by the exercise time integration unit 53 illustrated in Fig. 20.

The exercise time integration unit 53 inputs the maximum value corresponding oxygen intake rate (% peakVO2) d52 calculated by the maximum value corresponding oxygen intake rate calculation unit 52, generates exercise implementation status data d53 of the user 11, and records the generated exercise implementation status data d53 in the exercise implementation status record database 54.

The exercise time integration unit 53 generates the exercise implementation status data d53 classified into sections of the predefined maximum value corresponding oxygen intake rate (% peakVO2) for each predefined data generation period (for example, one week, one day, or the like), and records the generated exercise implementation status data d53 in the exercise implementation status record database 54.

As described above, the exercise implementation status record database 54 includes the record data illustrated in Fig. 13. As illustrated in Fig. 13, the exercise time executed by the user 11 is recorded in the exercise implementation status record database 54 in a predetermined section unit of the maximum value corresponding oxygen intake rate (% peakVO2).

Note that the exercise implementation status record database 54 illustrated in Fig. 13 is also configured to record the exercise time corresponding to the exercise type (Kest).

(Step S155)

Next, in step S155, the user terminal 30 transmits the exercise implementation status data recorded in the exercise implementation status record database 54 to the hospital terminal 70.

(Step S156)

Finally, in step S156, the user terminal 30 displays the exercise implementation status analysis result received from the hospital terminal 70 on the display unit.

This processing is display processing using the display unit 57 illustrated in Fig. 20.

As described above with reference to Fig. 15, the display unit 57 displays the display data as illustrated in Figs. 15(1) and (2) as the exercise implementation status analysis result.

The user 11 can confirm whether or not the rehabilitation exercise performed by the user himself/herself is an appropriate exercise according to the exercise prescription by viewing the display data. For example, it is possible to confirm the insufficient exercise for each exercise intensity, and it is possible to accurately execute the exercise according to each exercise intensity.

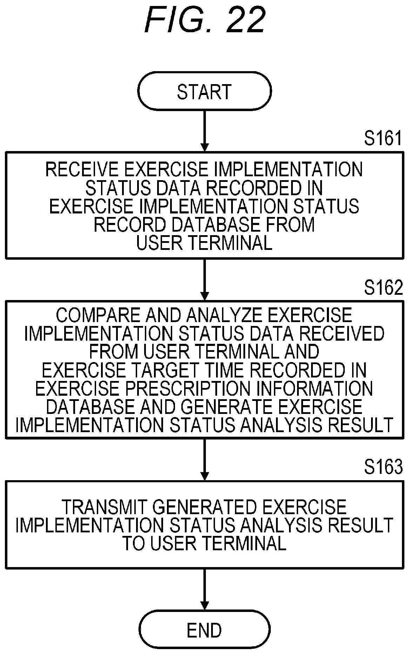

Next, a sequence of processing executed by the hospital terminal 70 will be described with reference to a flowchart illustrated in Fig. 22.

Note that the processing according to the flow illustrated in Fig. 22 can be executed according to a program stored in the storage unit of the hospital terminal 70. For example, the processing can be performed as program execution processing by a processor such as a CPU having a program execution function.

Hereinafter, processing of each step of the flow will be sequentially described

(Step S161)

First, in step S161, the hospital terminal 70 receives the exercise implementation status data recorded in the exercise implementation status record database 54 from the user terminal 30.

(Step S162)

Next, in step S162, the hospital terminal 70 compares and analyzes the exercise implementation status data received from the user terminal 30 with the exercise target time recorded in the exercise prescription information database 73, and generates the exercise implementation status analysis result.

This processing is processing executed by the exercise implementation status analysis unit 72 illustrated in Fig. 20.

The exercise implementation status analysis unit 72 executes comparison processing between the exercise implementation status data received from the user terminal 30 and the exercise prescription recorded in the exercise prescription information database 73 which is the exercise prescription created in advance by the rehabilitation supervisor such as a doctor.

The exercise implementation status analysis unit 72 executes analysis processing such as whether or not the actual exercise time of the user 11 reaches the target time and furthermore whether or not the actual exercise time is excessive compared to the target time, and generates the exercise implementation status analysis result d72 on the basis of the analysis result.

(Step S163)

Finally, in step S163, the hospital terminal 70 transmits the exercise implementation status analysis result generated in step S162 to the user terminal 30 via the communication unit 71.
After this transmission processing, the processing of step S156 of the flow illustrated in Fig. 21 is executed.
That is, in step S156, the exercise implementation status analysis result received from the hospital terminal 70 is displayed on the display unit.
The user 11 can confirm whether or not the rehabilitation exercise performed by the user himself/herself is an appropriate exercise according to the exercise prescription by viewing the display data. For example, it is possible to confirm the insufficient exercise for each exercise intensity, and it is possible to accurately execute the exercise according to each exercise intensity.

[7. Other examples]

Next, another embodiment of the above-described embodiment will be described.
A plurality of embodiments described below will be described.

(Third embodiment) Embodiment of urging electrocardiogram (ECG) measurement based on analysis result of exercise load
(Fourth embodiment) Embodiment of presenting route information of walking or running using map
(Fifth embodiment) Embodiment of performing exercise prescription update processing

[7-1. (Third embodiment) Embodiment of urging electrocardiogram (ECG) measurement based on analysis result of exercise load]

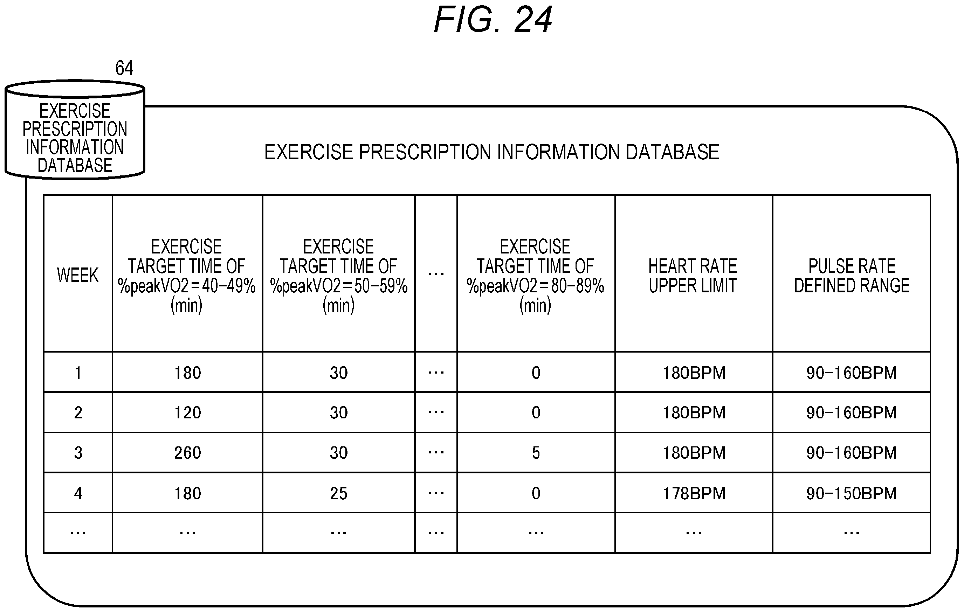

First, as the third embodiment, an example of urging electrocardiogram (ECG) measurement on the basis of an analysis result of an exercise load will be described
In a case where the user 11 is performing exercise as rehabilitation after being discharged from the hospital, if the user performs an excessive exercise, a burden on a heart increases, and thus, the user may fall into a dangerous state.
The third embodiment is an embodiment of preventing such danger.
While the user 11 is performing exercise as rehabilitation after being discharged from the hospital, a pulse rate of the user 11 is acquired, and in a case where it is detected that the pulse rate is out of a defined range, warning information for prompting the measurement of the electrocardiogram (ECG) is output.
The warning information may be configured to use display, vibration, sound, or the like of the warning information on the display unit of the user terminal 30.

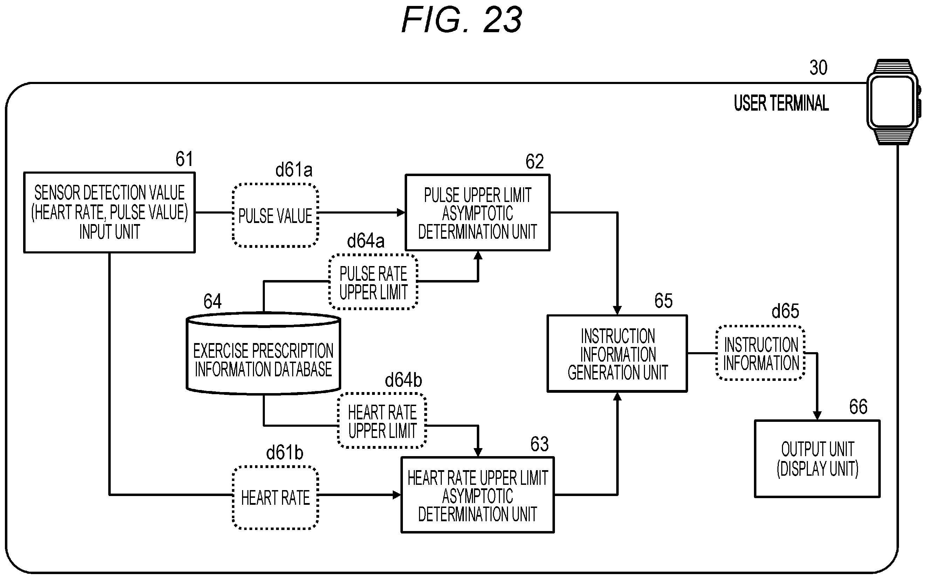

Fig. 23 is a block diagram illustrating a configuration of the user terminal 30 that executes processing according to the third embodiment.
An exercise prescription information database 64 is recorded in the storage unit of the user terminal 30 illustrated in Fig. 23.
A data configuration example of the exercise prescription information database 64 used in the present embodiment will be described with reference to Fig. 24.
The exercise prescription information database 64 used in the present embodiment records a pulse rate defined range (normal value range) and a heart rate upper limit (normal value upper limit) corresponding to the user 11 generated based on a determination of a rehabilitation supervisor such as a doctor together with the exercise time to be executed by the user 11 in the predetermined section unit of the data as described above with reference to Fig. 14, that is, the maximum value corresponding oxygen intake rate (%peakVO2).
Referring to Fig. 23, the configuration and processing of the user terminal 30 will be described.
The sensor detection value input unit 61 inputs a heart rate value and a pulse rate value from a sensor provided in the user 11 who performs exercise as rehabilitation
Note that immediately after the rehabilitation starts, the user 11 wears the pulse detection sensor and measures only the pulse rate value.
A pulse rate value d61a acquired by the sensor detection value input unit 61 is input to a pulse upper limit asymptotic determination unit 62.
The pulse upper limit asymptotic determination unit 62 compares a pulse rate value d61a input from the sensor detection value input unit 61 with a pulse rate defined range (normal value range) corresponding to the user 11 recorded in the exercise prescription information database 64, and determines whether or not the pulse rate value d61a is within the pulse rate defined range (normal value range) registered in the database.

In a case where the pulse rate value d61a is within the pulse rate defined range (normal value range) registered in the database, the determination information indicating that the pulse rate value is within the normal range is output to the instruction information generation unit 65.

The instruction information generation unit 65 generates instruction information d65 indicating a normal value and outputs the generated instruction information to the output unit (display unit or the like) 66.

Meanwhile, in a case where the pulse upper limit asymptotic determination unit 62 determines that the pulse rate value d61a input from the sensor detection value input unit 61 is not within the pulse rate defined range (normal value range) registered in the database, the determination information indicating that the pulse rate value is out of the normal range is output to the instruction information generation unit 65.

The instruction information generation unit 65 generates a message urging the user 11 to wear a heart rate meter for detecting a heart rate value, or instruction information d65 such as a warning sound or lamp light emission, and outputs the generated instruction information d65 to the output unit (display unit or the like) 66.

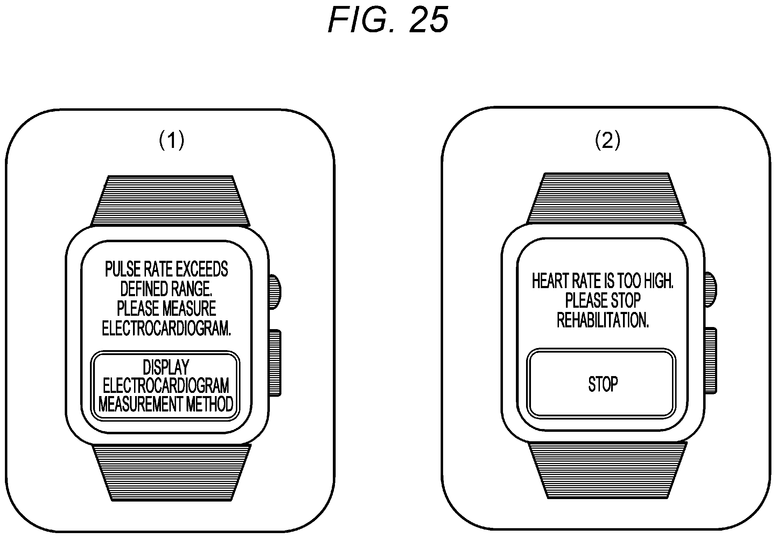

Specifically, for example, display data as illustrated in Fig. 25(1) is output to the output unit (display unit) 66 of the user terminal 30.

In response to the instruction information d65, the user 11 wears a heart rate meter for detecting a heart rate value and continues exercise as rehabilitation.

Next, processing after attaching the heart rate meter for detecting the heart rate value will be described.

The sensor detection value input unit 61 acquires the heart rate value of the user 11 from the heart rate meter worn by the user 11.

Note that, as the heart rate meter for detecting the heart rate value, for example, a heart rate meter using a second guidance method that measures the potential difference between the left and right hands, a wearable device type heart rate meter, or the like can be used.

A heart rate value d61b acquired by the sensor detection value input unit 61 is input to a heart rate upper limit asymptotic determination unit 63.

The heart rate upper limit asymptotic determination unit 63 compares the heart rate value d61b input from the sensor detection value input unit 61 with the heart rate upper limit (normal value upper limit) corresponding to the user 11 recorded in the exercise prescription information database 64, and determines whether or not the heart rate value d61b is equal to or less than the heart rate upper limit (normal value upper limit) registered in the database.

In a case where the heart rate value d61b is equal to or less than the heart rate upper limit (normal value upper limit) registered in the database, the determination information indicating that the heart rate value is within the normal range is output to the instruction information generation unit 65.

The instruction information generation unit 65 generates instruction information d65 indicating a normal value and outputs the generated instruction information to the output unit (display unit or the like) 66.

Meanwhile, in a case where the heart rate upper limit asymptotic determination unit 63 determines that a heart rate value d61b input from the sensor detection value input unit 61 is not less than or equal to the heart rate upper limit (normal value upper limit) registered in the database, the determination information indicating that the heart rate exceeds the upper limit is output to the instruction information generation unit 65.

The instruction information generation unit 65 generates a message urging the user 11 to stop exercise, or generates the instruction information d65 such as a warning sound or lamp light emission, and outputs the generated instruction information d65 to the output unit (display unit or the like) 66.

Specifically, for example, display data as illustrated in Fig. 25(2) is output to the output unit (display unit) 66 of the user terminal 30.

In response to the instruction information d65, the user 11 stops exercise.

With such a configuration, the user 11 can prevent a generation of an excessive cardiac load due to continuous exercise, and can perform safe rehabilitation.

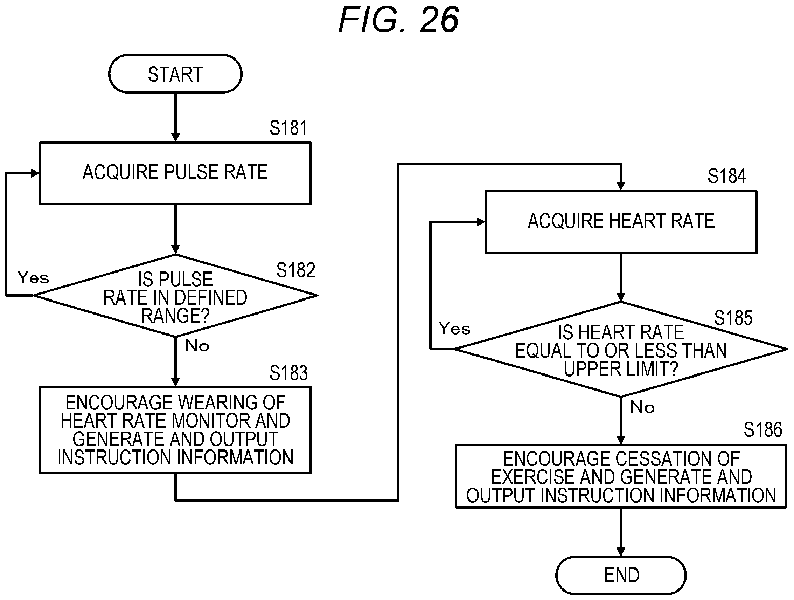

Next, the processing sequence of the third embodiment will be described with reference to the flowchart illustrated in Fig. 26.

Hereinafter, the processing of each step will be described in sequence.

(Step S181)

First, the user terminal 30 acquires the pulse rate value in step S181.

This processing is processing executed by the sensor detection value input unit 61 illustrated in Fig. 23.

The user 11 wears a pulse detection sensor and performs exercise as rehabilitation. A pulse rate value d61a acquired by the sensor detection value input unit 61 is input to a pulse upper limit asymptotic determination unit 62.

(Step S182)

Next, in step S182, the user terminal 30 compares the pulse rate value d61a input from the sensor detection value input unit 61 with the pulse rate defined range (normal value range) corresponding to the user 11 recorded in the exercise prescription information database 64, and determines whether or not the pulse rate value d61a is within the pulse rate defined range (normal value range) registered in the database.

In a case where the pulse rate value d61a is within the pulse rate defined range (normal value range) registered in the database, the process returns to S181 and the pulse rate value acquisition processing is continued.

In this case, note that the determination information indicating that the pulse rate value is within the normal range is output to the instruction information generation unit 65, and the instruction information generation unit 65 may generate the instruction information d65 indicating that the pulse rate value is within the normal range and output the generated instruction information d65 to the output unit (display unit, or the like) 66.

Meanwhile, in step S182, it is determined that the pulse rate value d61a input from the sensor detection value input unit 61 is not within the pulse rate specified range (normal value range) registered in the database, the process proceeds to step S183.

(Step S183)

In step S182, in a case where it is determined that the pulse rate value d61a is not within the pulse rate specified range (normal value range), the process proceeds to step S183.

In this case, in step S183, the user terminal 30 outputs determination information indicating that the pulse rate value d61a is outside the normal range to the instruction information generation unit 65, and the instruction information generation unit 65 generates a message urging the user 11 to wear the heart rate meter for detecting the heart rate value, or the instruction information d65 such as a warning sound and a lamp light emission, and outputs the generated instruction information d65 to the output unit (display unit, or the like) 66.

Specifically, for example, display data as illustrated in Fig. 25(1) is output to the output unit (display unit) 66 of the user terminal 30.

In response to the instruction information d65, the user 11 wears a heart rate meter for detecting a heart rate value and continues exercise as rehabilitation.

(Step S184)

The user 11 wears the heart rate meter for detecting the heart rate value and continues exercise as rehabilitation.

In step S184, the user terminal 30 acquires the heart rate value of the user 11 from the heart rate meter worn by the user 11.

This processing is processing executed by the sensor detection value input unit 61 illustrated in Fig. 23.

The user 11 wears the heart rate meter and executes exercise as rehabilitation. A heart rate value d61b acquired by the sensor detection value input unit 61 is input to a heart rate upper limit asymptotic determination unit 63.

(Step S185)

Next, in step S185, the user terminal 30 compares the heart rate value d61b input from the sensor detection value input unit 61 with the heart rate upper limit (normal value upper limit) corresponding to the user 11 recorded in the exercise prescription information database 64, and determines whether or not the heart rate value d61b is less than or equal to the heart rate upper limit (normal value upper limit) registered in the database.

In a case where the heart rate value d61b is less than or equal to the heart rate upper limit (normal value upper limit) registered in the database, the process returns to S184 and the heart rate value acquisition processing is continued.

In this case, note that the determination information indicating that the pulse rate value is within the normal range is output to the instruction information generation unit 65, and the instruction information generation unit 65 may generate the instruction information d65 indicating that the pulse value is within the normal range and output the generated instruction information d65 to the output unit (display unit, or the like) 66.

Meanwhile, in step S185, in a case where it is determined that the heart rate value d61b input from the sensor detection value input unit 61 is not less than or equal to the heart rate upper limit (normal value upper limit) registered in the database, the process proceeds to step S186.

(Step S186)

In step S185, in a case where it is determined that the heart rate value d61b is not within the heart rate specified range (normal value range), the process proceeds to step S186.

In this case, in step S186, the user terminal 30 outputs determination information indicating that the heart rate value d61b is outside the normal range to the instruction information generation unit 65, and the instruction information generation unit 65 generates a message urging the user 11 to stop exercise, or the instruction information d65 such as a warning sound and a lamp light emission, and outputs the generated instruction information d65 to the output unit (display unit, or the like) 66.

Specifically, for example, display data as illustrated in Fig. 25(2) is output to the output unit (display unit) 66 of the user terminal 30.

In response to the instruction information d65, the user 11 stops exercise.

With such a configuration, the user 11 can prevent a generation of an excessive cardiac load due to continuous exercise, and can perform safe rehabilitation.

[7-2. (Fourth embodiment) Embodiment of presenting route information of walking or running using map]

Next, as a fourth embodiment, an embodiment of presenting route information of walking or running using a map will be described.

The fourth embodiment is an embodiment of providing route information of optimal walking or running using a map in a case where the user 11 performs walking or running as exercise of rehabilitation.

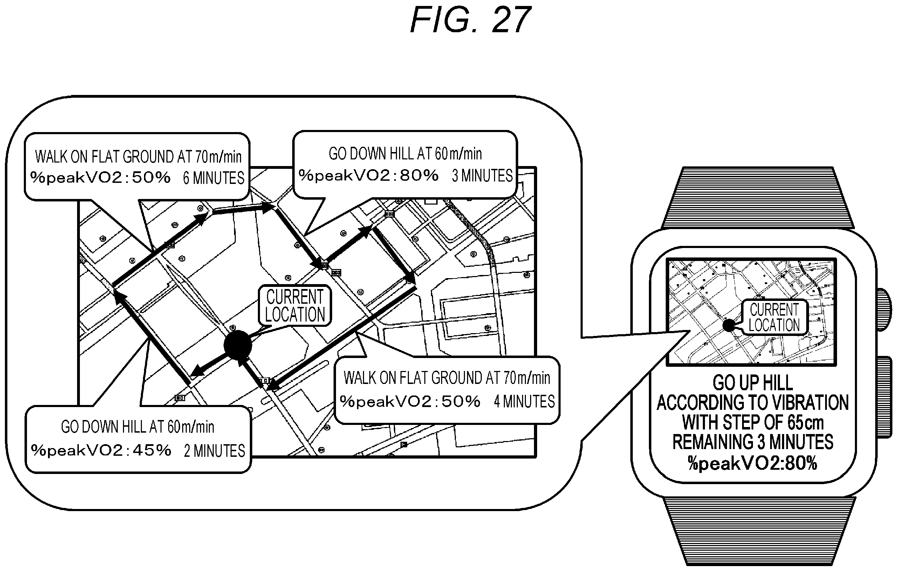

Specifically, for example, as illustrated in Fig. 27, a map is displayed on the display unit of the user terminal 30, and the optimal walking or running route information is provided to the user 11.

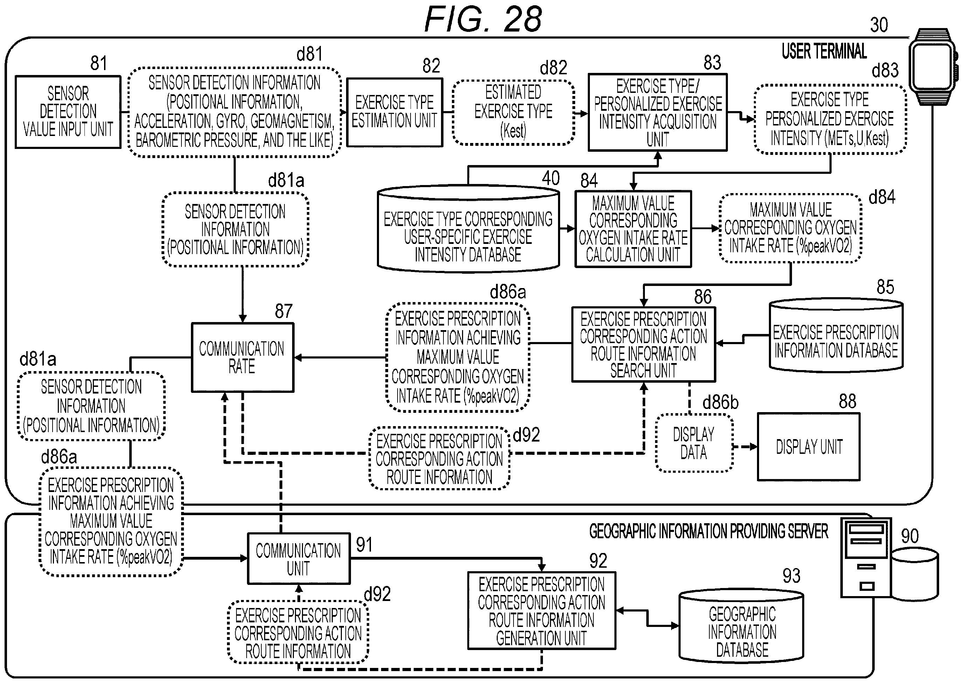

Fig. 28 is a diagram for describing the configurations and processing of the user terminal 30 that executes the processing of the fourth embodiment and a geographic information providing server 90.

The processing of the fourth embodiment using the user terminal 30 and the geographic information providing server 90 will be described with reference to Fig. 28.

The user terminal 30 is a terminal worn on the body while the user 11 who is a rehabilitation performer is performing exercise as rehabilitation.

The sensor detection information input unit 81 of the user terminal 30 acquires sensor detection information d81 of a sensor worn on the body of the user 11, for example, a sensor such as a position sensor, an acceleration sensor, a gyro, a geomagnetic sensor, or an atmospheric pressure sensor, and outputs the acquired sensor detection information d81 to the exercise type estimation unit 82.

Furthermore, in the present embodiment, the sensor detection information (position information) d81a of the position sensor included in the sensor detection information d81, that is, the sensor detection information (position information) d81a which is the position information indicating the position of the user 11 is transmitted to the geographic information providing server 90 via a communication unit 87.

Next, the processing after the exercise type estimation unit 82 of the user terminal 30 will be described.

The exercise type estimation unit 82 estimates the exercise type of the user 11 on the basis of the input sensor detection information d81. For example, the type of exercise being executed by the user 11 is estimated, such as walking (walking speed = 4.0 km/h on flatland walking), running (running speed = 7.0 km/h on flatland running), and AEROBIKE (registered trademark) (load amount = 120 W).

The exercise type estimation unit 82 outputs the estimated exercise type (Kest) as the estimation result to the exercise type/personalized exercise intensity acquisition unit 83.

The exercise type/personalized exercise intensity acquisition unit 83 searches the exercise type corresponding user-specific exercise intensity (METs, U, Kest) database 40 on the basis of the estimated exercise type (Kest) d82 input from the exercise type estimation unit 82, and acquires the exercise type/personalized exercise user-specific exercise intensity (METs, U, Kest) d83 registered in association with the estimated exercise type (Kest).

The exercise type/personalized user-specific exercise intensity (METs, U, Kest) d83 acquired from the database by the exercise type/personalized exercise intensity acquisition unit 83 is output to the maximum value corresponding oxygen intake rate calculation unit 84.

The maximum value corresponding oxygen intake rate calculation unit 84 acquires, from the exercise type corresponding user-specific exercise intensity (METs, U, Kest) database 40, the maximum value of the personalized user-specific exercise intensity (METs, U, Kest) registered in the database.

The maximum value of the personalized user-specific exercise intensity (METs, U, Kest) registered in the exercise type corresponding user-specific exercise intensity (METs, U, Kest) database 40 is set to be an exercise type user-specific exercise intensity maximum value (METsmax, U, Kest).

The maximum value corresponding oxygen intake rate calculation unit 84 inputs the following data:

(a) the exercise type/personalized user-specific exercise intensity (METs, U, Kest) d83 acquired from the exercise type/personalized exercise intensity acquisition unit 83, and

(b) the exercise type user-specific exercise intensity maximum value (METsmax, U, Kest) acquired from the exercise type corresponding user-specific exercise intensity (METs, U, Kest) database 40.

The maximum value corresponding oxygen intake rate calculation unit 84 calculates the maximum value corresponding oxygen intake rate (% peakVO2) d84 from the pieces of data.

The maximum value corresponding oxygen intake rate (% peakVO2) is calculated by the following (Equation 4).

```
Maximum value corresponding oxygen intake rate (% peakVO2) =
(Exercise type/personalized user-specific exercise intensity (METs, U,
Kest))/(exercise type user-specific exercise intensity maximum value
(METsmax, U, Kest))  … (Equation 4)
```

That is, the maximum value corresponding oxygen intake rate (% peakVO2) can be calculated by the ratio of the exercise type/personalized user-specific exercise intensity (METs, U, Kest) to the exercise type user-specific exercise intensity maximum value (METsmax, U, Kest).

Note that, as described above with reference to Fig. 5, the maximum oxygen intake (peakVO2) is the maximum value of oxygen amount (oxygen intake (VO2)) consumed by a body, and the maximum value corresponding oxygen intake rate (% peakVO2) is the ratio of the actual oxygen intake (VO2) to the maximal oxygen intake (peakVO2).

The maximum value corresponding oxygen intake rate (% peakVO2) d84 calculated by the maximum value corresponding oxygen intake rate calculation unit 84 is output to an exercise prescription corresponding action route information search unit 86.

The exercise prescription corresponding action route information search unit 86 inputs the maximum value corresponding oxygen intake rate (% peakVO2) d84 calculated by the maximum value corresponding oxygen intake rate calculation unit 84, and furthermore, refers to the exercise prescription information recorded in the exercise prescription information database 85 to generate exercise prescription information d86a that achieves the maximum value corresponding oxygen intake rate (% peakVO2).

Note that data similar to the data described above with reference to Fig. 14 is recorded in the exercise prescription information database 85.

The exercise prescription information d86a that achieves the maximum value corresponding oxygen intake rate (% peakVO2) generated by the exercise prescription corresponding action route information search unit 86 is transmitted to the geographic information providing server 90 via the communication unit 87.

The geographic information providing server 90 receives the following two pieces of information from the user terminal 30.

(a) Sensor detection information (position information) d81a indicating the current position of the user, and
(b) Exercise prescription information d86a that achieves the maximum value corresponding oxygen intake rate (% peakVO2) generated by the exercise prescription corresponding action route information search unit 86.

The geographic information providing server 90 inputs these two pieces of information via the communication unit 91, and transmits the input information to the exercise prescription corresponding action route information generation unit 92.

The exercise prescription corresponding action route information generation unit 92 generates exercise prescription corresponding action route information d92 using the input information (a) and (b), the map information stored in the geographic information database 93, and the like.

Note that, in addition to the map information, detailed information such as a road on a map is recorded in the geographic information database 93.

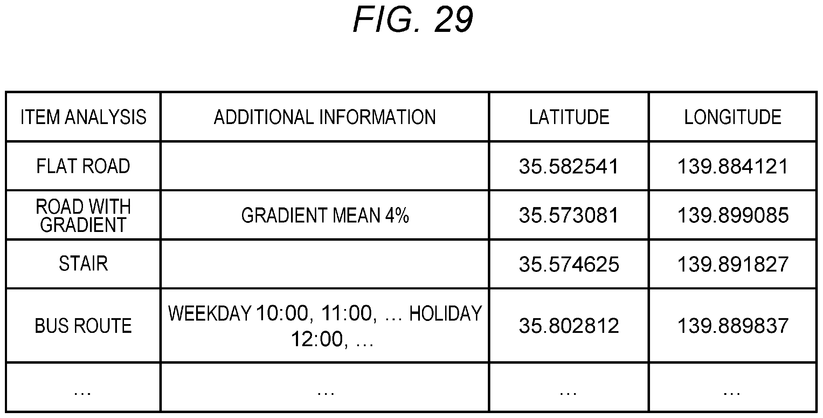

For example, as illustrated in Fig. 29, gradient information of a road on a map, stairs, bus route information, position information of each road, and the like are recorded.

The exercise prescription corresponding action route information generation unit 92 uses the input information (a) and (b), the map information stored in the geographic information database 93 including the detailed information of the road on the map as illustrated in Fig. 29, and the like to generate exercise prescription corresponding action route information d92.

The exercise prescription corresponding action route information d92 includes geographical route information that matches or is similar to the exercise prescription information d86a that achieves the maximum value corresponding oxygen intake rate (% peakVO2) generated by the exercise prescription corresponding action route information search unit 86 of the user terminal 30.

Specifically, it is data including route information on a map on which the user can move as illustrated in Fig. 27.

The geographic information providing server 90 transmits the exercise prescription corresponding action route information d92 generated by the exercise prescription corresponding action route information generation unit 92 to the user terminal 30 via the communication unit 91.

The user terminal 30 receives the exercise prescription corresponding action route information d92 transmitted by the geographic information providing server 90 via the communication unit 87 and transmits the received exercise prescription corresponding action route information d92 to the exercise prescription corresponding route information search unit 86.

The exercise prescription corresponding route information search unit 86 generates display data d86b on the basis of the exercise prescription corresponding action route information d92 received from the geographic information providing server 90 and the exercise prescription information recorded in the exercise prescription information database 85, and displays the display data d86b on the display unit 88.

The display data d86b generated by the exercise prescription corresponding route information search unit 86 is display data including information indicating an exercise type and a route to be executed by the user on the map as illustrated in Fig. 27.

The user 11 can check exercise performed by a user, a walking route or a walking speed in a device body by viewing the display data to perform an appropriate exercise as rehabilitation.

[7-3. (Fifth embodiment) Embodiment of performing exercise prescription update processing]

Next, as a fifth embodiment, an embodiment of performing exercise prescription update processing is performed will be described.

After the user 11 is discharged from the hospital, since the period during which the user performs the exercise as the rehabilitation may extend over a long period of time, and the effect of the rehabilitation varies depending on a person, the exercise to be executed as the rehabilitation may be different from the exercise prescription information set at the time of discharge.

In such a case, a rehabilitation supervisor such as a doctor in a hospital needs to update the exercise prescription and sequentially provide the updated exercise prescription information to the user 11.

The fifth embodiment is an embodiment that enables such an exercise prescription update processing.

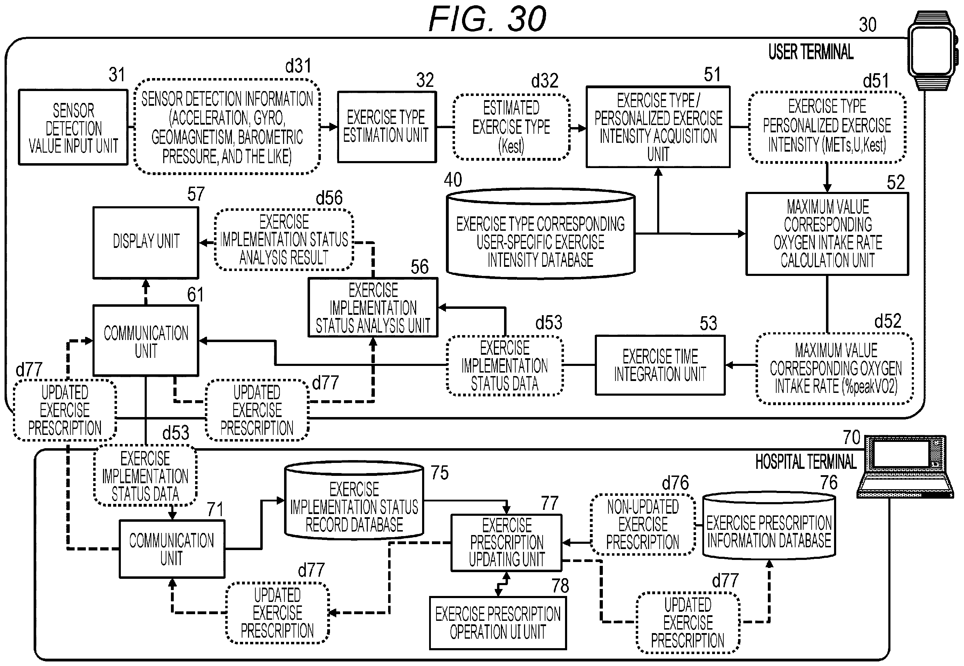

A configuration and processing for executing the exercise prescription update processing will be described with reference to Fig. 30 and subsequent drawings.

Fig. 30 is a diagram illustrating the configurations and processing of the user terminal 30 and the hospital terminal 70 that execute the exercise prescription update processing of this fifth embodiment.

The configuration of the user terminal 30 is substantially similar to the configuration of the user terminal 30 illustrated in Fig. 20 described above as second embodiment, and each component is denoted by reference numerals similar to that in Fig. 20.

The configurations of the user terminal 30 and the hospital terminal 70 illustrated in Fig. 30 and the processing to be executed will be described.

The user terminal 30 is a terminal worn on the body while the user 11 who is a rehabilitation performer is executing an exercise as rehabilitation.

The sensor detection information input unit 31 of the user terminal 30 acquires sensor detection information d31 of a sensor worn on the body of the user 11, for example, a sensor such as an acceleration sensor, a gyro, a geomagnetic sensor, or an atmospheric pressure sensor, and outputs the sensor detection information d31 to the exercise information estimation unit 32.

The exercise information estimation unit 32 estimates the exercise type being executed by the user 11 on the basis of the input sensor detection information d31.

The exercise type estimation unit 32 outputs the estimated exercise type (Kest) as the estimation result to the exercise type/personalized exercise intensity acquisition unit 51.

The exercise type/personalized exercise intensity acquisition unit 51 searches the exercise type corresponding user-specific exercise intensity (METs, U, Kest) database 40 on the basis of the estimated exercise type (Kest) d32 input from the exercise type estimation unit 32, and acquires the exercise type/personalized user-specific exercise intensity (METs, U, Kest) d51 registered in association with the estimated exercise type (Kest).

The exercise type/personalized user-specific exercise intensity (METs, U, Kest) d51 acquired from the database by the exercise type/personalized exercise intensity acquisition unit 51 is output to a maximum value corresponding oxygen intake rate calculation unit 52.

The maximum value corresponding oxygen intake rate calculation unit 52 acquires, from the exercise type corresponding user-specific exercise intensity (METs, U, Kest) database 40, the maximum value of the personalized user-specific exercise intensity (METs, U, Kest) registered in the database.

The maximum value corresponding oxygen intake rate calculation unit 52 includes:

(a) the exercise type/personalized user-specific exercise intensity (METs, U, Kest) d51 acquired from the exercise type/personalized exercise intensity acquisition unit 51, and

(b) the exercise type user-specific exercise intensity maximum value (METsmax, U, Kest) acquired from the exercise type corresponding user-specific exercise intensity (METs, U, Kest) database 40.

From the pieces of data, the maximum value corresponding oxygen intake rate (% peakVO2) d52 is calculated.

The maximum value corresponding oxygen intake rate (% peakVO2) d52 calculated by the maximum value corresponding oxygen intake rate calculation unit 52 is output to an exercise time integration unit 53.

The exercise time integration unit 53 inputs the maximum value corresponding oxygen intake rate (% peakV02) d52 calculated by the maximum value corresponding oxygen intake rate calculation unit 52, and generates the exercise implementation status data d53 of the user 11.

The exercise implementation status data d53 of the user 11 generated by the exercise time integration unit 53 is input to the exercise implementation status analysis unit 56 and transmitted to the hospital terminal 70 via the communication unit 61.

The hospital terminal 70 receives the exercise implementation status data d53 transmitted from the user terminal 30 via the communication unit 71, and records the received exercise implementation status data d53 in the exercise implementation status record database 75.

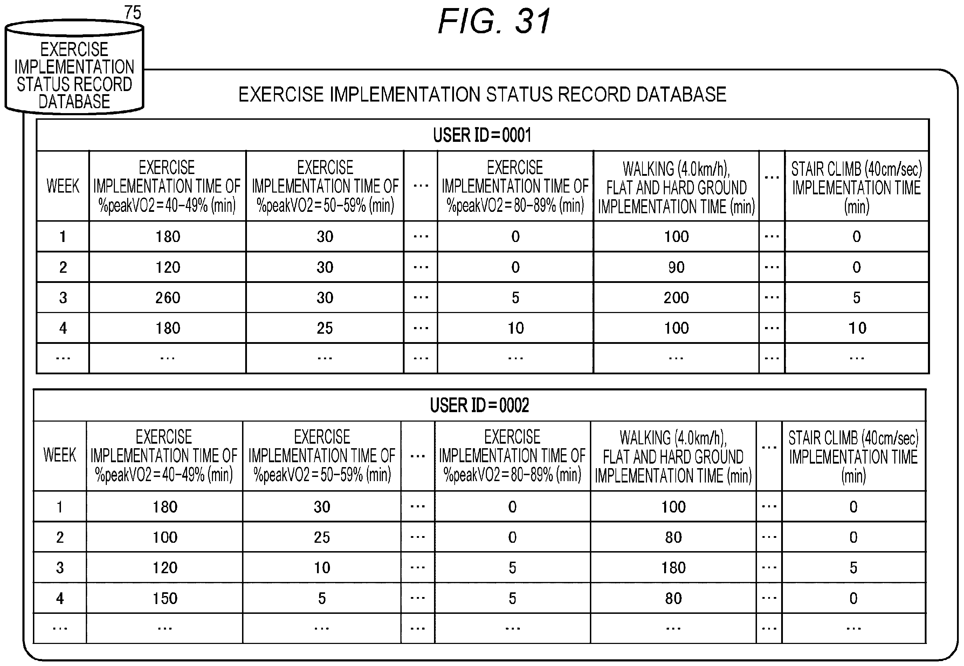

Fig. 31 illustrates an example of the exercise implementation status record database 75 stored in the storage unit of the hospital terminal 70.

As illustrated in Fig. 31, the exercise implementation status record data of the user unit is recorded in the exercise implementation status record database 75 stored in the storage unit of the hospital terminal 70.

The exercise time executed by each user is recorded in the exercise implementation status record data of the user unit in a predetermined section unit of the maximum value corresponding oxygen intake rate (% peakVO2). Furthermore, a configuration in which an exercise time corresponding to the exercise type (K) is also recorded is included.

Note that the example illustrated in Fig. 31 is an example in which record data is generated and recorded on a weekly basis. That is, the above example is a data recording example in which the exercise time executed by each user in one week is recorded in a predetermined section unit of the maximum value corresponding oxygen intake rate (% peakVO2), and furthermore, recorded in an exercise type unit.

An exercise prescription updating unit 77 of the hospital terminal 70 analyzes the received exercise implementation status data d53 received from the user terminal 30.

The exercise prescription updating unit 77 of the hospital terminal 70 performs the update processing of the exercise prescription information corresponding to the user 11 recorded in the exercise prescription information database 76 as necessary in the analysis processing of the exercise implementation status data.

That is, as illustrated in Fig. 30, the exercise prescription updating unit 77 reads a non-updated exercise prescription d76 corresponding to the user 11 recorded in the exercise prescription information database 76, generates a new updated exercise prescription d77 according to the analysis result of the received exercise implementation status data d53 received from the user terminal 30, and records the generated updated exercise prescription d77 in the exercise prescription information database 76.

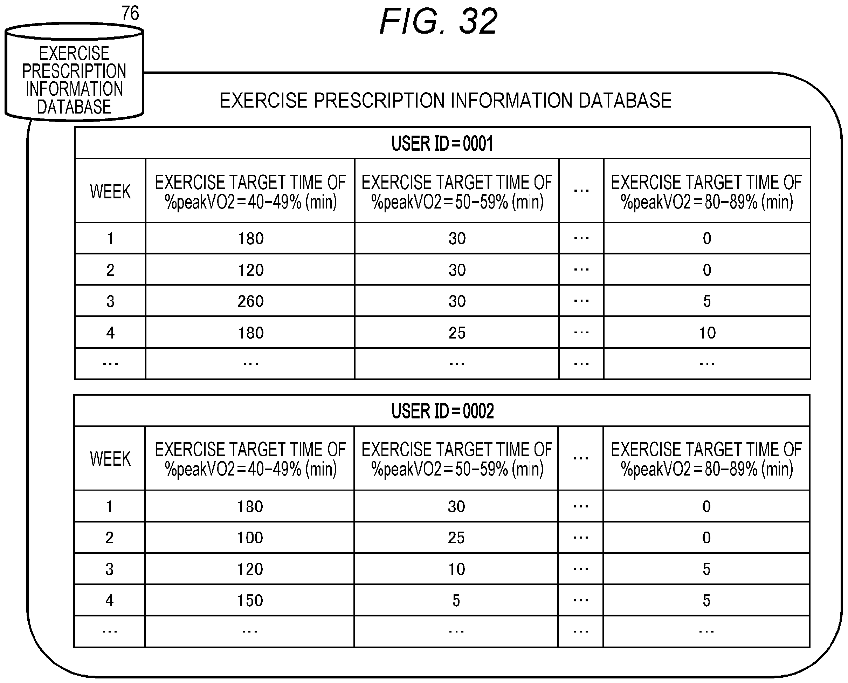

Fig. 32 illustrates an example of the exercise prescription information database 76 stored in the storage unit of the hospital terminal 70.

As illustrated in Fig. 32, similarly to the exercise implementation status record database 75 described above with reference to Fig. 31, the exercise time to be executed by each user is recorded in the exercise prescription information database 76 in a predetermined section unit of the maximum value corresponding oxygen intake rate (% peakVO2).

Note that, similarly to the exercise implementation status record database 75 illustrated in Fig. 31 described above, an example in which the exercise prescription information database 76 illustrated in Fig. 32 records the exercise target time in units of weeks is illustrated.

The exercise prescription update processing in the exercise prescription updating unit 77 is executed, for example, under the confirmation of a rehabilitation supervisor such as a doctor.

Specifically, for example, the exercise prescription information for each user is displayed on an exercise prescription operation UI unit 78, a rehabilitation supervisor such as a doctor confirms display data, and the change processing of the exercise prescription, that is, the update processing is performed as necessary.

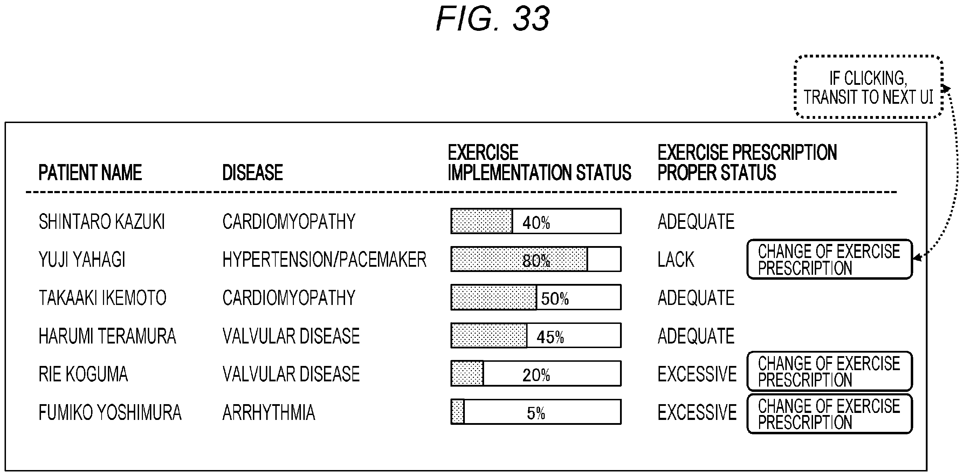

Fig. 33 is one display example of the exercise prescription information for each user displayed on the exercise prescription operation UI unit 78.

In a case where a rehabilitation supervisor such as a doctor confirms this display data and determines that it is necessary to perform the change processing of the exercise prescription, an update icon on a right end, that is, the update processing, is clicked.

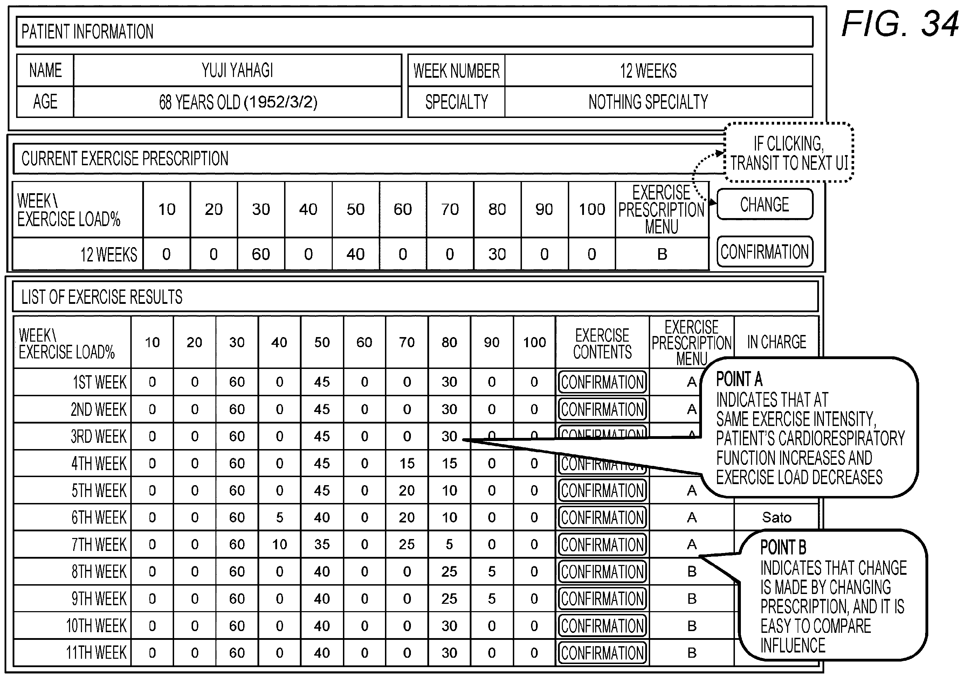

By the click processing, for example, detailed exercise prescription information corresponding to an individual user as illustrated in Fig. 34 is displayed.

The personalized exercise prescription information illustrated in Fig. 34 is information indicating the current exercise prescription (twelfth week) in a middle part and the past (first to eleventh weeks) exercise record in a lower part.

The current (twelfth week) exercise prescription in the middle part is information indicating a time (minute (min)) of exercise to be executed by a user (rehabilitation performer) on a weekly basis by dividing the exercise time for each exercise load.

The past (first week to eleventh week) exercise record in the lower part is information indicating a time (minute (min)) of exercise to be actually executed by a user (rehabilitation performer) on a weekly basis by dividing the exercise time for each exercise load.

A point A indicated in the past (first week to eleventh week) exercise records in the lower part is a point where it is confirmed that the exercise load has shifted from 80% to 70% in a period from the third week to the fourth week.

The point A is a point indicating that, in a case where the user (rehabilitation performer) performs an exercise with the same exercise intensity, a cardiopulmonary function of the user increases and the exercise load decreases.

In addition, the point B indicates a point at which the exercise prescription is switched from menu A to menu B. The point B indicates a time point when the exercise prescription is updated, and it is easy to confirm the influence of the exercise prescription update by clearly indicating the exercise prescription menu in each week.

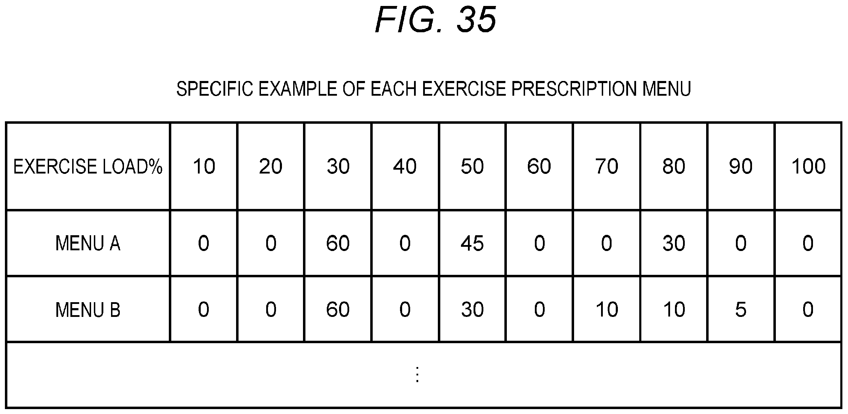

Fig. 35 illustrates specific examples of the exercise prescription menu A and the exercise prescription menu B. As described above, each menu is information in which the time (minute (min)) of the exercise to be executed by the user is shown by being divided for each exercise load.

When the "confirmation" icon indicated in the past (first week to eleventh week) exercise records in the lower part of Fig. 34 is clicked, specific exercise contents of the week are displayed.

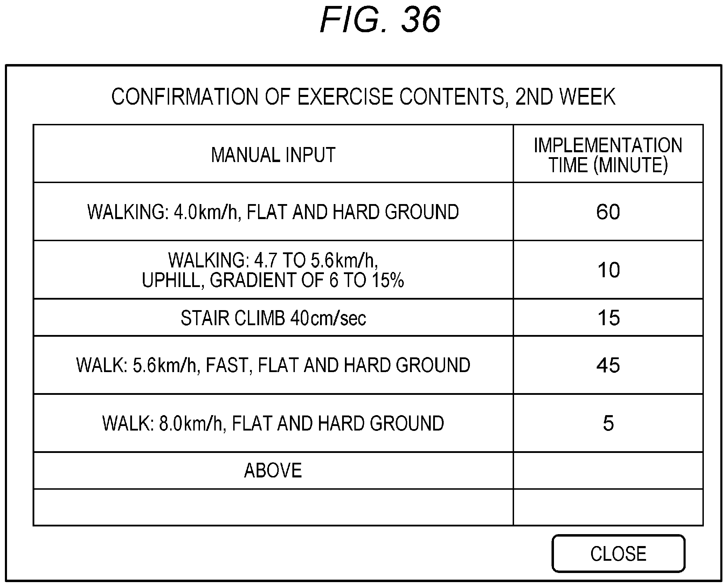

For example, as illustrated in Fig. 36, detailed information indicating the contents of the exercise actually executed by the user in the week is displayed.

In a case where a rehabilitation supervisor such as a doctor confirms these display data and determines that it is necessary to perform the change processing, that is, the update processing of the exercise prescription, a "change icon" on the right end of the current (twelfth week) exercise prescription in the middle part of the display data illustrated in Fig. 34 is clicked.

By this click processing, the change processing of the exercise prescription, that is, a UI with a display screen for the update processing is displayed.

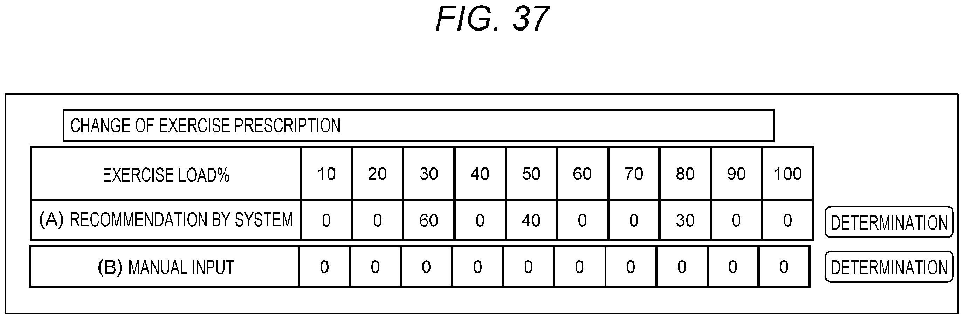

Fig. 37 illustrates a specific example of the UI for the update process.

As illustrated in Fig. 37, a table in which the exercise time (minute (min)) to be executed by the user on a weekly basis can be divided and set for each exercise load unit is displayed on the UI for the update processing.

In this table, recommendation data ((A) recommendation by the system) automatically calculated by the system on the basis of exercise results of the user and the like, and a manual input field ((B) manual input) by a rehabilitation supervisor such as a doctor are set.

The rehabilitation supervisor such as a doctor can also select recommendation data by (A) the system, and can also input arbitrary data in a manual input field ((B) manual input).

Furthermore, the update exercise prescription for the current week is determined by selecting one of (A) and (B) and clicking a determination icon on the right end.

The operation information in the exercise prescription operation UI unit 78 is input to the exercise prescription updating unit 77, and the exercise prescription updating unit 77 performs the updating processing of the exercise prescription according to the operation information in the exercise prescription operation UI unit 78.

The exercise prescription updating unit 77 records the new updated exercise prescription d77 generated according to the operation information in the exercise prescription operation UI unit 78 in the exercise prescription information database 76, and transmits the recorded updated exercise prescription d77 to the user terminal 30 via the communication unit 71.

The user terminal 30 receives the updated exercise prescription d77 transmitted by the hospital terminal 70 via the communication unit 61 and inputs the received updated exercise prescription d77 to the exercise implementation status analysis unit 56.

The exercise implementation status analysis unit 56 compares the exercise implementation status data d53 of the user 11 generated by the exercise time integration unit 53 with the latest updated exercise prescription d77 received from the hospital terminal 70, generates the exercise implementation status analysis result d56, and outputs the generated exercise implementation status analysis result d56 to the display unit 57.

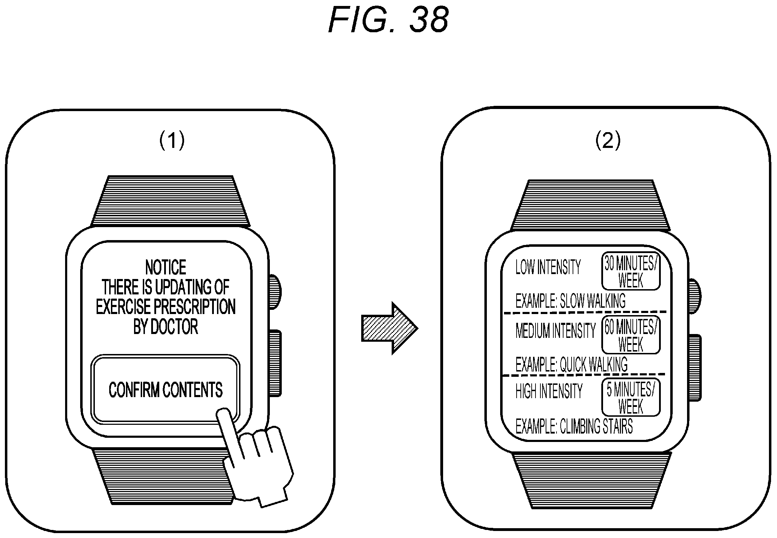

Note that the display data in a case where the exercise prescription information is updated is, for example, display data as illustrated in Fig. 38.

Fig. 38(1) is an initial screen of the display data in a case where the exercise prescription information is updated, and is a rehabilitation performer. The data is display data for notifying the user 11 that the exercise prescription information has been updated.

Fig. is the display screen of the updated exercise prescription information to be transitioned by touching "confirm contents" illustrated in (1).

The user 11 who is the rehabilitation performer can know that the exercise prescription information has been updated on the hospital side by viewing these pieces of display data, and can further confirm the specific updated exercise prescription information.

[8. Hardware configuration example of information processing apparatus]

Next, a hardware configuration example of the user terminal 30, the hospital terminal 70, and the information processing apparatus constituting the geographic information providing server 90 used in the processing of the present disclosure will be described.

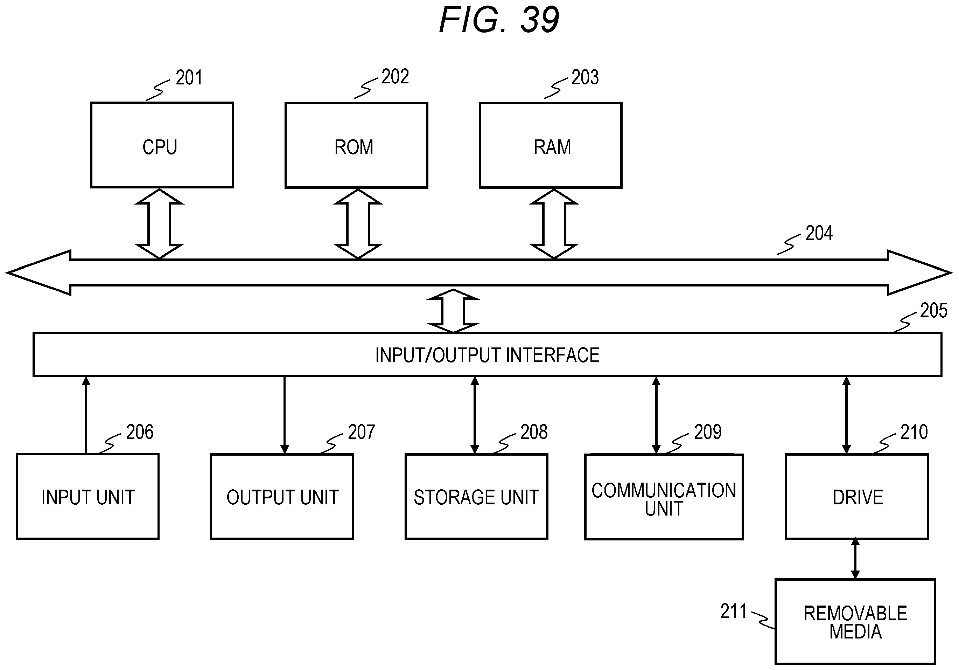

Fig. 39 is a diagram illustrating an example of the hardware configuration of the information processing apparatus constituting each of these devices.

The hardware configuration illustrated in Fig. 39 will be described.

A central processing unit (CPU) 201 functions as a data processing unit that executes various processes according to a program stored in a read only memory (ROM) 202 or a storage unit 208. For example, processing according to the sequence described in the above-described embodiment is executed.

A random access memory (RAM) 203 stores programs executed by the CPU 201, data, and the like. The CPU 201, the ROM 202, and the RAM 203 are mutually connected by a bus 204.

The CPU 201 is connected to an input/output interface 205 via the bus 204, and the input/output interface 205 is connected to an input unit 206 including various switches, a keyboard, a touch panel, a mouse, a microphone, a status data acquisition unit such as a sensor, and the like, and an output unit 207 including a display, a speaker, and the like.

The CPU 201 inputs commands, situation data, and the like input from the input unit 206, executes various types of processing, and outputs processing results to the output unit 207, for example.

The storage unit 208 connected to the input/output interface 205 includes, for example, a hard disk and the like, and stores programs executed by the CPU 201 and various data. The communication unit 209 functions as a transmission/reception unit for data communication via a network such as the Internet or a local area network, and communicates with an external device.

A drive 210 connected to the input/output interface 205 drives a removable medium 211 such as a magnetic disk, an optical disk, a magneto-optical disk, or a semiconductor memory such as a memory card, and records or reads data.

[9. Summary of configuration of present disclosure]

Hereinabove, the embodiments of the present disclosure have been described in detail with reference to specific embodiments. However, it is obvious that those skilled in the art can make modifications and substitutions of the embodiments without departing from the gist of the present disclosure. That is, the present invention has been disclosed in the form of exemplification, and should not be interpreted in a limited manner. In order to determine the gist of the present disclosure, the claims should be taken into consideration.

Note that the technology disclosed in the present specification can have the following configurations.

(1) An information processing apparatus, including:

an exercise type estimation unit configured to estimate an exercise type executed by a user on the basis of sensor detection information;
a database configured to store user-specific exercise intensity corresponding to the exercise type;
an exercise type/personalized exercise intensity acquisition unit configured to acquire, from the database, an exercise type/personalized exercise intensity that is the user-specific exercise intensity corresponding to the exercise type estimated by the exercise type estimation unit; and
an exercise implementation status analysis unit configured to analyze exercise implementation status data generated on the basis of the exercise type/personalized exercise intensity acquired from the database, and generate an exercise implementation status analysis result indicating whether or not exercise being executed by the user is an exercise according to a pre-generated exercise prescription.

(2) The information processing apparatus disclosed in (1), in which

the database is an exercise type corresponding user-specific exercise intensity database that stores user corresponding exercise intensity corresponding to an exercise type, and

the exercise implementation status analysis unit analyzes exercise implementation status data generated on the basis of the exercise type/personalized exercise intensity acquired from the exercise type corresponding user-specific exercise intensity database, and generates the exercise implementation status analysis result indicating whether or not the exercise being executed by the user is the exercise according to the pre-generated exercise prescription.

(3) The information processing apparatus disclosed in (2), in which

the exercise type corresponding user-specific exercise intensity database is a database that stores exercise intensity obtained by analyzing exhalation analysis result data obtained by allowing the user to execute exercise while wearing an exhaled gas analyzer.

(4) The information processing apparatus disclosed in any one of (1) to (3), in which

the user is a user executing an exercise as rehabilitation according to the pre-generated exercise prescription, and

the exercise implementation status analysis unit compares the exercise implementation status data generated on the basis of the exercise type/personalized exercise intensity with data stored in an exercise prescription information database storing the pre-generated exercise prescription, and generates the exercise implementation status analysis result indicating whether or not the exercise being executed by the user is an exercise according to the exercise prescription.

(5) The information processing apparatus disclosed in (4), in which

the information processing apparatus includes an exercise implementation status record database that stores the exercise implementation status data, and

the exercise implementation status analysis unit acquires the exercise implementation status data of the user from the exercise implementation status record database, and generates the exercise implementation status analysis result indicating whether or not the exercise being executed by the user is the exercise according to an exercise prescription.

(6) The information processing apparatus disclosed in any one of (1) to (5), further including:

a maximum value corresponding oxygen intake rate calculation unit configured to input the exercise type/personalized exercise intensity acquired by the exercise type/personalized exercise intensity acquisition unit, and calculate a maximum value corresponding oxygen intake rate according to the exercise type executed by the user; and

an exercise time integration unit configured to generate, as exercise implementation status data, an exercise time integration result of an exercise corresponding to each of the maximum value corresponding oxygen intake rates calculated by the maximum value corresponding oxygen intake rate calculation unit,

in which the exercise implementation status analysis unit generates the exercise implementation status analysis result indicating whether or not the exercise being executed by the user is the exercise according to the exercise prescription is generated on the basis of the exercise implementation status data generated by the exercise time integration unit.

(7) The information processing apparatus disclosed in any one of (1) to (6), in which

the exercise intensity is metabolic equivalents (METs).

(8) The information processing apparatus disclosed in (7), in which

the database is a database that stores user-specific METs as exercise intensity corresponding to the user-specific exercise type.

(9) The information processing apparatus disclosed in (8), in which

the user-specific METs that are an exercise intensity corresponding to the user-specific exercise type are METs that are calculated in consideration of the exhalation analysis result data obtained by allowing the user to execute the exercise while wearing the exhaled gas analyzer and a weight of the user.

(10) The information processing apparatus disclosed in any one of (1) to (9), in which

the exercise implementation status analysis unit outputs the exercise implementation status analysis result indicating whether or not the exercise being executed by the user is the exercise according to the pre-generated exercise prescription to a display unit.

(11) An information processing apparatus, including:

an exercise type estimation unit configured to estimate an exercise type executed by a user on the basis of sensor detection information;

a steady state activity amount analysis unit configured to input an estimated exercise type estimated by the exercise type estimation unit and an exhaled gas analysis result of the user to calculate an exhalation analysis result-based exercise type/personalized activity amount;

an exercise intensity-based exercise type/personalized activity amount calculation unit configured to acquire an exercise type corresponding standard exercise intensity estimated by the exercise type estimation unit from an exercise type corresponding standard exercise intensity database storing the exercise type corresponding standard exercise intensity, and calculate an exercise intensity-based exercise type/personalized activity amount on the basis of the acquired standard exercise intensity and a weight of the user; and

an exercise type/personalized exercise intensity calculation unit configured to calculate an exercise type/personalized exercise intensity that is the user corresponding exercise intensity corresponding to the exercise type on the basis of each data of (a) an exhalation analysis result-based exercise type/personalized activity amount and (b) the exercise intensity-based exercise type/personalized activity amount.

(12) The information processing apparatus disclosed in (11), further including:

a personalized exercise intensity correction factor calculation unit configured to calculate a personalized exercise intensity correction factor corresponding to the user by inputting each data of (a) the exhalation analysis result-based exercise type/personalized activity amount and (b) the exercise intensity-based exercise type/personalized activity amount,

in which the exercise type/personalized exercise intensity calculation unit calculates the exercise type/personalized exercise intensity that is the user corresponding exercise intensity by using the personalized exercise intensity correction factor.

(13) The information processing apparatus disclosed in (12), in which
the exercise type/personalized exercise intensity calculation unit calculates the exercise type/personalized exercise intensity by using information on the weight of the user.

(14) The information processing apparatus disclosed in any one of (11) to (13), in which
the exercise type/personalized exercise intensity calculation unit records the calculated exercise type/personalized exercise intensity in an exercise type corresponding user-specific exercise intensity database.

(15) An information processing system, including:

a user terminal; and a hospital terminal,
in which the user terminal includes:

an exercise type estimation unit configured to estimate an exercise type executed by a user on the basis of sensor detection information;

a database configured to store user-specific exercise intensity corresponding to the exercise type;

an exercise type/personalized exercise intensity acquisition unit configured to acquire, from the database, an exercise type/personalized exercise intensity that is the user-specific exercise intensity corresponding to the exercise type estimated by the exercise type estimation unit; and

a communication unit configured to transmit exercise implementation status data generated on the basis of the exercise type/personalized exercise intensity acquired from the database to the hospital terminal,

the hospital terminal includes:

an exercise implementation status analysis unit configured to analyze the exercise implementation status data received from the user terminal and generate an exercise implementation status analysis result indicating whether or not the exercise being executed by the user is the exercise according to the pre-generated exercise prescription; and

a communication unit configured to transmit the exercise implementation status analysis result generated by the exercise implementation status analysis unit to the user terminal, and

the user terminal displays the exercise implementation status analysis result received from the hospital terminal on a display unit.

(16) An information processing system, including:

a user terminal; and a geographic information providing server,

in which the user terminal includes:

an exercise type estimation unit configured to estimate an exercise type executed by a user on the basis of sensor detection information;

a database configured to store user-specific exercise intensity corresponding to the exercise type;

an exercise type/personalized exercise intensity acquisition unit configured to acquire, from the database, an exercise type/personalized exercise intensity that is the user-specific exercise intensity corresponding to the exercise type estimated by the exercise type estimation unit;

a maximum value corresponding oxygen intake rate calculation unit configured to input the exercise type/personalized exercise intensity acquired by the exercise type/personalized exercise intensity acquisition unit, and calculate a maximum value corresponding oxygen intake rate according to the exercise type executed by the user; and

a communication unit configured to generate exercise prescription information for realizing the maximum value corresponding oxygen intake rate on the basis of the maximum value corresponding oxygen intake rate calculated by the maximum value corresponding oxygen intake rate calculation unit and transmit the generated exercise prescription information to the geographic information providing server,

the geographic information providing server includes:

an exercise prescription corresponding action route information generation unit configured to analyze the exercise prescription information received from the user terminal and generate the exercise prescription corresponding action route information for realizing the exercise prescription information; and

a communication unit configured to transmit the exercise prescription corresponding action route information to the user terminal, and

the user terminal displays the exercise prescription corresponding action route information received from the geographic information providing server on a display unit.

(17) An information processing method executed by an information processing apparatus, the method including:

an exercise type estimation step of estimating, by an exercise type estimation unit, an exercise type executed by a user on the basis of sensor detection information;

an exercise type/personalized exercise intensity acquisition step of acquiring, by an exercise type/personalized exercise intensity acquisition unit, from the database, an exercise type/personalized exercise intensity that is the user-specific exercise intensity corresponding to the exercise type estimated by the exercise type estimation unit; and

an exercise implementation status analysis step of analyzing, by an exercise implementation status analysis unit, exercise implementation status data generated on the basis of the exercise type/personalized exercise intensity acquired from the database, and generating an exercise implementation status analysis result indicating whether or not exercise being executed by the user is an exercise according to a pre-generated exercise prescription.

(18) An information processing method executed by an information processing apparatus, the method including:

an exercise type estimation step of estimating, by an exercise type estimation unit, an exercise type executed by a user on the basis of sensor detection information;

a steady state activity amount analysis step of inputting, by a steady state activity amount analysis unit, an estimated exercise type estimated by the exercise type estimation unit and an exhaled gas analysis result of the user to calculate an exhalation analysis result-based exercise type/personalized activity amount;

an exercise intensity-based exercise type/personalized activity amount calculation step of acquiring, by an exercise intensity-based exercise type/personalized activity amount calculation unit, an exercise type corresponding standard exercise intensity estimated by the exercise type estimation unit from an exercise type corresponding standard exercise intensity database storing the exercise type corresponding standard exercise intensity, and calculating an exercise intensity-based exercise type/personalized activity amount on the basis of the acquired standard exercise intensity and a weight of the user; and

an exercise type/personalized exercise intensity calculation step of calculating, by an exercise type/personalized exercise intensity calculation unit, an exercise type/personalized exercise intensity that is the user corresponding exercise intensity corresponding to the exercise type on the basis of each data of (a) an exhalation analysis result-based exercise type/personalized activity amount and (b) the exercise intensity-based exercise type/personalized activity amount.

(19) A program for allowing an information processing apparatus to execute information processing, the program executing:

an exercise type estimation step of estimating, by an exercise type estimation unit, an exercise type executed by a user on the basis of sensor detection information;
an exercise type/personalized exercise intensity acquisition step of acquiring, by an exercise type/personalized exercise intensity acquisition unit, from the database, an exercise type/personalized exercise intensity that is the user-specific exercise intensity corresponding to the exercise type estimated by the exercise type estimation unit; and
an exercise implementation status analysis step of analyzing, by an exercise implementation status analysis unit, exercise implementation status data generated on the basis of the exercise type/personalized exercise intensity acquired from the database, and generating an exercise implementation status analysis result indicating whether or not exercise being executed by the user is an exercise according to a pre-generated exercise prescription.

(20) A program for allowing an information processing apparatus to execute information processing, the program executing:

an exercise type estimation step of estimating, by an exercise type estimation unit, an exercise type executed by a user on the basis of sensor detection information;
a steady state activity amount analysis step of inputting, by a steady state activity amount analysis unit, an estimated exercise type estimated by the exercise type estimation unit and an exhaled gas analysis result of the user to calculate an exhalation analysis result-based exercise type/personalized activity amount;
an exercise intensity-based exercise type/personalized activity amount calculation step of acquiring, by an exercise intensity-based exercise type/personalized activity amount calculation unit, an exercise type corresponding standard exercise intensity estimated by the exercise type estimation unit from an exercise type corresponding standard exercise intensity database storing the exercise type corresponding standard exercise intensity, and calculating an exercise intensity-based exercise type/personalized activity amount on the basis of the acquired standard exercise intensity and a weight of the user; and
an exercise type/personalized exercise intensity calculation step of calculating, by an exercise type/personalized exercise intensity calculation unit, an exercise type/personalized exercise intensity that is the user corresponding exercise intensity corresponding to the exercise type on the basis of each data of (a) an exhalation analysis result-based exercise type/personalized activity amount and (b) the exercise intensity-based exercise type/personalized activity amount.

Note that the series of processing described in the specification can be executed by hardware, software, or a combined configuration of both. In the case of executing processing by software, a program recording a processing sequence can be installed and executed in a memory in a computer incorporated in dedicated hardware, or the program can be installed and executed in a general-purpose computer capable of executing various types of processing. For example, the program can be recorded in advance in a recording medium. In addition to installation from the recording medium to the computer, the program can be received via a network such as a local area network (LAN) or the Internet and installed in a recording medium such as a built-in hard disk.

In addition, various types of processing described in the specification may be executed not only in time series according to the description but also in parallel or individually according to the processing capability of the device that executes the processing or as necessary. In addition, in the present specification, a system is a logical set configuration of a plurality of devices, and is not limited to a system in which devices of respective configurations are in the same housing.

INDUSTRIAL APPLICABILITY

As described above, according to the configuration of one embodiment of the present disclosure, a configuration in which rehabilitation can be appropriately executed while confirming whether or not exercise being executed by a rehabilitation execution user is an appropriate exercise according to an exercise prescription can be achieved.

Specifically, for example, the configuration includes an exercise type estimation unit configured to estimate an exercise type executed by a user on the basis of detection information of a sensor worn on a body of the user, a database configured to store user-specific exercise intensity corresponding to the exercise type, an exercise type/personalized exercise intensity acquisition unit configured to acquire, from the database, an exercise type/personalized exercise intensity that is user-specific exercise intensity corresponding to the exercise type estimated by the exercise type estimation unit, and an exercise implementation status analysis unit configured to analyze exercise implementation status data generated on the basis of the exercise type/personalized exercise intensity acquired from the database and generate an exercise implementation status analysis result indicating whether or not exercise being executed by the user is an exercise according to a pre-generated exercise prescription, in which the generated exercise implementation status analysis result is output to a display unit.

By the present configuration, a configuration that enables a rehabilitation execution user to appropriately execute rehabilitation while confirming whether or not exercise being executed by the user is an appropriate exercise according to an exercise prescription is achieved.

REFERENCE SIGNS LIST

| | |
|---|---|
| 11 | User |
| 12 | Sensor |
| 20 | Exhaled gas analyzer |
| 21 | Activity amount acquisition unit |
| 30 | User terminal |
| 31 | Sensor detection value input unit |
| 32 | Exercise type estimation unit |
| 33 | Steady state activity amount analysis unit |
| 34 | Exercise type corresponding standard exercise intensity database |
| 35 | Personal information database |
| 36 | Exercise type/personalized exercise intensity acquisition unit |
| 37 | Exercise intensity-based exercise type/personalized activity amount calculation unit |
| 38 | Personalized exercise intensity correction factor calculation unit |
| 39 | Exercise type/personalized exercise intensity calculation unit |
| 40 | Exercise type corresponding user-specific exercise intensity database |
| 51 | Exercise type/personalized exercise intensity acquisition unit |
| 52 | Maximum value corresponding oxygen intake rate calculation unit |
| 53 | Exercise time integration unit |
| 54 | Exercise implementation status record database |
| 55 | Exercise prescription information database |
| 56 | Exercise implementation status analysis unit |
| 57 | Display unit |
| 61 | Communication unit |
| 62 | Pulse upper limit asymptotic determination unit |
| 63 | Heart rate upper limit asymptotic determination unit |
| 64 | Exercise prescription information database |
| 65 | Instruction information generation unit |
| 66 | Output unit (display unit) |
| 70 | Horizontal terminal |
| 71 | Communication unit |
| 72 | Exercise implementation status analysis unit |
| 75 | Exercise implementation status record database |
| 76 | Exercise prescription information database |
| 77 | Exercise prescription updating unit |
| 78 | Exercise prescription operation UI |
| 81 | Sensor detection value input unit |
| 82 | Exercise type estimation unit |
| 83 | Exercise type/personalized exercise intensity acquisition unit |
| 84 | Maximum value corresponding oxygen intake rate calculation unit |
| 85 | Exercise prescription information database |
| 86 | Exercise prescription corresponding action route search unit |
| 87 | Communication unit |
| 90 | Geographic information providing server |
| 91 | Communication unit |
| 92 | Exercise prescription corresponding action route information generation unit |
| 201 | CPU |

202    ROM
203    RAM
204    Bus
205    Input/output interface
206    Input unit
207    Output unit
208    Storage unit
209    Communication unit
210    Drive
211    Removable medium

**Claims**

1.  An information processing apparatus, comprising:

    an exercise type estimation unit configured to estimate an exercise type executed by a user on a basis of sensor detection information;
    a database configured to store user-specific exercise intensity corresponding to the exercise type;
    an exercise type/personalized exercise intensity acquisition unit configured to acquire, from the database, an exercise type/personalized exercise intensity that is the user-specific exercise intensity corresponding to the exercise type estimated by the exercise type estimation unit; and
    an exercise implementation status analysis unit configured to analyze exercise implementation status data generated on a basis of the exercise type/personalized exercise intensity acquired from the database, and generate an exercise implementation status analysis result indicating whether or not exercise being executed by the user is an exercise according to a pre-generated exercise prescription.

2.  The information processing apparatus according to claim 1, wherein

    the database is an exercise type corresponding user-specific exercise intensity database that stores user corresponding exercise intensity corresponding to an exercise type, and
    the exercise implementation status analysis unit analyzes exercise implementation status data generated on a basis of the exercise type/personalized exercise intensity acquired from the exercise type corresponding user-specific exercise intensity database, and generates the exercise implementation status analysis result indicating whether or not the exercise being executed by the user is the exercise according to the pre-generated exercise prescription.

3.  The information processing apparatus according to claim 2, wherein
    the exercise type corresponding user-specific exercise intensity database is a database that stores exercise intensity obtained by analyzing exhalation analysis result data obtained by allowing the user to execute exercise while wearing an exhaled gas analyzer.

4.  The information processing apparatus according to claim 1, wherein

    the user is a user executing an exercise as rehabilitation according to the pre-generated exercise prescription, and
    the exercise implementation status analysis unit compares the exercise implementation status data generated on a basis of the exercise type/personalized exercise intensity with data stored in an exercise prescription information database storing the pre-generated exercise prescription, and generates the exercise implementation status analysis result indicating whether or not the exercise being executed by the user is an exercise according to the exercise prescription.

5.  The information processing apparatus according to claim 4, wherein

    the information processing apparatus includes an exercise implementation status record database that stores the exercise implementation status data, and
    the exercise implementation status analysis unit acquires the exercise implementation status data of the user from the exercise implementation status record database, and generates the exercise implementation status analysis result indicating whether or not the exercise being executed by the user is the exercise according to an exercise prescription.

6. The information processing apparatus according to claim 1, further comprising:

a maximum value corresponding oxygen intake rate calculation unit configured to input the exercise type/personalized exercise intensity acquired by the exercise type/personalized exercise intensity acquisition unit, and calculate a maximum value corresponding oxygen intake rate according to the exercise type executed by the user; and

an exercise time integration unit configured to generate, as exercise implementation status data, an exercise time integration result of an exercise corresponding to each of the maximum value corresponding oxygen intake rates calculated by the maximum value corresponding oxygen intake rate calculation unit,

wherein the exercise implementation status analysis unit generates the exercise implementation status analysis result indicating whether or not the exercise being executed by the user is the exercise according to the exercise prescription is generated on a basis of the exercise implementation status data generated by the exercise time integration unit.

7. The information processing apparatus according to claim 1, wherein the exercise intensity is metabolic equivalents (METs).

8. The information processing apparatus according to claim 7, wherein the database is a database that stores user-specific METs as exercise intensity corresponding to the user-specific exercise type.

9. The information processing apparatus according to claim 8, wherein the user-specific METs that are an exercise intensity corresponding to the user-specific exercise type are METs that are calculated in consideration of the exhalation analysis result data obtained by allowing the user to execute the exercise while wearing the exhaled gas analyzer and a weight of the user.

10. The information processing apparatus according to claim 1, wherein the exercise implementation status analysis unit outputs the exercise implementation status analysis result indicating whether or not the exercise being executed by the user is the exercise according to the pre-generated exercise prescription to a display unit.

11. An information processing apparatus, comprising:

an exercise type estimation unit configured to estimate an exercise type executed by a user on a basis of sensor detection information;

a steady state activity amount analysis unit configured to input an estimated exercise type estimated by the exercise type estimation unit and an exhaled gas analysis result of the user to calculate an exhalation analysis result-based exercise type/personalized activity amount;

an exercise intensity-based exercise type/personalized activity amount calculation unit configured to acquire an exercise type corresponding standard exercise intensity estimated by the exercise type estimation unit from an exercise type corresponding standard exercise intensity database storing the exercise type corresponding standard exercise intensity, and calculate an exercise intensity-based exercise type/personalized activity amount on a basis of the acquired standard exercise intensity and a weight of the user; and

an exercise type/personalized exercise intensity calculation unit configured to calculate an exercise type/personalized exercise intensity that is the user corresponding exercise intensity corresponding to the exercise type on a basis of each data of (a) an exhalation analysis result-based exercise type/personalized activity amount and (b) the exercise intensity-based exercise type/personalized activity amount.

12. The information processing apparatus according to claim 11, further comprising:

a personalized exercise intensity correction factor calculation unit configured to calculate a personalized exercise intensity correction factor corresponding to the user by inputting each data of (a) the exhalation analysis result-based exercise type/personalized activity amount and (b) the exercise intensity-based exercise type/personalized activity amount,

wherein the exercise type/personalized exercise intensity calculation unit calculates the exercise type/personalized exercise intensity that is the user corresponding exercise intensity by using the personalized exercise intensity correction factor.

13. The information processing apparatus according to claim 12, wherein
the exercise type/personalized exercise intensity calculation unit calculates the exercise type/personalized exercise intensity by using information on the weight of the user.

14. The information processing apparatus according to claim 11, wherein
the exercise type/personalized exercise intensity calculation unit records the calculated exercise type/personalized exercise intensity in an exercise type corresponding user-specific exercise intensity database.

15. An information processing system, comprising:

a user terminal; and a hospital terminal,
wherein the user terminal includes:

an exercise type estimation unit configured to estimate an exercise type executed by a user on a basis of sensor detection information;
a database configured to store user-specific exercise intensity corresponding to the exercise type;
an exercise type/personalized exercise intensity acquisition unit configured to acquire, from the database, an exercise type/personalized exercise intensity that is the user-specific exercise intensity corresponding to the exercise type estimated by the exercise type estimation unit; and
a communication unit configured to transmit exercise implementation status data generated on a basis of the exercise type/personalized exercise intensity acquired from the database to the hospital terminal,
the hospital terminal includes:

an exercise implementation status analysis unit configured to analyze the exercise implementation status data received from the user terminal and generate an exercise implementation status analysis result indicating whether or not the exercise being executed by the user is the exercise according to the pre-generated exercise prescription; and
a communication unit configured to transmit the exercise implementation status analysis result generated by the exercise implementation status analysis unit to the user terminal, and
the user terminal displays the exercise implementation status analysis result received from the hospital terminal on a display unit.

16. An information processing system, comprising:

a user terminal; and a geographic information providing server,
wherein the user terminal includes:

an exercise type estimation unit configured to estimate an exercise type executed by a user on a basis of sensor detection information;
a database configured to store user-specific exercise intensity corresponding to the exercise type;
an exercise type/personalized exercise intensity acquisition unit configured to acquire, from the database, an exercise type/personalized exercise intensity that is the user-specific exercise intensity corresponding to the exercise type estimated by the exercise type estimation unit;
a maximum value corresponding oxygen intake rate calculation unit configured to input the exercise type/personalized exercise intensity acquired by the exercise type/personalized exercise intensity acquisition unit, and calculate a maximum value corresponding oxygen intake rate according to the exercise type executed by the user; and
a communication unit configured to generate exercise prescription information for realizing the maximum value corresponding oxygen intake rate on a basis of the maximum value corresponding oxygen intake rate calculated by the maximum value corresponding oxygen intake rate calculation unit and transmit the generated exercise prescription information to the geographic information providing server,
the geographic information providing server includes:

an exercise prescription corresponding action route information generation unit configured to analyze the exercise prescription information received from the user terminal and generate the exercise prescription corresponding action route information for realizing the exercise prescription information; and a communication unit configured to transmit the exercise prescription corresponding action route information to the user terminal, and

the user terminal displays the exercise prescription corresponding action route information received from the geographic information providing server on a display unit.

17. An information processing method executed by an information processing apparatus, the method comprising:

an exercise type estimation step of estimating, by an exercise type estimation unit, an exercise type executed by a user on a basis of sensor detection information;

an exercise type/personalized exercise intensity acquisition step of acquiring, by an exercise type/personalized exercise intensity acquisition unit, from the database, an exercise type/personalized exercise intensity that is the user-specific exercise intensity corresponding to the exercise type estimated by the exercise type estimation unit; and

an exercise implementation status analysis step of analyzing, by an exercise implementation status analysis unit, exercise implementation status data generated on a basis of the exercise type/personalized exercise intensity acquired from the database, and generating an exercise implementation status analysis result indicating whether or not exercise being executed by the user is an exercise according to a pre-generated exercise prescription.

18. An information processing method executed by an information processing apparatus, the method comprising:

an exercise type estimation step of estimating, by an exercise type estimation unit, an exercise type executed by a user on a basis of sensor detection information;

a steady state activity amount analysis step of inputting, by a steady state activity amount analysis unit, an estimated exercise type estimated by the exercise type estimation unit and an exhaled gas analysis result of the user to calculate an exhalation analysis result-based exercise type/personalized activity amount;

an exercise intensity-based exercise type/personalized activity amount calculation step of acquiring, by an exercise intensity-based exercise type/personalized activity amount calculation unit, an exercise type corresponding standard exercise intensity estimated by the exercise type estimation unit from an exercise type corresponding standard exercise intensity database storing the exercise type corresponding standard exercise intensity, and calculating an exercise intensity-based exercise type/personalized activity amount on a basis of the acquired standard exercise intensity and a weight of the user; and

an exercise type/personalized exercise intensity calculation step of calculating, by an exercise type/personalized exercise intensity calculation unit, an exercise type/personalized exercise intensity that is the user corresponding exercise intensity corresponding to the exercise type on a basis of each data of (a) an exhalation analysis result-based exercise type/personalized activity amount and (b) the exercise intensity-based exercise type/personalized activity amount.

19. A program for allowing an information processing apparatus to execute information processing, the program executing:

an exercise type estimation step of estimating, by an exercise type estimation unit, an exercise type executed by a user on a basis of sensor detection information;

an exercise type/personalized exercise intensity acquisition step of acquiring, by an exercise type/personalized exercise intensity acquisition unit, from the database, an exercise type/personalized exercise intensity that is the user-specific exercise intensity corresponding to the exercise type estimated by the exercise type estimation unit; and

an exercise implementation status analysis step of analyzing, by an exercise implementation status analysis unit, exercise implementation status data generated on a basis of the exercise type/personalized exercise intensity acquired from the database, and generating an exercise implementation status analysis result indicating whether or not exercise being executed by the user is an exercise according to a pre-generated exercise prescription.

20. A program for allowing an information processing apparatus to execute information processing, the program executing:

an exercise type estimation step of estimating, by an exercise type estimation unit, an exercise type executed by a user on a basis of sensor detection information;

a steady state activity amount analysis step of inputting, by a steady state activity amount analysis unit, an estimated exercise type estimated by the exercise type estimation unit and an exhaled gas analysis result of the user to calculate an exhalation analysis result-based exercise type/personalized activity amount;

an exercise intensity-based exercise type/personalized activity amount calculation step of acquiring, by an exercise intensity-based exercise type/personalized activity amount calculation unit, an exercise type corresponding standard exercise intensity estimated by the exercise type estimation unit from an exercise type corresponding standard exercise intensity database storing the exercise type corresponding standard exercise intensity, and calculating an exercise intensity-based exercise type/personalized activity amount on a basis of the acquired standard exercise intensity and a weight of the user; and

an exercise type/personalized exercise intensity calculation step of calculating, by an exercise type/personalized exercise intensity calculation unit, an exercise type/personalized exercise intensity that is the user corresponding exercise intensity corresponding to the exercise type on a basis of each data of (a) an exhalation analysis result-based exercise type/personalized activity amount and (b) the exercise intensity-based exercise type/personalized activity amount.

## FIG. 1

```
ADMISSION ─ SURGERY ──────────────────────── DISCHARGE ──────────────►
```

**S01**
REHABILITATION IN HOSPITAL ROOM AND WARD (WALKING PRACTICE USING HOSPITAL ROOM, CORRIDOR AND THE LIKE)

**S02**
REHABILITATION USING REHABILITATION CENTER IN HOSPITAL

**S03a**
OUTPATIENT REHABILITATION (OUTPATIENT USE OF IN-HOSPITAL REHABILITATION CENTER)

+

**S03b**
HOME REHABILITATION (EXERCISE WITH OWN WILL ACCORDING TO EXERCISE PRESCRIPTION)

**S04**
MAINTENANCE-PERIOD HOME REHABILITATION (EXERCISE WITH OWN WILL ACCORDING TO EXERCISE PRESCRIPTION)

EP 4 129 261 A1

*FIG. 2*

S01
REHABILITATION IN HOSPITAL ROOM AND WARD (WALKING PRACTICE USING HOSPITAL ROOM, CORRIDOR AND THE LIKE)

S02
REHABILITATION USING REHABILITATION CENTER IN HOSPITAL

ADMISSION

OPERATION

DISCHARGE

30 USER TERMINAL

40
EXERCISE TYPE CORRESPONDING USER-SPECIFIC EXERCISE INTENSITY DATABASE

11 USER
(REHABILITATION PRACTITIONER (PATIENT))

12 SENSOR

12 SENSOR

(RUNNING)

(FIXED BIKE)

20 BREATH GAS ANALYZER

# FIG. 3

```
                         S03b                              S04
            ┌──────────┐  ┌──────────────────────┐  ┌──────────────────────┐
            │          │  │  HOME REHABILITATION  │  │ MAINTENANCE-PERIOD    │
            │ DISCHARGE├──┤ (EXERCISE WITH OWN WILL├──┤ HOME REHABILITATION   ├──▶
            │          │  │     ACCORDING TO      │  │ (EXERCISE WITH OWN WILL│
            └──────────┘  │ EXERCISE PRESCRIPTION)│  │     ACCORDING TO      │
                          └──────────────────────┘  │ EXERCISE PRESCRIPTION) │
                                                     └──────────────────────┘
```

**EXERCISE TYPE CORRESPONDING USER-SPECIFIC EXERCISE INTENSITY DATABASE** — 40

30 USER TERMINAL

**REHABILITATION ANALYSIS RESULT DATA**

11 USER

12 SENSOR

12 SENSOR

12 SENSOR

(RUNNING)

(FIXED BIKE)

EXERCISE IMPLEMENTATION TARGET TIME HAS NOT BEEN ACHIEVED YET THIS WEEK. THERE IS STILL IN TIME.

CHECK INSUFFICIENT EXERCISE CONTENT

2 DAYS LEFT
INSUFFICIENT EXERCISE CONTENTS

LOW INTENSITY — 30 MINUTES
EXAMPLE: SLOW WALKING

MEDIUM INTENSITY — 10 MINUTES
EXAMPLE: QUICK WALKING

HIGH INTENSITY — 5 MINUTES
EXAMPLE: UP STAIRS

EP 4 129 261 A1

*FIG. 4*

S03b
HOME REHABILITATION
(EXERCISE WITH OWN WILL
ACCORDING TO
EXERCISE PRESCRIPTION)

S04
MAINTENANCE-PERIOD
HOME REHABILITATION
(EXERCISE WITH OWN WILL
ACCORDING TO
EXERCISE PRESCRIPTION)

DISCHARGE

40
EXERCISE TYPE
CORRESPONDING
USER-SPECIFIC EXERCISE
INTENSITY DATABASE

30 USER TERMINAL

11 USER

11 USER

REHABILITATION
ANALYSIS
RESULT DATA

ADVICE

70 HOSPITAL TERMINAL

(RUNNING)

(FIXED BIKE)

EP 4 129 261 A1

## FIG. 5

(a) OXYGEN INTAKE AMOUNT-RELATED DATA

|  | TERM | MARK | CONTENTS |
|---|---|---|---|
| (a1) | OXYGEN INTAKE | VO2 | AMOUNT OF OXYGEN BODY INTAKES. UNIT IS [ml/min/kg] |
| (a2) | MAXIMUM OXYGEN INTAKE | peakVO2 | MAXIMUM VALUE OF AMOUNT OF OXYGEN (VO2) BODY INTAKES |
| (a3) | MAXIMUM VALUE CORRESPONDING OXYGEN INTAKE RATE | %peakVO2 | RATIO OF ACTUAL OXYGEN INTAKE (VO2) TO MAXIMUM OXYGEN INTAKE (peakVO2) (IN ADDITION, IN PROCESS OF PRESENT DISCLOSURE, THERE IS CASE IN WHICH ESTIMATED VALUE IS CALCULATED AND USED) |

EP 4 129 261 A1

# FIG. 6

(b) EXERCISE INTENSITY-RELATED DATA

| | TERM | MARK | CONTENTS |
|---|---|---|---|
| (b1) | STANDARD EXERCISE INTENSITY | METs | AT REST METs=1, EXERCISE INTENSITY STANDARD VALUE (PUBLIC DATA) INDICATING HOW MANY TIMES EACH EXERCISE SUCH AS WALKING AND RUNNING CONSUMES CALORIES (=ACTIVITY AMOUNT) COMPARED TO REST. (Metabolic equivalents) IN ADDITION, CONSUMED CALORIE [kcal] (=ACTIVITY AMOUNT) CAN BE CALCULATED FROM THE FOLLOWING FORMULA. CONSUMED CALORIE [kcal] = METs * WEIGHT [kg] * EXERCISE TIME [hr] * 1.05 |
| (b2) | STANDARD EXERCISE INTENSITY CORRESPONDING TO EXERCISE TYPE (STANDARD EXERCISE INTENSITY CORRESPONDING TO ESTIMATED EXERCISE TYPE) | METs, K (METs, Kest) | EXERCISE INTENSITY STANDARD VALUE (PUBLIC DATA) CORRESPONDING TO EACH TYPE OF EXERCISE (K), SUCH AS WALKING AND RUNNING |
| (b3) | PERSONALIZED EXERCISE INTENSITY | METs, U | VALUE INDICATING EXERCISE INTENSITY CORRESPONDING TO USER INDIVIDUAL (U) CALCULATED BY DIVIDING [ACTIVITY AMOUNT ($EE_{ref}$) CALCULATED USING EXPIRATORY ANALYSIS RESULT] OBTAINED FROM ANALYSIS RESULT OF EXHALED GAS ANALYSIS DEVICE WORN ON USER PERFORMING EXERCISE BY USER'S WEIGHT [kg] AND EXERCISE TIME [hr]. UNIT IS [kcal/kg/hr]. METs, $U = (EE_{ref}) / ((\text{WEIGHT [kg]}) \times (\text{EXERCISE TIME [hr]}))$ |
| (b4) | EXERCISE TYPE / PERSONALIZED EXERCISE INTENSITY (ESTIMATED EXERCISE TYPE / PERSONALIZED EXERCISE INTENSITY) | METs, U, K (METs, U, Kest) | PERSONALIZED EXERCISE INTENSITY ACCORDING TO EXERCISE TYPE (K) |

EP 4 129 261 A1

## FIG. 7

(c) ACTIVITY AMOUNT-RELATED DATA

| | TERM | MARK | CONTENTS |
|---|---|---|---|
| (c1) | ACTIVITY AMOUNT (CONSUMED CALORIE) | EE | CONSUMED CALORIE BY ACTIVITY (EXERCISE). HOWEVER, CALORIE CONSUMED BY BASAL METABOLISM IS ALSO INCLUDED. UNIT IS [kcal]. EE(energy expenditure). |
| (c2) | EXERCISE INTENSITY (METs)-BASED PERSONALIZED ACTIVITY AMOUNT | $EE_{mets,U}$ | PERSONALIZED ACTIVITY AMOUNT (CONSUMED CALORIE) CALCULATED BASED ON STANDARD EXERCISE INTENSITY (METs), WEIGHT OF USER (U), AND EXERCISE TIME.<br><br>$EE_{mets,U}[kcal] = METs * WEIGHT\,[kg] * EXERCISE\,TIME\,[hr] * 1.05$ |
| (c3) | EXERCISE INTENSITY (METs)-BASED EXERCISE TYPE/ PERSONALIZED ACTIVITY AMOUNT | $EE_{mets,U,K}$ | EXERCISE INTENSITY (METs)-BASED PERSONALIZED ACTIVITY AMOUNT CORRESPONDING TO EXERCISE TYPE (K) |
| (c4) | EXHALATION ANALYSIS RESULT-BASED PERSONALIZED ACTIVITY AMOUNT | $EE_{ref,U}$ | ACTIVITY AMOUNT ESTIMATION VALUE CALCULATED USING ANALYSIS RESULT OF EXHALED GAS ANALYSIS DEVICE. ONE STANDARD (Gold Standard) OF ACTIVITY AMOUNT MEASUREMENT. BY EXERCISE INTENSITY (METs)-BASED PERSONALIZED ACTIVITY AMOUNT ($EE_{METs}$), ESTIMATED ACCURACY IS HIGH<br><br>$EE_{ref,\,U}[kcal] = (3.94\,VO_2 + 1.11\,VCO_2) * EXERCISE\,TIME\,[min]$ (Weir's Formula) |
| (c5) | EXHALATION ANALYSIS RESULT-BASED EXERCISE TYPE/ PERSONALIZED ACTIVITY AMOUNT | $EE_{ref,U,K}$ | DURING EXERCISE IMPLEMENTATION OF EXERCISE TYPE (K), WHEN OXYGEN INTAKE (VO2) MEASUREMENT VALUE OF EXHALED GAS ANALYSIS DEVICE REACHES STEADY STATE, EXHALATION ANALYSIS RESULT USING PERSONALIZED ACTIVITY AMOUNT CALCULATED BASED ON EXHALED GAS ANALYSIS RESULT ($VO_2$ AND $VCO_2$) |

# FIG. 8

(d) OTHER DATA

| | TERM | MARK | CONTENTS |
|---|---|---|---|
| (d1) | ESTIMATED EXERCISE TYPE | Kest | TYPE OF EXERCISE THAT USER IS EXECUTING ESTIMATED BASED ON DETECTION INFORMATION OF SENSOR WORN ON USER'S BODY |
| (d2) | PERSONALIZED EXERCISE INTENSITY CORRECTION FACTOR | Rk | RATIO OF [EXHALATION ANALYSIS RESULT-BASED EXERCISE TYPE/PERSONALIZED ACTIVITY AMOUNT (EEref, U, K)] TO [EXERCISE INTENSITY (METs)-BASED EXERCISE TYPE CORRESPONDING ACTIVITY AMOUNT (EEmets, K)] $Rk = (EEref, U, K) / (EEmets, K)$ |

EP 4 129 261 A1

*FIG. 9*

EXHALED GAS ANALYZER 20

21 ACTIVITY ACQUISITION UNIT

d21 EXHALATION ANALYSIS-BASED PERSONALIZED ACTIVITY AMOUNT (EEref, U)

USER TERMINAL 30

31 SENSOR DETECTION VALUE INPUT UNIT

33 STEADY STATE ACTIVITY AMOUNT ANALYSIS UNIT

d33 EXHALATION ANALYSIS RESULT-BASED EXERCISE TYPE/PERSONALIZED ACTIVITY AMOUNT (EEref, U, Kest)

38 PERSONALIZED EXERCISE INTENSITY CORRECTION FACTOR CALCULATION UNIT

d31 SENSOR DETECTION INFORMATION (ACCELERATION, GYRO, GEOMAGNETISM, BAROMETRIC PRESSURE, AND THE LIKE)

32 EXERCISE TYPE ESTIMATION UNIT

d32 ESTIMATED EXERCISE TYPE (Kest)

d37 EXERCISE INTENSITY-BASED EXERCISE TYPE/PERSONALIZED ACTIVITY AMOUNT (EEmets, U, Kest)

d38 PERSONALIZED EXERCISE INTENSITY CORRECTION FACTOR (Rk)

34 EXERCISE TYPE CORRESPONDING STANDARD EXERCISE INTENSITY (METs, K) DATABASE

d34 EXERCISE TYPE CORRESPONDING STANDARD EXERCISE INTENSITY (METs, K)

36 EXERCISE TYPE/ PERSONALIZED EXERCISE INTENSITY ACQUISITION UNIT

d36 ESTIMATED EXERCISE TYPE CORRESPONDING STANDARD EXERCISE INTENSITY (METs, Kest)

37 EXERCISE INTENSITY-BASED EXERCISE TYPE/ PERSONALIZED ACTIVITY AMOUNT CALCULATION UNIT

39 EXERCISE TYPE/ PERSONALIZED EXERCISE INTENSITY CALCULATION UNIT

35 PERSONAL INFORMATION DATABASE

d35 WEIGHT INFORMATION

40 EXERCISE TYPE CORRESPONDING USER-SPECIFIC EXERCISE INTENSITY DATABASE

d39 EXERCISE TYPE/ PERSONALIZED EXERCISE INTENSITY (METs, U, Kest)

EP 4 129 261 A1

## FIG. 10

EP 4 129 261 A1

**34** EXERCISE TYPE CORRESPONDING STANDARD EXERCISE INTENSITY DATABASE

EXERCISE TYPE CORRESPONDING
STANDARD EXERCISE INTENSITY (METs, K)
DATABASE

| (a) EXERCISE TYPE (K) | | (b) EXERCISE TYPE CORRESPONDING STANDARD EXERCISE INTENSITY (METs, K) |
|---|---|---|
| (LARGE ITEM) | (INDIVIDUAL ACTIVITY) | |
| (k1) FIXED BIKE | LOAD AMOUNT = 120W | 8.5 |
| (k2) WALKING | WALKING SPEED = 4.0km/h, PEDESTRIAN ROAD = FLAT AND HARD GROUND | 3 |
| (k3) WALKING | WALKING SPEED = 3.2km/h, PEDESTRIAN ROAD = FLAT AND HARD GROUND | 2.8 |
| (k4) STAIR CLIMB | STAIR STEP HEIGHT DIFFERENCE = 40cm/s | 5.22 |
| ... | ... | ... | ... |

**35** PERSONAL INFORMATION DATABASE

PERSONAL INFORMATION DATABASE

| ITEM | VALUE |
|---|---|
| WEIGHT | 65.2kg |

# FIG. 11

EXERCISE TYPE CORRESPONDING USER-SPECIFIC EXERCISE INTENSITY DATABASE

40

## EXERCISE TYPE CORRESPONDING USER-SPECIFIC EXERCISE INTENSITY (METs, U, Kest) DATABASE

| (a) EXERCISE TYPE (K(Kest)) | | | (b) EXERCISE TYPE/ PERSONALIZED EXERCISE INTENSITY (METs, U, Kest) |
|---|---|---|---|
| | (LARGE ITEM) | (INDIVIDUAL ACTIVITY) | |
| (k1) | FIXED BIKE | LOAD AMOUNT = 120W | 9 |
| (k2) | WALKING | WALKING SPEED = 4.0km/h, PEDESTRIAN ROAD = FLAT AND HARD GROUND | 3.6 |
| (k3) | STAIR CLIMB | STAIR STEP HEIGHT DIFFERENCE = 40cm/s | 5.2 |
| ... | ... | ... | ... |

## FIG. 12

**USER TERMINAL** 30

31 **SENSOR DETECTION VALUE INPUT UNIT**

d31 **SENSOR DETECTION INFORMATION (ACCELERATION, GYRO, GEOMAGNETISM, BAROMETRIC PRESSURE, AND THE LIKE)**

32 **EXERCISE TYPE ESTIMATION UNIT**

d32 **ESTIMATED EXERCISE TYPE (Kest)**

51 **EXERCISE TYPE/ PERSONALIZED EXERCISE INTENSITY ACQUISITION UNIT**

d51 **EXERCISE TYPE PERSONALIZED EXERCISE INTENSITY (METs,U,Kest)**

40 **EXERCISE TYPE CORRESPONDING USER-SPECIFIC EXERCISE INTENSITY DATABASE**

52 **MAXIMUM VALUE CORRESPONDING OXYGEN INTAKE RATE CALCULATION UNIT**

54 **EXERCISE IMPLEMENTATION STATUS RECORD DATABASE**

d53 **EXERCISE IMPLEMENTATION STATUS DATA**

53 **EXERCISE TIME INTEGRATION UNIT**

d52 **MAXIMUM VALUE CORRESPONDING OXYGEN INTAKE RATE (%peakVO2)**

55 **EXERCISE PRESCRIPTION INFORMATION DATABASE**

d54 **EXERCISE IMPLEMENTATION STATUS DATA**

56 **EXERCISE IMPLEMENTATION STATUS ANALYSIS UNIT**

d56 **EXERCISE IMPLEMENTATION STATUS ANALYSIS RESULT**

57 **DISPLAY UNIT**

61

## FIG. 13

EXERCISE IMPLEMENTATION STATUS RECORD DATABASE 54

EXERCISE IMPLEMENTATION STATUS RECORD DATABASE

| WEEK | EXERCISE IMPLEMENTATION TIME OF %peakVO2=40-49% (min) | EXERCISE IMPLEMENTATION TIME OF %peakVO2=50-59% (min) | ... | EXERCISE IMPLEMENTATION TIME OF %peakVO2=80-89% (min) | WALKING (4.0 km/h), FLAT AND HARD GROUND IMPLEMENTATION TIME (min) | ... | STAIR CLIMB (40 cm/sec) IMPLEMENTATION TIME (min) |
|---|---|---|---|---|---|---|---|
| 1 | 180 | 30 | ... | 0 | 100 | ... | 0 |
| 2 | 120 | 30 | ... | 0 | 90 | ... | 0 |
| 3 | 260 | 30 | ... | 5 | 200 | ... | 5 |
| 4 | 180 | 25 | ... | 0 | 100 | ... | 0 |
| ... | ... | ... | ... | ... | ... | ... | ... |

## FIG. 14

**55** EXERCISE PRESCRIPTION INFORMATION DATABASE

EXERCISE PRESCRIPTION INFORMATION DATABASE

| WEEK | EXERCISE TARGET TIME OF %peakVO2=40-49% (min) | EXERCISE TARGET TIME OF %peakVO2=50-59% (min) | ... | EXERCISE TARGET TIME OF %peakVO2=80-89% (min) |
|---|---|---|---|---|
| 1 | 180 | 30 | ... | 0 |
| 2 | 120 | 30 | ... | 0 |
| 3 | 260 | 30 | ... | 5 |
| 4 | 180 | 25 | ... | 0 |
| ... | ... | ... | ... | ... |

EP 4 129 261 A1

FIG. 15

(1)

ALL EXERCISE IMPLEMENTATION TARGET TIME HAVE BEEN ACHIEVED THIS WEEK.

LET'S DO OUR BEST NEXT WEEK AS WELL

(2a)

EXERCISE IMPLEMENTATION TARGET TIME HAS NOT YET ACHIEVED THIS WEEK. THERE IS STILL IN TIME.

CONFIRM INSUFFICIENT EXERCISE CONTENTS

(2b)

2 DAYS LEFT

INSUFFICIENT EXERCISE CONTENTS

LOW INTENSITY | 30 MINUTES
EXAMPLE: SLOW WALKING

MEDIUM INTENSITY | 10 MINUTES
EXAMPLE: QUICK WALKING

HIGH INTENSITY | 5 MINUTES
EXAMPLE: CLIMBING STAIRS

## FIG. 16

```
                    START

                                              S121
        INPUT SENSOR DETECTION
           INFORMATION AND
       ESTIMATE EXERCISE TYPE (Kest)

                                              S122
       GENERATE EXHALATION ANALYSIS RESULT
        BASE EXERCISE TYPE / PERSONALIZED
       ACTIVITY AMOUNT (EEref, U, Kest) BASED
         ON ESTIMATED EXERCISE TYPE (Kest)
         AND EXHALED GAS ANALYSIS RESULT

                                              S123
      SELECT ENTRY OF EXERCISE TYPE THAT MATCHES
       OR IS MOST SIMILAR TO ESTIMATED EXERCISE
      TYPE (Kest) FROM EXERCISE TYPE CORRESPONDING
      STANDARD EXERCISE INTENSITY (METs, K) DATABASE,
      AND DETERMINE EXERCISE TYPE CORRESPONDING
        STANDARD EXERCISE INTENSITY (METs, K) OF
       SELECTED ENTRY AS ESTIMATED EXERCISE TYPE
      CORRESPONDING STANDARD EXERCISE INTENSITY
                     (METs, Kest)

                                              S124
       CALCULATE EXERCISE INTENSITY BASE EXERCISE
          TYPE / PERSONALIZED ACTIVITY AMOUNT
       (EEmets, U, Kest) BASED ON ESTIMATED EXERCISE
        TYPE CORRESPONDING STANDARD EXERCISE
         INTENSITY (METs, Kest) AND USER WEIGHT
```

```
                                                          S125
       CALCULATE PERSONALIZED EXERCISE INTENSITY
       CORRECTION FACTOR (Rk) BASED ON EXHALATION
       ANALYSIS RESULT-BASED EXERCISE TYPE /
       PERSONALIZED ACTIVITY AMOUNT (EEref, U, Kest)
        AND EXERCISE INTENSITY BASE EXERCISE /
       PERSONALIZED ACTIVITY AMOUNT (EEmets, U, Kest)

                                                          S126
            CALCULATE EXERCISE TYPE /
         PERSONALIZED EXERCISE INTENSITY
          (METs, U, Kest) BASED ON
         PERSONALIZED EXERCISE INTENSITY
          CORRECTION FACTOR (Rk) AND
           USER WEIGHT INFORMATION

                                                          S127
         STORE CALCULATED EXERCISE TYPE /
         PERSONALIZED EXERCISE INTENSITY
          (METs, U, Kest) IN EXERCISE TYPE /
          PERSONALIZED USER-SPECIFIC
          EXERCISE INTENSITY DATABASE

                         END
```

## FIG. 17

**11 USER**

**20 EXHALED GAS ANALYZER**

**30 USER TERMINAL**

MAXIMUM VALUE OF EXERCISE TYPE/PERSONALIZED EXERCISE INTENSITY (METs, U, K) CORRESPONDS TO EXERCISE INTENSITY AT THE TIME OF MEASUREMENT OF MAXIMUM OXYGEN INTAKE = peakVO2, AND WHEN MAXIMUM IS SEARCHED, IT IS POSSIBLE TO ACQUIRE MAXIMUM EXERCISE INTENSITY (METs) THAT CAN BE PERFORMED BY USER

IN LAMP LOAD TEST, OXYGEN INTAKE WHEN EXHALATION ANALYSIS RESULT BASE EXERCISE TYPE/PERSONALIZED ACTIVITY AMOUNT (EEref,U,K) IS MAXIMUM TO BE MAXIMUM OXYGEN INTAKE = peakVO2.

**STOP**

**LAMP LOAD TEST**
USE FIXED BIKE TO GRADUALLY INCREASE LOAD AT CERTAIN RATE. TERMINATE IF SUBJECT COULD NOT PEDAL

LOAD AMOUNT (W)

100

50

0

0    4    8    12    16    20  TIME (MINUTES)

REST PERIOD

LOW LOAD PERIOD

LOAD INCREASING PERIOD

EP 4 129 261 A1

## FIG. 18

```
        ( START )
            |
            v
```

**S131**

"START LAMP LOAD TEST"
START ACQUISITION PROCESSING AND RECORD PROCESSING
OF EXHALATION ANALYSIS RESULT BASE EXERCISE TYPE /
PERSONALIZED ACTIVITY AMOUNT (EEref,U,K)

```
            |
            v
```

**S132** — no

IS LAMP LOAD TEST
TERMINATED?

yes

**S133**

CALCULATE EXERCISE TYPE / PERSONALIZED EXERCISE
INTENSITY (METs, U, K) ACCORDING TO THE FOLLOWING FORMULA
METs, U, K = (EEref, U, K) / ((WEIGHT [kg]) × (EXERCISE TIME [hr]))

```
            |
            v
```

**S134**

RECORD CALCULATED EXERCISE TYPE / PERSONALIZE EXERCISE
INTENSITY (METs, U, K) IN EXERCISE TYPE USER-SPECIFIC
EXERCISE INTENSITY DATABASE BY BEING ASSOCIATED WITH
EXERCISE TYPE (K)

```
            |
            v
        ( END )
```

## FIG. 19

START

S141
INPUT SENSOR DETECTION INFORMATION AND ESTIMATE EXERCISE TYPE (Kest)

S142
SELECT ENTRY OF EXERCISE TYPE THAT MATCHES OR IS MOST SIMILAR TO ESTIMATED EXERCISE TYPE (Kest) FROM EXERCISE TYPE / PERSONALIZED EXERCISE INTENSITY (METs, U, K) DATABASE, AND ACQUIRE EXERCISE TYPE / PERSONALIZED EXERCISE INTENSITY (METs, U, K) OF SELECTED ENTRY

S143
CALCULATE MAXIMUM VALUE CORRESPONDING OXYGEN INTAKE RATE (%peakVO2) BASED ON ESTIMATED EXERCISE TYPE / PERSONALIZED EXERCISE INTENSITY (METs, U, Kest) AND USER WEIGHT

S144
GENERATE EXERCISE IMPLEMENTATION STATUS DATA BASED ON MAXIMUM VALUE CORRESPONDING OXYGEN INTAKE RATE (%peakVO2) AND EXERCISE TIME OF EACH EXERCISE AND RECORD GENERATED EXERCISE IMPLEMENTATION STATUS DATA IN EXERCISE IMPLEMENTATION STATUS RECORD DATABASE

S145
COMPARE AND ANALYZE EXERCISE IMPLEMENTATION STATUS DATA RECORDED IN EXERCISE IMPLEMENTATION STATUS RECORD DATABASE AND EXERCISE TARGET TIME RECORDED IN EXERCISE PRESCRIPTION INFORMATION DATABASE TO GENERATE EXERCISE IMPLEMENTATION STATUS ANALYSIS RESULT

S146
DISPLAY GENERATED EXERCISE IMPLEMENTATION STATUS ANALYSIS RESULT ON DISPLAY

END

# FIG. 20

**USER TERMINAL** 30

- 31 SENSOR DETECTION VALUE INPUT UNIT
- d31 SENSOR DETECTION INFORMATION (ACCELERATION, GYRO, GEOMAGNETISM, BAROMETRIC PRESSURE, AND THE LIKE)
- 32 EXERCISE TYPE ESTIMATION UNIT
- d32 ESTIMATED EXERCISE TYPE (Kest)
- 51 EXERCISE TYPE / PERSONALIZED EXERCISE INTENSITY ACQUISITION UNIT
- d51 EXERCISE TYPE PERSONALIZED EXERCISE INTENSITY (METs,U,Kest)
- 57 DISPLAY UNIT
- 40 EXERCISE TYPE CORRESPONDING USER-SPECIFIC EXERCISE INTENSITY DATABASE
- 52 MAXIMUM VALUE CORRESPONDING OXYGEN INTAKE RATE CALCULATION UNIT
- 61 COMMUNICATION UNIT
- 54 EXERCISE IMPLEMENTATION STATUS RECORD DATABASE
- d53 EXERCISE IMPLEMENTATION STATUS DATA
- 53 EXERCISE TIME INTEGRATION UNIT
- d52 MAXIMUM VALUE CORRESPONDING OXYGEN INTAKE RATE (%peakVO2)
- d54 EXERCISE IMPLEMENTATION STATUS DATA

**HOSPITAL TERMINAL** 70

- 71 COMMUNICATION UNIT
- 72 EXERCISE IMPLEMENTATION STATUS ANALYSIS UNIT
- 73 EXERCISE PRESCRIPTION INFORMATION DATABASE
- d72 EXERCISE IMPLEMENTATION STATUS ANALYSIS RESULT

EP 4 129 261 A1

## FIG. 21

START

↓

**S151**
INPUT SENSOR DETECTION INFORMATION AND ESTIMATE EXERCISE TYPE (Kest)

↓

**S152**
SELECT ENTRY OF EXERCISE TYPE THAT MATCHES OR IS MOST SIMILAR TO ESTIMATED EXERCISE TYPE (Kest) FROM EXERCISE TYPE/ PERSONALIZED EXERCISE INTENSITY (METs, U, K) DATABASE, AND ACQUIRE EXERCISE TYPE/ PERSONALIZED EXERCISE INTENSITY (METs, U, K) OF SELECTED ENTRY

↓

**S153**
CALCULATE MAXIMUM VALUE CORRESPONDING OXYGEN INTAKE RATE (%peakVO2) BASED ON ESTIMATED EXERCISE TYPE/PERSONALIZED EXERCISE INTENSITY (METs, U, Kest) AND USER WEIGHT

↓

**S154**
GENERATE EXERCISE IMPLEMENTATION STATUS DATA BASED ON MAXIMUM VALUE CORRESPONDING OXYGEN INTAKE RATE (%peakVO2) AND EXERCISE TIME OF EACH EXERCISE AND RECORD GENERATED EXERCISE IMPLEMENTATION STATUS DATA IN EXERCISE IMPLEMENTATION STATUS RECORD DATABASE

↓

**S155**
TRANSMIT EXERCISE IMPLEMENTATION STATUS DATA RECORDED IN EXERCISE IMPLEMENTATION STATUS RECORD DATABASE TO HOSPITAL TERMINAL

↓

**S156**
DISPLAY EXERCISE IMPLEMENTATION STATUS ANALYSIS RESULT RECEIVED FROM HOSPITAL TERMINAL ON DISPLAY UNIT

↓

END

# FIG. 22

```
        ┌─────────────┐
        │    START    │
        └──────┬──────┘
               │                          S161
        ┌──────▼───────────────────────┐
        │ RECEIVE EXERCISE IMPLEMENTATION│
        │    STATUS DATA RECORDED IN     │
        │  EXERCISE IMPLEMENTATION STATUS│
        │      RECORD DATABASE FROM      │
        │         USER TERMINAL          │
        └──────┬─────────────────────────┘
               │                          S162
        ┌──────▼───────────────────────┐
        │   COMPARE AND ANALYZE EXERCISE │
        │ IMPLEMENTATION STATUS DATA RECEIVED│
        │     FROM USER TERMINAL AND      │
        │  EXERCISE TARGET TIME RECORDED IN│
        │ EXERCISE PRESCRIPTION INFORMATION│
        │   DATABASE AND GENERATE EXERCISE │
        │IMPLEMENTATION STATUS ANALYSIS RESULT│
        └──────┬─────────────────────────┘
               │                          S163
        ┌──────▼───────────────────────┐
        │   TRANSMIT GENERATED EXERCISE  │
        │IMPLEMENTATION STATUS ANALYSIS RESULT│
        │         TO USER TERMINAL       │
        └──────┬─────────────────────────┘
               │
        ┌──────▼──────┐
        │     END     │
        └─────────────┘
```

## FIG. 23

SENSOR DETECTION VALUE (HEART RATE, PULSE VALUE) INPUT UNIT — 61

d61a PULSE VALUE

PULSE UPPER LIMIT ASYMPTOTIC DETERMINATION UNIT — 62

d64a PULSE RATE UPPER LIMIT

EXERCISE PRESCRIPTION INFORMATION DATABASE — 64

d64b HEART RATE UPPER LIMIT

d61b HEART RATE

HEART RATE UPPER LIMIT ASYMPTOTIC DETERMINATION UNIT — 63

INSTRUCTION INFORMATION GENERATION UNIT — 65

d65 INSTRUCTION INFORMATION

OUTPUT UNIT (DISPLAY UNIT) — 66

USER TERMINAL — 30

EP 4 129 261 A1

## FIG. 24

64

EXERCISE PRESCRIPTION INFORMATION DATABASE

EXERCISE PRESCRIPTION INFORMATION DATABASE

| WEEK | EXERCISE TARGET TIME OF %peakVO2=40-49% (min) | EXERCISE TARGET TIME OF %peakVO2=50-59% (min) | ... | EXERCISE TARGET TIME OF %peakVO2=80-89% (min) | HEART RATE UPPER LIMIT | PULSE RATE DEFINED RANGE |
|------|------|------|------|------|------|------|
| 1 | 180 | 30 | ... | 0 | 180BPM | 90-160BPM |
| 2 | 120 | 30 | ... | 0 | 180BPM | 90-160BPM |
| 3 | 260 | 30 | ... | 5 | 180BPM | 90-160BPM |
| 4 | 180 | 25 | ... | 0 | 178BPM | 90-150BPM |
| ... | ... | ... | ... | ... | ... | ... |

EP 4 129 261 A1

# FIG. 25

(1)

PULSE RATE EXCEEDS
DEFINED RANGE.
PLEASE MEASURE
ELECTROCARDIOGRAM.

DISPLAY
ELECTROCARDIOGRAM
MEASUREMENT METHOD

(2)

HEART RATE IS TOO HIGH.
PLEASE STOP
REHABILITATION.

STOP

## FIG. 26

START

S181 ACQUIRE PULSE RATE

S182 IS PULSE RATE IN DEFINED RANGE?

Yes → (back to ACQUIRE PULSE RATE)

No →

S183 ENCOURAGE WEARING OF HEART RATE MONITOR AND GENERATE AND OUTPUT INSTRUCTION INFORMATION

S184 ACQUIRE HEART RATE

S185 IS HEART RATE EQUAL TO OR LESS THAN UPPER LIMIT?

Yes → (back to ACQUIRE HEART RATE)

No →

S186 ENCOURAGE CESSATION OF EXERCISE AND GENERATE AND OUTPUT INSTRUCTION INFORMATION

END

FIG. 27

## FIG. 28

USER TERMINAL

81 — SENSOR DETECTION VALUE INPUT UNIT

d81 — SENSOR DETECTION INFORMATION (POSITIONAL INFORMATION, ACCELERATION, GYRO, GEOMAGNETISM, BAROMETRIC PRESSURE, AND THE LIKE)

82 — EXERCISE TYPE ESTIMATION UNIT

d82 — ESTIMATED EXERCISE TYPE (Kest)

83 — EXERCISE TYPE/ PERSONALIZED EXERCISE INTENSITY ACQUISITION UNIT

d83 — EXERCISE TYPE PERSONALIZED EXERCISE INTENSITY (METs,U,Kest)

d81a — SENSOR DETECTION INFORMATION (POSITIONAL INFORMATION)

40 — EXERCISE TYPE CORRESPONDING USER-SPECIFIC EXERCISE INTENSITY DATABASE

84 — MAXIMUM VALUE CORRESPONDING OXYGEN INTAKE RATE CALCULATION UNIT

d84 — MAXIMUM VALUE CORRESPONDING OXYGEN INTAKE RATE (%peakVO2)

87 — COMMUNICATION RATE

d86a — EXERCISE PRESCRIPTION INFORMATION ACHIEVING MAXIMUM VALUE CORRESPONDING OXYGEN INTAKE RATE (%peakVO2)

86 — EXERCISE PRESCRIPTION CORRESPONDING ACTION ROUTE INFORMATION SEARCH UNIT

85 — EXERCISE PRESCRIPTION INFORMATION DATABASE

d81a — SENSOR DETECTION INFORMATION (POSITIONAL INFORMATION)

d92 — EXERCISE PRESCRIPTION CORRESPONDING ACTION ROUTE INFORMATION

d86b — DISPLAY DATA

88 — DISPLAY UNIT

d86a — EXERCISE PRESCRIPTION INFORMATION ACHIEVING MAXIMUM VALUE CORRESPONDING OXYGEN INTAKE RATE (%peakVO2)

GEOGRAPHIC INFORMATION PROVIDING SERVER

90

91 — COMMUNICATION UNIT

d92 — EXERCISE PRESCRIPTION CORRESPONDING ACTION ROUTE INFORMATION

92 — EXERCISE PRESCRIPTION CORRESPONDING ACTION ROUTE INFORMATION GENERATION UNIT

93 — GEOGRAPHIC INFORMATION DATABASE

EP 4 129 261 A1

## FIG. 29

| ITEM ANALYSIS | ADDITIONAL INFORMATION | LATITUDE | LONGITUDE |
|---|---|---|---|
| FLAT ROAD | | 35.582541 | 139.884121 |
| ROAD WITH GRADIENT | GRADIENT MEAN 4% | 35.573081 | 139.899085 |
| STAIR | | 35.574625 | 139.891827 |
| BUS ROUTE | WEEKDAY 10:00, 11:00, … HOLIDAY 12:00, … | 35.802812 | 139.889837 |
| … | … | … | … |

## FIG. 30

USER TERMINAL                                    30

31          d31                    32          d32                    51          d51

SENSOR DETECTION VALUE INPUT UNIT → SENSOR DETECTION INFORMATION (ACCELERATION, GYRO, GEOMAGNETISM, BAROMETRIC PRESSURE, AND THE LIKE) → EXERCISE TYPE ESTIMATION UNIT → ESTIMATED EXERCISE TYPE (Kest) → EXERCISE TYPE/PERSONALIZED EXERCISE INTENSITY ACQUISITION UNIT → EXERCISE TYPE PERSONALIZED EXERCISE INTENSITY (METs,U,Kest)

40

57          d56

DISPLAY UNIT ← EXERCISE IMPLEMENTATION STATUS ANALYSIS RESULT

EXERCISE TYPE CORRESPONDING USER-SPECIFIC EXERCISE INTENSITY DATABASE

52

MAXIMUM VALUE CORRESPONDING OXYGEN INTAKE RATE CALCULATION UNIT

56

EXERCISE IMPLEMENTATION STATUS ANALYSIS UNIT

61                     d53          53                    d52

COMMUNICATION UNIT

d77

UPDATED EXERCISE PRESCRIPTION

UPDATED EXERCISE PRESCRIPTION          d77

EXERCISE IMPLEMENTATION STATUS DATA ← EXERCISE TIME INTEGRATION UNIT ← MAXIMUM VALUE CORRESPONDING OXYGEN INTAKE RATE (%peakVO2)

d53

EXERCISE IMPLEMENTATION STATUS DATA

HOSPITAL TERMINAL                    70

75                              d76          76

EXERCISE IMPLEMENTATION STATUS RECORD DATABASE          77

NON-UPDATED EXERCISE PRESCRIPTION

EXERCISE PRESCRIPTION INFORMATION DATABASE

71                              EXERCISE PRESCRIPTION UPDATING UNIT

COMMUNICATION UNIT

d77          78                              d77

UPDATED EXERCISE PRESCRIPTION          EXERCISE PRESCRIPTION OPERATION UI UNIT          UPDATED EXERCISE PRESCRIPTION

EP 4 129 261 A1

79

## FIG. 31

EXERCISE IMPLEMENTATION STATUS RECORD DATABASE

**USER ID=0001**

| WEEK | EXERCISE IMPLEMENTATION TIME OF %peakVO2=40-49% (min) | EXERCISE IMPLEMENTATION TIME OF %peakVO2=50-59% (min) | ··· | EXERCISE IMPLEMENTATION TIME OF %peakVO2=80-89% (min) | WALKING (4.0km/h), FLAT AND HARD GROUND IMPLEMENTATION TIME (min) | ··· | STAIR CLIMB (40cm/sec) IMPLEMENTATION TIME (min) |
|---|---|---|---|---|---|---|---|
| 1 | 180 | 30 | ··· | 0 | 100 | ··· | 0 |
| 2 | 120 | 30 | ··· | 0 | 90 | ··· | 0 |
| 3 | 260 | 30 | ··· | 5 | 200 | ··· | 5 |
| 4 | 180 | 25 | ··· | 10 | 100 | ··· | 10 |
| ··· | ··· | ··· | ··· | ··· | ··· | ··· | ··· |

**USER ID=0002**

| WEEK | EXERCISE IMPLEMENTATION TIME OF %peakVO2=40-49% (min) | EXERCISE IMPLEMENTATION TIME OF %peakVO2=50-59% (min) | ··· | EXERCISE IMPLEMENTATION TIME OF %peakVO2=80-89% (min) | WALKING (4.0km/h), FLAT AND HARD GROUND IMPLEMENTATION TIME (min) | ··· | STAIR CLIMB (40cm/sec) IMPLEMENTATION TIME (min) |
|---|---|---|---|---|---|---|---|
| 1 | 180 | 30 | ··· | 0 | 100 | ··· | 0 |
| 2 | 100 | 25 | ··· | 0 | 80 | ··· | 0 |
| 3 | 120 | 10 | ··· | 5 | 180 | ··· | 5 |
| 4 | 150 | 5 | ··· | 5 | 80 | ··· | 0 |
| ··· | ··· | ··· | ··· | ··· | ··· | ··· | ··· |

EP 4 129 261 A1

# FIG. 32

EXERCISE
PRESCRIPTION
INFORMATION
DATABASE

76

EXERCISE PRESCRIPTION INFORMATION DATABASE

### USER ID = 0001

| WEEK | EXERCISE TARGET TIME OF %peakVO2 = 40-49% (min) | EXERCISE TARGET TIME OF %peakVO2 = 50-59% (min) | ... | EXERCISE TARGET TIME OF %peakVO2 = 80-89% (min) |
|---|---|---|---|---|
| 1 | 180 | 30 | ... | 0 |
| 2 | 120 | 30 | ... | 0 |
| 3 | 260 | 30 | ... | 5 |
| 4 | 180 | 25 | ... | 10 |
| ... | ... | ... | ... | ... |

### USER ID = 0002

| WEEK | EXERCISE TARGET TIME OF %peakVO2 = 40-49% (min) | EXERCISE TARGET TIME OF %peakVO2 = 50-59% (min) | ... | EXERCISE TARGET TIME OF %peakVO2 = 80-89% (min) |
|---|---|---|---|---|
| 1 | 180 | 30 | ... | 0 |
| 2 | 100 | 25 | ... | 0 |
| 3 | 120 | 10 | ... | 5 |
| 4 | 150 | 5 | ... | 5 |
| ... | ... | ... | ... | ... |

EP 4 129 261 A1

# FIG. 33

IF CLICKING, TRANSIT TO NEXT UI

| PATIENT NAME | DISEASE | EXERCISE IMPLEMENTATION STATUS | EXERCISE PRESCRIPTION PROPER STATUS | |
|---|---|---|---|---|
| SHINTARO KAZUKI | CARDIOMYOPATHY | 40% | ADEQUATE | |
| YUJI YAHAGI | HYPERTENSION/PACEMAKER | 80% | LACK | CHANGE OF EXERCISE PRESCRIPTION |
| TAKAAKI IKEMOTO | CARDIOMYOPATHY | 50% | ADEQUATE | |
| HARUMI TERAMURA | VALVULAR DISEASE | 45% | ADEQUATE | |
| RIE KOGUMA | VALVULAR DISEASE | 20% | EXCESSIVE | CHANGE OF EXERCISE PRESCRIPTION |
| FUMIKO YOSHIMURA | ARRHYTHMIA | 5% | EXCESSIVE | CHANGE OF EXERCISE PRESCRIPTION |

EP 4 129 261 A1

EP 4 129 261 A1

**FIG. 34**

**PATIENT INFORMATION**

| NAME | YUJI YAHAGI | WEEK NUMBER | 12 WEEKS |
|---|---|---|---|
| AGE | 68 YEARS OLD (1952/3/2) | SPECIALTY | NOTHING SPECIALTY |

**CURRENT EXERCISE PRESCRIPTION**

*IF CLICKING, TRANSIT TO NEXT UI*

| WEEK\ EXERCISE LOAD% | 10 | 20 | 30 | 40 | 50 | 60 | 70 | 80 | 90 | 100 | EXERCISE PRESCRIPTION MENU | |
|---|---|---|---|---|---|---|---|---|---|---|---|---|
| 12 WEEKS | 0 | 0 | 60 | 0 | 40 | 0 | 0 | 30 | 0 | 0 | B | CHANGE / CONFIRMATION |

**LIST OF EXERCISE RESULTS**

| WEEK\ EXERCISE LOAD% | 10 | 20 | 30 | 40 | 50 | 60 | 70 | 80 | 90 | 100 | EXERCISE CONTENTS | EXERCISE PRESCRIPTION MENU | IN CHARGE |
|---|---|---|---|---|---|---|---|---|---|---|---|---|---|
| 1ST WEEK | 0 | 0 | 60 | 0 | 45 | 0 | 0 | 30 | 0 | 0 | CONFIRMATION | A | |
| 2ND WEEK | 0 | 0 | 60 | 0 | 45 | 0 | 0 | 30 | 0 | 0 | CONFIRMATION | A | |
| 3RD WEEK | 0 | 0 | 60 | 0 | 45 | 0 | 0 | 30 | 0 | 0 | CONFIRMATION | A | |
| 4TH WEEK | 0 | 0 | 60 | 0 | 45 | 0 | 15 | 15 | 0 | 0 | CONFIRMATION | | |
| 5TH WEEK | 0 | 0 | 60 | 0 | 45 | 0 | 20 | 10 | 0 | 0 | CONFIRMATION | A | |
| 6TH WEEK | 0 | 0 | 60 | 5 | 40 | 0 | 20 | 10 | 0 | 0 | CONFIRMATION | A | Sato |
| 7TH WEEK | 0 | 0 | 60 | 10 | 35 | 0 | 25 | 5 | 0 | 0 | CONFIRMATION | A | |
| 8TH WEEK | 0 | 0 | 60 | 0 | 40 | 0 | 0 | 25 | 5 | 0 | CONFIRMATION | B | |
| 9TH WEEK | 0 | 0 | 60 | 0 | 40 | 0 | 0 | 25 | 5 | 0 | CONFIRMATION | B | |
| 10TH WEEK | 0 | 0 | 60 | 0 | 40 | 0 | 0 | 30 | 0 | 0 | CONFIRMATION | B | |
| 11TH WEEK | 0 | 0 | 60 | 0 | 40 | 0 | 0 | 30 | 0 | 0 | CONFIRMATION | B | |

**POINT A** INDICATES THAT AT SAME EXERCISE INTENSITY, PATIENT'S CARDIORESPIRATORY FUNCTION INCREASES AND EXERCISE LOAD DECREASES

**POINT B** INDICATES THAT CHANGE IS MADE BY CHANGING PRESCRIPTION, AND IT IS EASY TO COMPARE INFLUENCE

# FIG. 35

SPECIFIC EXAMPLE OF EACH EXERCISE PRESCRIPTION MENU

| EXERCISE LOAD% | 10 | 20 | 30 | 40 | 50 | 60 | 70 | 80 | 90 | 100 |
|---|---|---|---|---|---|---|---|---|---|---|
| MENU A | 0 | 0 | 60 | 0 | 45 | 0 | 0 | 30 | 0 | 0 |
| MENU B | 0 | 0 | 60 | 0 | 30 | 0 | 10 | 10 | 5 | 0 |
| ⋮ | | | | | | | | | | |

## FIG. 36

| CONFIRMATION OF EXERCISE CONTENTS, 2ND WEEK | |
| --- | --- |
| MANUAL INPUT | IMPLEMENTATION TIME (MINUTE) |
| WALKING: 4.0km/h, FLAT AND HARD GROUND | 60 |
| WALKING: 4.7 TO 5.6km/h, UPHILL, GRADIENT OF 6 TO 15% | 10 |
| STAIR CLIMB 40cm/sec | 15 |
| WALK: 5.6km/h, FAST, FLAT AND HARD GROUND | 45 |
| WALK: 8.0km/h, FLAT AND HARD GROUND | 5 |
| ABOVE | |
| | |

CLOSE

# FIG. 37

CHANGE OF EXERCISE PRESCRIPTION

| EXERCISE LOAD% | 10 | 20 | 30 | 40 | 50 | 60 | 70 | 80 | 90 | 100 | |
|---|---|---|---|---|---|---|---|---|---|---|---|
| (A) RECOMMENDATION BY SYSTEM | 0 | 0 | 60 | 0 | 40 | 0 | 0 | 30 | 0 | 0 | DETERMINATION |
| (B) MANUAL INPUT | 0 | 0 | 0 | 0 | 0 | 0 | 0 | 0 | 0 | 0 | DETERMINATION |

# FIG. 38

EP 4 129 261 A1

(1)

NOTICE
THERE IS UPDATING OF
EXERCISE PRESCRIPTION
BY DOCTOR

CONFIRM CONTENTS

(2)

| LOW INTENSITY | 30 MINUTES/WEEK |
EXAMPLE: SLOW WALKING

| MEDIUM INTENSITY | 60 MINUTES/WEEK |
EXAMPLE: QUICK WALKING

| HIGH INTENSITY | 5 MINUTES/WEEK |
EXAMPLE: CLIMBING STAIRS

## FIG. 39

EP 4 129 261 A1

**INTERNATIONAL SEARCH REPORT**

| International application No. |
| --- |
| PCT/JP2021/003933 |

A. CLASSIFICATION OF SUBJECT MATTER
Int. Cl. A61H1/02(2006.01)i, G16H20/30(2018.01)i, A61B5/083(2006.01)i, A61B5/11(2006.01)i, A61B5/22(2006.01)i
FI: A61B5/22 100, A61H1/02 A, A61B5/083, A61B5/11 200, G16H20/30
According to International Patent Classification (IPC) or to both national classification and IPC

B. FIELDS SEARCHED

Minimum documentation searched (classification system followed by classification symbols)
Int. Cl. A61H1/02, G16H20/30, A61B5/083, A61B5/11, A61B5/22

Documentation searched other than minimum documentation to the extent that such documents are included in the fields searched
Published examined utility model applications of Japan     1922-1996
Published unexamined utility model applications of Japan   1971-2021
Registered utility model specifications of Japan           1996-2021
Published registered utility model applications of Japan   1994-2021

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X | WO 2008/120677 A1 (MATSUSHITA ELECTRIC WORKS, LTD.) 09 October 2008, paragraphs [0020]-[0047], | 1-2, 7-8, 10, 17, 19 |
| Y | fig. 1-13, paragraphs [0020]-[0047], fig. 1-13 | 3-6, 9, 11, 14-15, 18, 20 |
| A | | 12-13, 16 |
| Y | JP 6115312 B2 (PANASONIC INTELLECTUAL PROPERTY MANAGEMENT CO., LTD.) 19 April 2017, paragraphs [0030], [0031] | 3, 9, 11, 14, 18, 20 |
| Y | WO 2015/133417 A1 (BANDO CHEMICAL INDUSTRIES, LTD.) 11 September 2015, paragraph [0076] | 4-5, 15 |
| Y | JP 2005-636 A (O2RUN CO., LTD.) 06 January 2005, paragraph [0038] | 6 |

☒ Further documents are listed in the continuation of Box C.     ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 24.03.2021 | 13.04.2021 |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2021/003933

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2013-244357 A (TOYOTA TSUSHO CORP.) 09 December 2013, paragraph [0050] | 6 |
| A | JP 2006-101973 A (MICROSTONE CORP.) 20 April 2006, entire text, all drawings | 1-20 |
| A | JP 2018-201905 A (FUJI XEROX CO., LTD.) 27 December 2018, entire text, all drawings | 1-20 |

Form PCT/ISA/210 (continuation of second sheet) (January 2015)

<table>
<tr><td colspan="2"><b>INTERNATIONAL SEARCH REPORT</b><br>Information on patent family members</td><td colspan="2">International application No.<br>PCT/JP2021/003933</td></tr>
</table>

| Patent Documents referred to in the Report | Publication Date | Patent Family | Publication Date |
|---|---|---|---|
| WO 2008/120677 A1 | 09.10.2008 | US 2010/0130890 A1 paragraphs [0036]-[0057], fig. 1-13 CN 101668482 A | |
| JP 6115312 B2 | 19.04.2017 | (Family: none) | |
| WO 2015/133417 A1 | 11.09.2015 | US 2017/0074636 A1 paragraph [0163] EP 3115740 A1 CN 106062505 A KR 10-2016-0129006 A | |
| JP 2005-636 A | 06.01.2005 | WO 2004/109575 A1 p. 14 KR 10-0533105 B1 | |
| JP 2013-244357 A | 09.12.2013 | (Family: none) | |
| JP 2006-101973 A | 20.04.2006 | (Family: none) | |
| JP 2018-201905 A | 27.12.2018 | US 2018/0345082 A1 entire text, all drawings | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014018213 A

- JP 2016158711 A